(19) Europäisches Patentamt
European Patent Office
Office européen des brevets

(11) EP 3 532 479 B1

(12) # EUROPEAN PATENT SPECIFICATION

(45) Date of publication and mention of the grant of the patent:
**07.06.2023 Bulletin 2023/23**

(21) Application number: **17863621.3**

(22) Date of filing: **24.10.2017**

(51) International Patent Classification (IPC):
***C07D 493/10*** *(2006.01)* ***C07D 495/04*** *(2006.01)*
***A61K 31/5377*** *(2006.01)*

(52) Cooperative Patent Classification (CPC):
**C07D 213/56; A61P 1/04; A61P 3/00; A61P 7/00; A61P 9/00; A61P 9/10; A61P 11/06; A61P 13/12; A61P 15/10; A61P 17/00; A61P 17/06; A61P 19/02; A61P 21/00; A61P 25/00; A61P 25/14;** (Cont.)

(86) International application number:
**PCT/US2017/058071**

(87) International publication number:
**WO 2018/081108 (03.05.2018 Gazette 2018/18)**

(54) **AMIDE COMPOUNDS AS KINASE INHIBITORS**

AMIDVERBINDUNGEN ALS KINASEHEMMER

COMPOSÉS AMIDES EN TANT QU'INHIBITEURS DE KINASE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.10.2016 US 201662411908 P**

(43) Date of publication of application:
**04.09.2019 Bulletin 2019/36**

(73) Proprietor: **Translational Drug Development, LLC**
**Scottsdale, Arizona 85259 (US)**

(72) Inventors:
- **WANG, Tong**
  **Scottdale, Arizona 85259 (US)**
- **GATELY, Stephen**
  **Scottsdale, Arizona 85259 (US)**

(74) Representative: **Petty, Catrin Helen et al**
**Venner Shipley LLP**
**200 Aldersgate**
**London EC1A 4HD (GB)**

(56) References cited:
**WO-A1-2012/006202** **WO-A1-2012/006203**
**US-B2- 9 079 880** **US-B2- 9 382 197**

- **L. E. RAJAGOPALAN ET AL: "Biochemical, Cellular, and Anti-Inflammatory Properties of a Potent, Selective, Orally Bioavailable Benzamide Inhibitor of Rho Kinase Activity", JOURNAL OF PHARMACOLOGY AND EXPERIMENTAL THERAPEUTICS, vol. 333, no. 3, 1 January 2010 (2010-01-01), pages 707-716, XP055480499, US ISSN: 0022-3565, DOI: 10.1124/jpet.110.166033**
- **RAJAGOPALAN ET AL.: 'Biochemical , Cellular, and Anti-Inflammatory Properties of a Potent, Selective, Orally Bioavailable Benzamide Inhibitor of Rho Kinase Activity' THE JOURNAL OF PHARMACOLOGY AND EXPERIMENTAL THERAPEUTICS vol. 333, 14 May 2010, pages 707 - 716, XP055480499**

Remarks:
The file contains technical information submitted after the application was filed and not included in this specification

EP 3 532 479 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)
**A61P 25/16; A61P 25/28; A61P 27/02; A61P 29/00; A61P 31/00; A61P 35/00; A61P 35/02; A61P 37/06; A61P 37/08; A61P 43/00; C07D 211/26; C07D 213/81; C07D 231/12; C07D 401/04; C07D 401/12; C07D 405/12; C07D 413/12; C07D 487/04; C07D 513/04**

## Description

### CROSS REFERENCE TO RELATED APPLICATIONS

This application claims priority to U.S. Provisional Patent Application No. 62/411,908, filed October 24, 2016.

### FIELD OF THE INVENTION

The present invention relates to amide compounds, their compositions and medicaments containing the same, as well as processes for the preparation and use of such compounds, compositions and medicaments. Such compounds are potentially useful in the treatment of diseases associated with inappropriate tyrosine and/or serine/threonine kinase activities including cell proliferation diseases (e.g., cancer) and neurological and inflammatory diseases. Specifically, this disclosure is concerned with compounds and compositions inhibiting Rho-kinases, methods of treating diseases associated with Rho-kinases, and methods of synthesizing these compounds.

### BACKGROUND OF THE INVENTION

An important large family of enzymes is the protein kinase enzyme family. Currently, there are about 500 different known protein kinases. Protein kinases serve to catalyze the phosphorylation of an amino acid side chain in various proteins by the transfer of the γ-phosphate of the ATP-$Mg^{2+}$ complex to said amino acid side chain. These enzymes control the majority of the signaling processes inside the cells, thereby governing cell function, growth, differentiation, and destruction (apoptosis) through reversible phosphorylation of the hydroxyl groups of serine, threonine and tyrosine residues in proteins. Studies have shown that protein kinases are key regulators of many cell functions, including signal transduction, transcriptional regulation, cell motility, and cell division. Several oncogenes have also been shown to encode protein kinases, suggesting that kinases play a role in oncogenesis. These processes are highly regulated, often by complex intermeshed pathways where each kinase will itself be regulated by one or more kinases. Consequently, aberrant or inappropriate protein kinase activity can contribute to the rise of disease states associated with such aberrant kinase activity. Due to their physiological relevance, variety and ubiquitousness, protein kinases have become one of the most important and widely studied family of enzymes in biochemical and medical research.

The protein kinase family of enzymes is typically classified into two main subfamilies: Protein Tyrosine Kinases and Protein Serine/Threonine Kinases, based on the amino acid residue they phosphorylate. The serine/threonine kinases (PSTK), include cyclic AMP- and cyclic GMP-dependent protein kinases, calcium- and phospholipid-dependent protein kinases, calcium- and calmodulin-dependent protein kinases, casein kinases, cell division cycle protein kinases, and others. These kinases are usually cytoplasmic or associated with the particulate fractions of cells, possibly by anchoring proteins. Aberrant protein serine/threonine kinase activity has been implicated or is suspected in a number of pathologies such as rheumatoid arthritis, psoriasis, septic shock, bone loss, many cancers, and other proliferative diseases. Accordingly, serine/threonine kinases and the signal transduction pathways which they are part of are important targets for drug design. The tyrosine kinases phosphorylate tyrosine residues. Tyrosine kinases play an equally important role in cell regulation. These kinases include several receptors for molecules such as growth factors and hormones, including epidermal growth factor receptor, insulin receptor, platelet derived growth factor receptor, and others. Studies have indicated that many tyrosine kinases are transmembrane proteins with their receptor domains located on the outside of the cell and their kinase domains on the inside. Much work is also under progress to identify modulators of tyrosine kinases as well.

A major signal transduction systems utilized by cells is the RhoA- signaling pathways. RhoA is a small GTP binding protein that can be activated by several extracellular stimuli such as growth factor, hormones, mechanic stress, osmotic change, as well as high concentration of metabolite like glucose. RhoA activation involves GTP binding, conformation alteration, post-translational modification (geranylgeranyllization and famesylation), and activation of its intrinsic GTPase activity. Activated RhoA is capable of interacting with several effector proteins including ROCKs and transmit signals into cellular cytoplasm and the nucleus.

Rho-associated protein kinases, ROCK1 and ROCK2, constitute a family of kinases that can be activated by RhoA-GTP complex via physical association. Activated ROCKs phosphorylate a number of substrates and play important roles in pivotal cellular functions. The substrates for ROCKs include the myosin binding subunit of myosin light chain phosphatase (MBS, also named MYPT1), adducin, moesin, myosin light chain (MLC), LIM kinase, as well as transcription factor FHL. The phosphorylation of these substrates modulate the biological activity of the proteins and thus provide a means to alter cell's response to external stimuli. One well documented example is the participation of ROCK in smooth muscle contraction. Upon stimulation by phenylephrine, the vascular smooth muscle contracts. Studies have shown that phenylephrine stimulates alpha-adrenergic receptors and leads to the activation of RhoA. Activated RhoA in turn stimulates kinase activity of ROCK1 which in turn phosphorylates MBS. Such phosphorylation inhibits the enzyme activity

of myosin light chain phosphatase and increases the phosphorylation of myosin light chain itself by a calcium-dependent myosin light chain kinase (MLCK) and consequently increases the contractility of myosin-actin bundle, leading to smooth muscle contraction. This phenomena is sometimes called calcium sensitization. In addition to smooth muscle contraction, ROCKs have also been shown to be involved in cellular functions including apoptosis, cell migration, transcriptional activation, fibrosis, cytokinesis, inflammation, and cell proliferation. Moreover, in neurons, ROCK plays a critical role in the inhibition of axonal growth by myelin-associated inhibitory factors such as myelin-associated glycoprotein (MAG). ROCK-activity also mediates the collapse of growth cones in developing neurons. Both processes are thought to be mediated by ROCK-induced phosphorylation of substrates such as LIM kinase and myosin light chain phosphatase, resulting in increased contractility of the neuronal actin-myosin system. Inhibitors of ROCKs have been suggested for use in the treatments of a variety of diseases. They include cardiovascular diseases such as hypertension, chronic and congestive heart failure, cardiac hypertrophy, restenosis, chronic renal failure, and atherosclerosis. In addition, because of its muscle relaxing properties, it is also suitable for asthma, male erectile dysfunctions, female sexual dysfunction, and overactive bladder syndrome. ROCK inhibitors have been shown to possess anti-inflammatory properties. Thus they can be used as treatment for neuroinflammatory diseases such as stroke, multiple sclerosis, Alzheimer’s disease, Parkinson’s disease, amyotrophic lateral sclerosis, and inflammatory pain, as well as other inflammatory diseases such as rheumatoid arthritis, irritable bowel syndrome, and inflammatory bowel disease. In addition, based on their neurite outgrowth inducing effects, ROCK inhibitors could be useful drugs for neuronal regeneration, inducing new axonal growth and axonal rewiring across lesions within the CNS. ROCK inhibitors are therefore likely to be useful for regenerative (recovery) treatment of CNS disorders such as spinal cord injury, acute neuronal injury (stroke, traumatic brain injury), Parkinsons disease, Alzheimers disease, and other neurodegenerative disorders. Since ROCK inhibitors reduce cell proliferation and cell migration, they could be useful in treating cancer and tumor metastasis. Furthermore, there is evidence suggesting that ROCK inhibitors suppress cytoskeletal rearrangement upon virus invasion, thus they also have potential therapeutic value in antiviral and anti-bacterial applications. ROCK inhibitors may also be useful for the treatment of insulin resistance and diabetes.

WO 2012/006202 A1 describes amide derivatives which can be used to inhibit Rho kinase. Similarly, WO 2012/006203 A1 describes substituted amide derivatives which can be used to inhibit Rho kinase.

The present inventors have discovered novel heterocyclic compounds, which are inhibitors of ROCK activities. Such derivatives are useful in the treatment of disorders associated with inappropriate or dysregulated ROCK activities.

## SUMMARY OF THE INVENTION

In one aspect of the present invention, there is provided a compound of Formula (I):

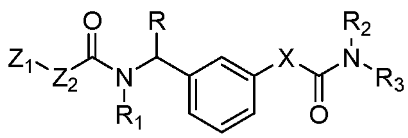

**I**

wherein:

$Z_1$ is pyridine or pyrazole, optionally substituted with halo, -OH, -CN, -COOR’, -OR’, -SR’, -OC(O)R’, -NHR’, -NR’R", -NHC(O)R’, -NHC(O)NR’R", -C(O)NR’R", -NS(O)2R’, -S(O)2NR’R", -S(O)2R’, guanidino, nitro, nitroso, -Cl-C6 alkyl, aryl, -C3-C7 cycloalkyl, or a 3 to 10-membered heterocycle, wherein the -Cl-C6 alkyl, aryl, -C3-C7 cycloalkyl, or 3 to 10-membered heterocycle is unsubstituted or substituted with one or more of the following: halo, -OH, -CN, -COOR’, -OR’, -SR’, -OC(O)R’, -NHR’, -NR’R", -NHC(O)R’, -NHC(O)NR’R", -C(O)NR’R", -NS(O)2R’, -S(O)2NR’R", -S(O)2R’, guanidino, nitro, nitroso, -Cl-C6 alkyl, aryl, or -C3-C7 cycloalkyl;

$Z_2$ is phenyl, naphthyl, or C5-C10 membered heterocycle, optionally substituted with halo, -OH, -CN, -COOR’, -OR’, -SR’, -OC(O)R’, -NHR’, -NR’R", -NHC(O)R’, -NHC(O)NR’R", -C(O)NR’R", -NS(O)2R’, -S(O)2NR’R", -S(O)2R’, guanidino, nitro, nitroso, -Cl-C6 alkyl, aryl, -C3-C7 cycloalkyl, or a 3 to 10-membered heterocycle, wherein the -Cl-C6 alkyl, aryl, -C3-C7 cycloalkyl, or 3 to 10-membered heterocycle any of which are unsubstituted or substituted with one or more of the following: halo, -OH, -CN, -COOR’, -OR’, -SR’, -OC(O)R’, -NHR’, -NR’R", -NHC(O)R’, -NHC(O)NR’R", -C(O)NR’R", -NS(O)2R’, -S(O)2NR’R", -S(O)2R’, guanidino, nitro, nitroso, -Cl-C6 alkyl, aryl, or -C3-C7 cycloalkyl;

R is -C1-C6 alkyl, optionally substituted with one or more -OH;

$R_1$ is H, -C1-C6 alkyl or -C3-C7 cycloalkyl, wherein -C1-C6 alkyl or -C3-C7 cycloalkyl are optionally substituted with

one or more of the following: halo, -OH, -CN, -COOR', -OR', - SR', -OC(O)R', -NHR', -NR'R", -NHC(O)R', -NHC(O)NR'R", -C(O)NR'R", -NS(O)2R', - S(O)2NR'R", or -S(O)2R';

X is a bond or -O(C1-C6 alkyl); and

$R_2$ and $R_3$ are independently H, -C1-C6 alkyl or -C3-C7 cycloalkyl, aryl, or C5-C10 membered heterocycle, optionally substituted with halo, -OH, -CN, -COOR', -OR', -SR', - OC(O)R', -NHR', -NR'R", -CNR'R", -NHC(O)R', -NHC(O)NR'R", -C(O)NR'R", -NS(O)2R', - S(O)2NR'R", -S(O)2R', guanidino, nitro, nitroso, -Cl-C6 alkyl, aryl, -C3-C7 cycloalkyl, or 3 to 10-membered heterocycle, wherein the -Cl-C6 alkyl, aryl, -C3-C7 cycloalkyl, or 3 to 10-membered heterocycle any of which are unsubstituted or substituted with one or more of the following: halo, -OH, -CN, -COOR', -OR', -SR', -OC(O)R', -NHR', -NR'R", -NHC(O)R', - NHC(O)NR'R", -C(O)NR'R", -NS(O)2R', -S(O)2NR'R", -S(O)2R', guanidino, nitro, nitroso, -Cl-C6 alkyl, aryl, -C3-C7 cycloalkyl; or

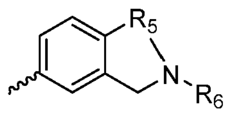
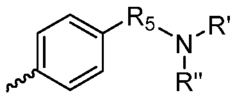
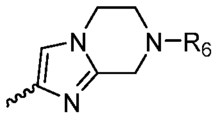
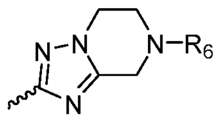

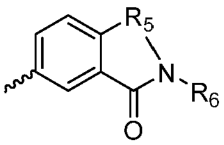
,

wherein $R_5$ is -Cl-C6 alkyl, -OCH2CH2-, -NR6CH2CH2-, -NC(O)CH2CH2-; and R6 is H, -C1-C6 alkyl or -C3-C7 cycloalkyl; and

wherein R' or R" are independently -H or -Cl-C6 alkyl; and wherein R' and R" together, optionally attaching to N or O atom, may form a 4 to 8 membered cyclic structure.

Also described is a compound of Formula (II):

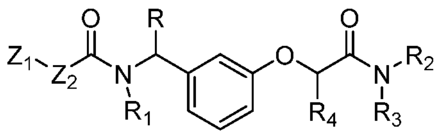

**II**

wherein: Z1 is H, pyridine, pyrimidine, pyrazole, imidazole, oxazole, thiazole, indazole or tetrazole, optionally substituted with H, halo, -OH, -CN, -COOR', -OR', -SR', -OC(O)R', -NHR', -NR'R", -NHC(O)R', -NHC(O)NR'R", -C(O)NR'R", -NS(O)2R', -S(O)2NR'R", -S(O)2R', guanidino, nitro, nitroso, -Cl-C6 alkyl, aryl, -C3-C7 cycloalkyl, or a 3 to 10-membered heterocycle, wherein the -Cl-C6 alkyl, aryl, -C3-C7 cycloalkyl, or 3 to 10-membered heterocycle any of which are unsubstituted or substituted with one or more of the following: halo, -OH, -CN, -COOR', -OR', -SR', -OC(O)R', -NHR', -NR'R", -NHC(O)R', -NHC(O)NR'R", -C(O)NR'R", -NS(O)2R', -S(O)2NR'R", -S(O)2R', guanidino, nitro, nitroso, -Cl-C6 alkyl, aryl, or -C3-C7 cycloalkyl; Z2 is phenyl, naphthyl, or a C5-C10 membered heterocycle, optionally substituted with H, halo, -OH, -CN, -COOR', -OR', -SR', -OC(O)R', -NHR', -NR'R", - NHC(O)R', -NHC(O)NR'R", -C(O)NR'R", -NS(O)2R', -S(O)2NR'R", -S(O)2R', guanidino, nitro, nitroso, -Cl-C6 alkyl, aryl, -C3-C7 cycloalkyl, or a 3 to 10-membered heterocycle, wherein the -Cl-C6 alkyl, aryl, -C3-C7 cycloalkyl, or 3 to 10-membered heterocycle any of which are unsubstituted or substituted with one or more of the following: halo, -OH, -CN, -COOR', -OR', - SR', -OC(O)R', -NHR', -NR'R", -NHC(O)R', -NHC(O)NR'R", -C(O)NR'R", -NS(O)2R', -S(O)2NR'R", -S(O)2R', guanidino, nitro, nitroso, -Cl-C6 alkyl, aryl, or -C3-C7 cycloalkyl; R is -C1-C6 alkyl, optionally substituted with one or more of the following: H, halo, -OH, -CN, - COOR', -OR', -SR', -OC(O)R', -NHR', -NR'R", -NHC(O)R', -NHC(O)NR'R", -C(O)NR'R" , - NS(O)2R', -S(O)2NR'R", or -S(O)2R'; R1 is H, -C1-C6 alkyl or -C3-C7 cycloalkyl, wherein - C1-C6 alkyl or -C3-C7 cycloalkyl are optionally substituted with one or more of the following: halo, -OH, -CN, -COOR', -OR', -SR', -OC(O)R', -NHR', -NR'R", -NHC(O)R', -NHC(O)NR'R", -C(O)NR'R", -NS(O)2R', -S(O)2NR'R", or -S(O)2R'; R2 and R3 are independently H, -C1-C6 alkyl or -C3-C7 cycloalkyl, aryl, or a C5-C10 membered heterocycle, optionally substituted with H, halo, -OH, -CN, -COOR', -OR', -SR', -OC(O)R', -NHR', -NR'R", -CNR'R", -NHC(O)R', - NHC(O)NR'R", -C(O)NR'R", -NS(O)2R', -S(O)2NR'R", -S(O)2R', guanidino, nitro, nitroso, - C1-C6 alkyl, aryl, -C3-C7 cycloalkyl, or a 3 to 10-membered heterocycle, wherein the -Cl-C6 alkyl, aryl, -C3-C7 cycloalkyl, or 3 to 10-membered heterocycle any of which are unsubstituted or substituted with one or more of the following: halo, -OH, -CN, -COOR’, -OR’, -SR’, - OC(O)R’, -NHR’, -NR’R", -NHC(O)R’, -NHC(O)NR’R", -C(O)NR’R", -NS(O)2R’, - S(O)2NR’R", -S(O)2R’, guanidino, nitro, nitroso, -Cl-C6 alkyl, aryl, -C3-C7 cycloalkyl; or

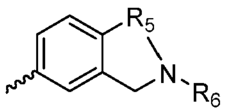 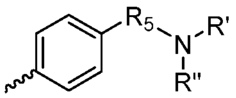 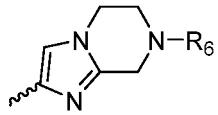 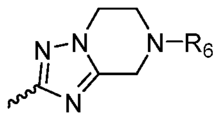

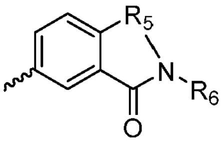

,

wherein R5 is -Cl-C6 alkyl, -OCH2CH2-, -NR6CH2CH2-, or -NC(O)CH2CH2-; R6 is H, -C1-C6 alkyl or -C3-C7 cycloalkyl; and wherein R’ or R" are independently -H or -Cl-C6 alkyl; R’ and R" together, optionally attaching to N or O atom, may form a 4 to 8 membered cyclic structure; and R4 is H, -C1-C6 alkyl or -C3-C7 cycloalkyl.

In an embodiment of the invention is provided a compound of Formula (III):

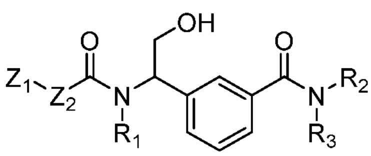

**III**

wherein:

$Z_1$ is pyridine or pyrazole, wherein the pyridine or pyrazole is optionally substituted with halo, -OH, -CN, -COOR’, -OR’, -SR’, -OC(O)R’, -NHR’, -NR’R", -NHC(O)R’, -NHC(O)NR’R", -C(O)NR’R", -NS(O)2R’, -S(O)2NR’R", -S(O)2R’, guanidino, nitro, nitroso, -Cl-C6 alkyl, aryl, -C3-C7 cycloalkyl, or a 3 to 10-membered heterocycle, wherein the -Cl-C6 alkyl, aryl, -C3-C7 cycloalkyl, or 3 to 10-membered heterocycle any of which are unsubstituted or substituted with one or more of the following: halo, -OH, -CN, -COOR’, -OR’, -SR’, -OC(O)R’, -NHR’, -NR’R", -NHC(O)R’, -NHC(O)NR’R", -C(O)NR’R", -NS(O)2R’, -S(O)2NR’R", -S(O)2R’, guanidino, nitro, nitroso, -Cl-C6 alkyl, aryl, or -C3-C7 cycloalkyl;

$Z_2$ is phenyl, naphthyl, or a C5-C10 membered heterocycle, optionally substituted with halo, -OH, -CN, -COOR’, -OR’, -SR’, -OC(O)R’, -NHR’, -NR’R", -NHC(O)R’, -NHC(O)NR’R", -C(O)NR’R", -NS(O)2R’, -S(O)2NR’R", -S(O)2R’, guanidino, nitro, nitroso, -Cl-C6 alkyl, aryl, -C3-C7 cycloalkyl, or a 3 to 10-membered heterocycle, wherein the -Cl-C6 alkyl, aryl, -C3-C7 cycloalkyl, or 3 to 10-membered heterocycle any of which are unsubstituted or substituted with one or more of the following: halo, -OH, -CN, -COOR’, -OR’, -SR’, -OC(O)R’, -NHR’, -NR’R", -NHC(O)R’, -NHC(O)NR’R", -C(O)NR’R", -NS(O)2R’, -S(O)2NR’R", -S(O)2R’, guanidino, nitro, nitroso, -Cl-C6 alkyl, aryl, or -C3-C7 cycloalkyl;

$R_1$ is H, -C1-C6 alkyl or -C3-C7 cycloalkyl, wherein -C1-C6 alkyl or -C3-C7 cycloalkyl are optionally substituted with one or more of the following: halo, -OH, -CN, -COOR’, -OR’, -SR’, -OC(O)R’, -NHR’, -NR’R", -NHC(O)R’, -NHC(O)NR’R", -C(O)NR’R", -NS(O)2R’, -S(O)2NR’R", or -S(O)2R’;

$R_2$ and $R_3$ are independently H, -C1-C6 alkyl, -C3-C7 cycloalkyl, aryl, a C5-C10 membered heterocycle,

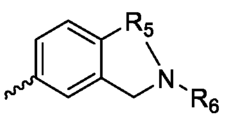 , 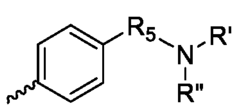 , 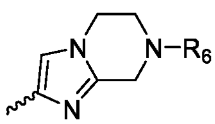 ,

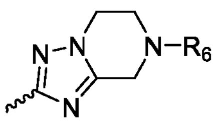 , or 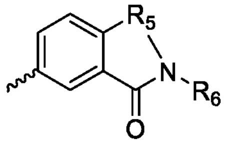 ,

wherein the $C_1$-$C_6$ alkyl, $C_3$-$C_7$ cycloalkyl, aryl, or C5-C10 membered heterocycle is optionally substituted with halo, -OH, -CN, -COOR’, -OR’, -SR’, - OC(O)R’, -NHR’, -NR’R", -CNR’R", -NHC(O)R’, -NHC(O)NR’R", -C(O)NR’R", -NS(O)2R’, - S(O)2NR’R", -S(O)2R’, guanidino, nitro, nitroso, -Cl-C6 alkyl, aryl, -C3-C7 cycloalkyl, or a 3 to 10-membered heterocycle, wherein the -Cl-C6 alkyl, aryl, -C3-C7 cycloalkyl, or 3 to 10-membered heterocycle any of which are unsubstituted or substituted with one or more of the following: halo, -OH, -CN, -COOR’, -OR’, -SR’, -OC(O)R’, -NHR’, -NR’R", -NHC(O)R’, - NHC(O)NR’R", -C(O)NR’R", -NS(O)2R’, -S(O)2NR’R", -S(O)2R’, guanidino, nitro, nitroso, - C1-C6 alkyl, aryl, -C3-C7 cycloalkyl;

wherein $R_5$ is -Cl-C6 alkyl, -OCH2CH2-, -NR6CH2CH2-, or -NC(O)CH2CH2-; and $R_6$ is H, -C1-C6 alkyl or -C3-C7 cycloalkyl; and

wherein R’ or R" are independently -H or -Cl-C6 alkyl, or -C3-C7 cycloalkyl; and R’ and R" together, optionally attaching to N or O atom, may form a 4 to 8 membered cyclic structure.

In another embodiment, the present invention relates to a compound of Formula (IV):

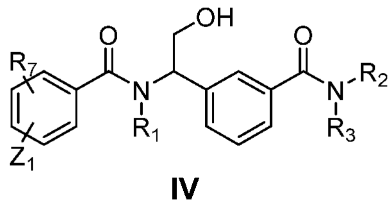

**IV**

wherein:

$Z_1$ is pyridine or pyrazole, wherein the pyridine or pyrazole is optionally substituted with halo, -OH, -CN, -COOR’, -OR’, -SR’, -OC(O)R’, -NHR’, -NR’R", -NHC(O)R’, -NHC(O)NR’R", -C(O)NR’R", -NS(O)2R’, -S(O)2NR’R", -S(O)2R’, guanidino, nitro, nitroso, -Cl-C6 alkyl, aryl, -C3-C7 cycloalkyl, or a 3 to 10-membered heterocycle, wherein the -Cl-C6 alkyl, aryl, -C3-C7 cycloalkyl, or 3 to 10-membered heterocycle any of which are unsubstituted or substituted with one or more of the following: halo, -OH, -CN, -COOR’, -OR’, -SR’, -OC(O)R’, -NHR’, -NR’R", -NHC(O)R’, -NHC(O)NR’R", -C(O)NR’R", -NS(O)2R’, -S(O)2NR’R", -S(O)2R’, guanidino, nitro, nitroso, -Cl-C6 alkyl, aryl, or -C3-C7 cycloalkyl;

$R_1$ is H, -C1-C6 alkyl or -C3-C7 cycloalkyl, wherein -C1-C6 alkyl or -C3-C7 cycloalkyl are optionally substituted with one or more of the following: halo, -OH, -CN, -COOR’, -OR’, -SR’, -OC(O)R’, -NHR’, -NR’R", -NHC(O)R’, -NHC(O)NR’R", -C(O)NR’R", -NS(O)2R’, -S(O)2NR’R", or -S(O)2R’;

$R_2$ and $R_3$ are independently H, -C1-C6 alkyl, -C3-C7 cycloalkyl, aryl, a C5-C10 membered heterocycle,

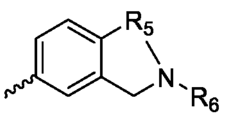 , 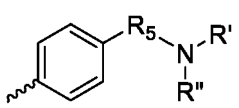 , 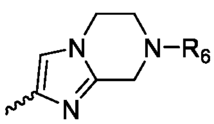 ,

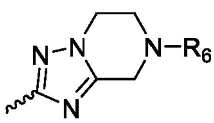 , or 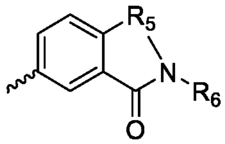 ,

wherein the $C_1$-$C_6$ alkyl, $C_3$-$C_7$ cycloalkyl, aryl, or C5-C10 membered heterocycle is optionally substituted with halo, -OH, -CN, -COOR’, -OR’, -SR’, - OC(O)R’, -NHR’, -NR’R", -CNR’R", -NHC(O)R’, -NHC(O)NR’R", -C(O)NR’R", -NS(O)2R’, - S(O)2NR’R", -S(O)2R’, guanidino, nitro, nitroso, -Cl-C6 alkyl, aryl, -C3-C7 cycloalkyl, or a 3 to 10-membered heterocycle, wherein the -Cl-C6 alkyl, aryl, -C3-C7 cycloalkyl, or 3 to 10-membered heterocycle any of

which are unsubstituted or substituted with one or more of the following: halo, -OH, -CN, -COOR’, -OR’, -SR’, -OC(O)R’, -NHR’, -NR’R", -NHC(O)R’, - NHC(O)NR’R", -C(O)NR’R", -NS(O)2R’, -S(O)2NR’R", -S(O)2R’, guanidino, nitro, nitroso, - Cl-C6 alkyl, aryl, -C3-C7 cycloalkyl;

wherein $R_5$ is -Cl-C6 alkyl, -OCH2CH2-, -NR6CH2CH2-, or -NC(O)CH2CH2-; and $R_6$ is H, -C1-C6 alkyl or -C3-C7 cycloalkyl;

wherein R’ or R" are independently -H or -Cl-C6 alkyl; and R’ and R" together, optionally attaching to N or O atom, may form a 4 to 8 membered cyclic structure; and

$R_7$ is H, halo, -OH, -CN, -COOR’, -OR’, -SR’, -OC(O)R’, -NHR’, -NR’R", -NHC(O)R’, - NHC(O)NR’R", -C(O)NR’R", -NS(O)2R’, -S(O)2NR’R", -S(O)2R’, -C1-C6 alkyl or -C3-C7 cycloalkyl, aryl, or a C5-C10 membered heterocycle, wherein the -C1-C6 alkyl, -C3-C7 cycloalkyl, aryl, or C5-C10 membered heterocycle is optionally substituted with halo, -OH, -CN, -COOR’, -OR’, -SR’, -OC(O)R’, -NHR’, -NR’R", -NHC(O)R’, -NHC(O)NR’R", -C(O)NR’R", -NS(O)2R’, -S(O)2NR’R", -S(O)2R’, guanidino, nitro, nitroso, -Cl-C6 alkyl, aryl, -C3-C7 cycloalkyl, or a 3 to 10-membered heterocycle.

In some aspects, R is methyl with R configuration. In other aspects, R is hydroxymethyl with S configuration.

In another embodiment, the present invention relates to a compound, wherein R2 and R3 are independently H, -C3-C7 cycloalkyl, or -C3-C7 cycloalkyl methyl; wherein the -C3-C7 cycloalkyl or -C3-C7 cycloalkyl methyl is optionally substituted with H, halo, -OH, -CN, - COOR’, -OR’, -SR’, -OC(O)R’, -NHR’, -NR’R", -CNR’R", -NHC(O)R’, -NHC(O)NR’R", -C(O)NR’R", -NS(O)2R’, -S(O)2NR’R", -S(O)2R’, guanidino, nitro, nitroso, - C1-C6 alkyl, aryl, -C3-C7 cycloalkyl, or a 3 to 10-membered heterocycle; with the proviso that R2 and R3 are not both H.

In certain aspects, R2 and R3 are independently H, cyclohexyl, cyclopentyl, cyclobutyl, cyclopropyl, cyclohexyl methyl, cyclopentyl methyl, cyclobutyl methyl or cyclopropyl methyl; and R2 and R3 are independently optionally substituted with H, halo, -OH, -CN, -COOR’, -OR’, -SR’, -OC(O)R’, -NHR’, -NR’R", -CNR’R", -NHC(O)R’, -NHC(O)NR’R", -C(O)NR’R", -NS(O)2R’, -S(O)2NR’R", -S(O)2R’, guanidino, nitro, nitroso, -Cl-C6 alkyl, aryl, -C3-C7 cycloalkyl, or a 3 to 10-membered heterocycle. In one aspect, R1 and/or R2 are H.

In some embodiments, the present invention relates to a compound selected from the group consisting of

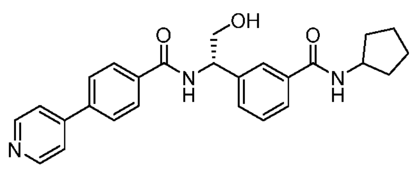

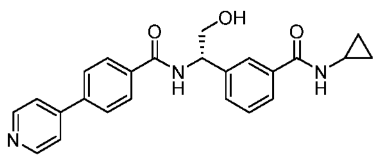

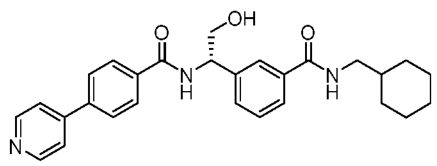

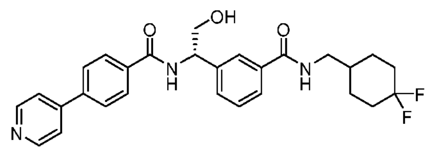

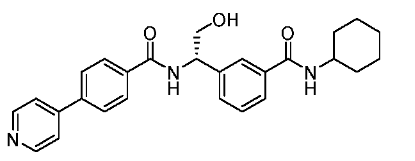

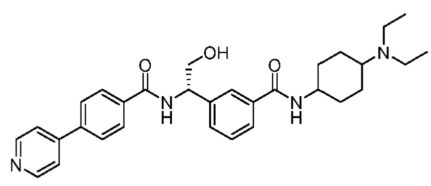

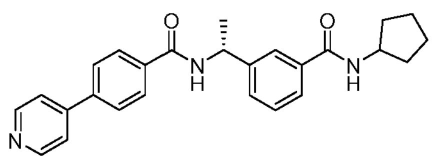

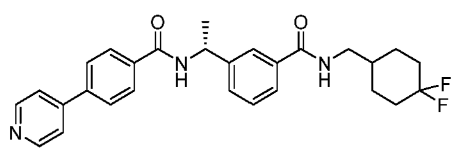

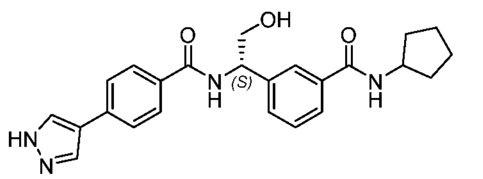

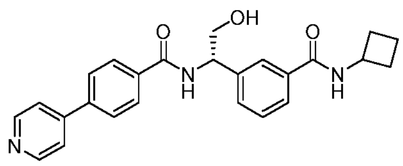

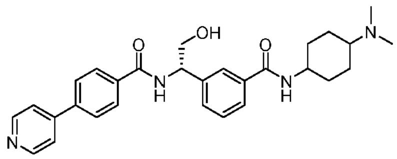

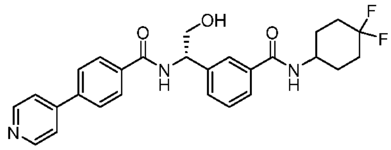

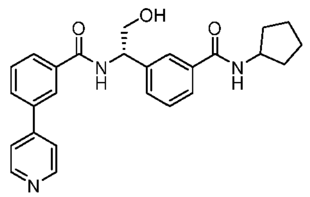

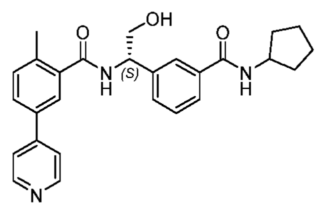

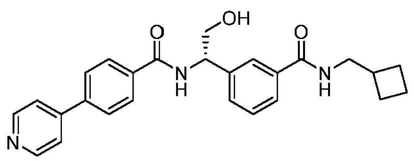

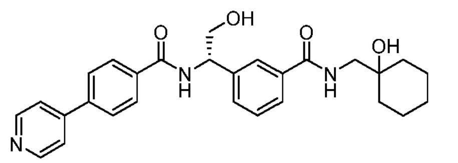

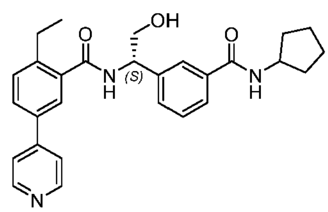

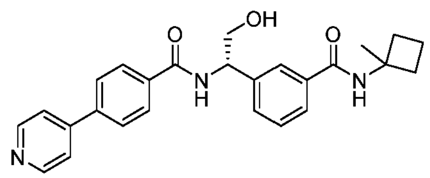

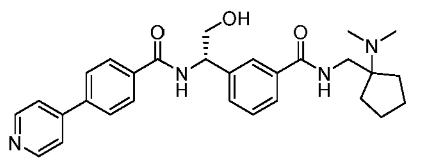

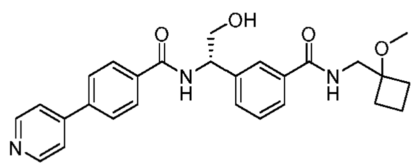

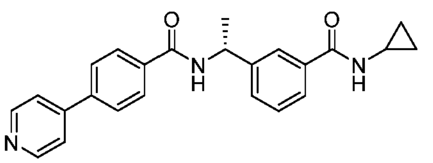

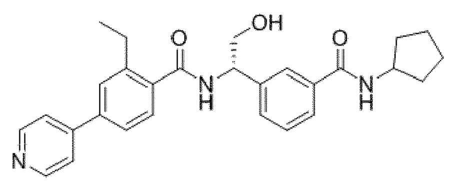

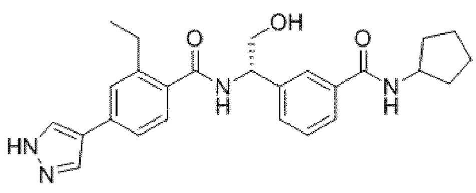

and

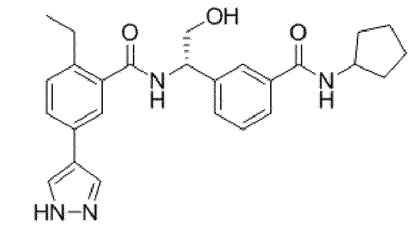

.

In other embodiments, R2 and R3 are independently H, phenyl, or benzyl; wherein the phenyl or benzyl is optionally substituted with H, halo, -OH, -CN, -COOR', -OR', -SR', - OC(O)R', -NHR', -NR'R", -CNR'R", -NHC(O)R', -NHC(O)NR'R", -C(O)NR'R", -NS(O)2R', - S(O)2NR'R", -S(O)2R', guanidino, nitro, nitroso, -Cl-C6 alkyl, aryl, -C3-C7 cycloalkyl, or a 3 to 10-membered heterocycle; with the proviso that R2 and R3 are not both H.

In one aspect, the present invention provides a compound selected from the group consisting of

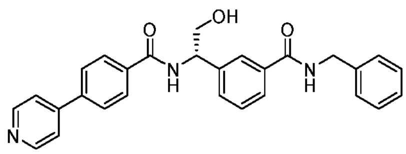

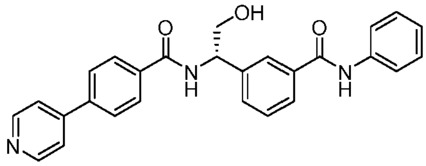

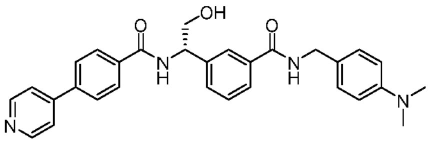

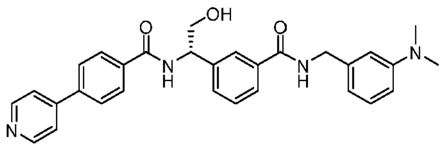

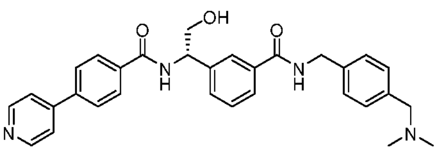

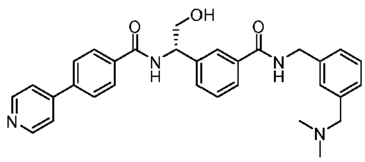

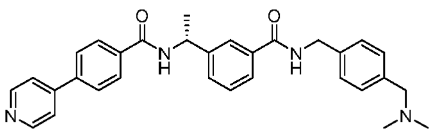

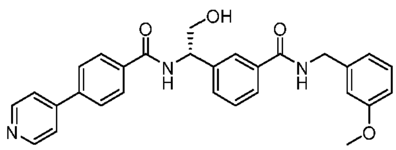

and

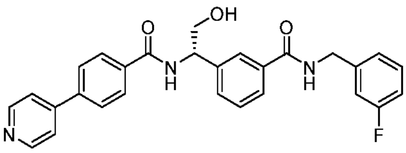

.

In certain aspects, Z1 is pyridine, optionally substituted with one or more of the following: halo, -OH, -CN, -COOR', -OR', -SR', -OC(O)R', -NHR', -NR'R", -NHC(O)R', - NHC(O)NR'R", -C(O)NR'R", -NS(O)2R', -S(O)2NR'R", -S(O)2R', guanidino, nitro, nitroso, - C1-C6 alkyl, aryl, or -C3-C7 cycloalkyl; Z2 is a C5-C10 membered heterocycle, optionally substituted with H, halo, -OH, -CN, -COOR', -OR', -SR', -OC(O)R', -NHR', -NR'R", - NHC(O)R', -NHC(O)NR'R", -C(O)NR'R", -NS(O)2R', -S(O)2NR'R", -S(O)2R', guanidino, nitro, nitroso, -Cl-C6 alkyl, aryl, -C3-C7 cycloalkyl, or a 3 to 10-membered heterocycle; R2 and R3 are independently H, phenyl, benzyl, -C3-C7 cycloalkyl, or -C3-C7 cycloalkyl methyl; wherein the phenyl, benzyl, -C3-C7 cycloalkyl or -C3-C7 cycloalkyl methyl is optionally substituted with H, halo, -OH, -CN, -COOR', -OR', -SR', -OC(O)R', -NHR', -NR'R", -NHC(O)R', -NHC(O)NR'R", -C(O)NR'R", -NS(O)2R', -S(O)2NR'R", -S(O)2R', guanidino, nitro, nitroso, -Cl-C6 alkyl, aryl, -C3-C7 cycloalkyl, or a 3 to 10-membered heterocycle;

with the proviso that R2 and R3 are not both H. In one aspect, R1 and/or R2 are H. In another aspect, Z2 is pyridine or pyrazole.

In another embodiment, the present invention provides a compound selected from the group consisting of

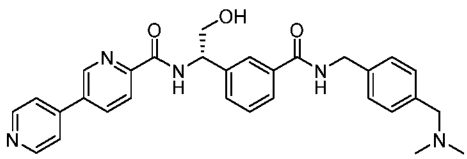

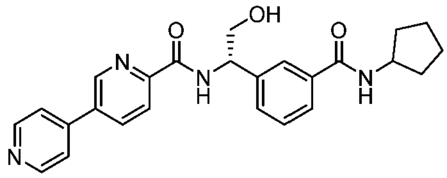

and

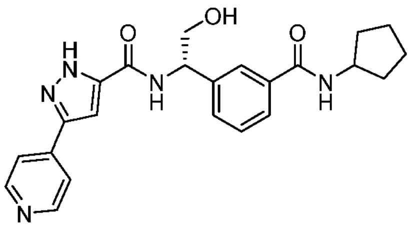

In yet another embodiment, the present invention relates to a compound selected from the group consisting of:

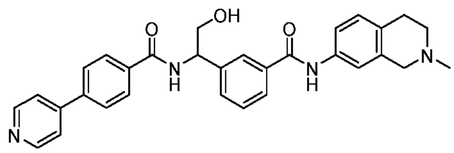

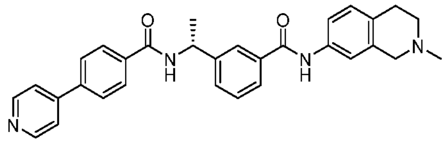

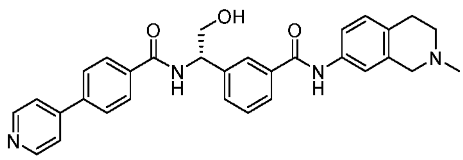

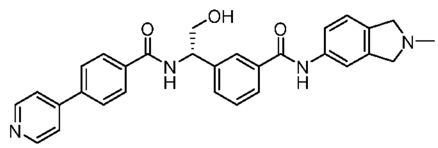

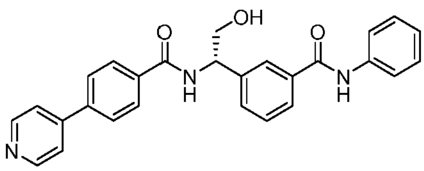

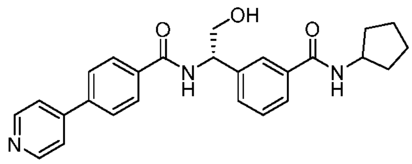

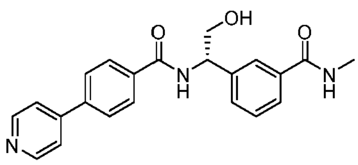

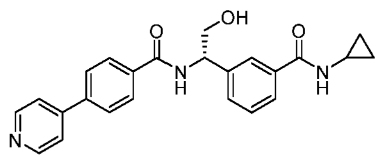

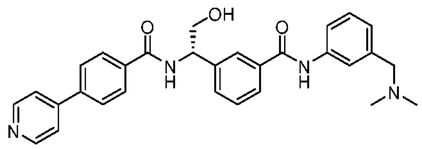
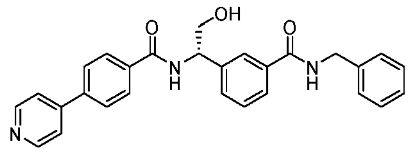

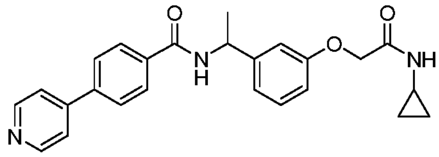
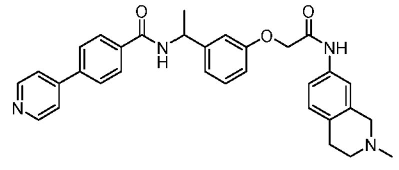

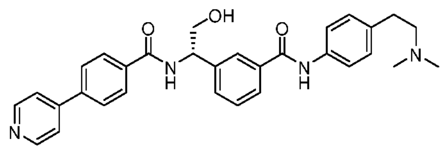

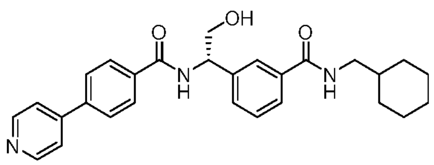
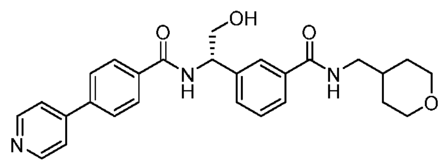

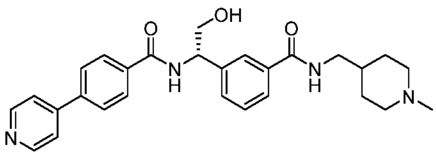
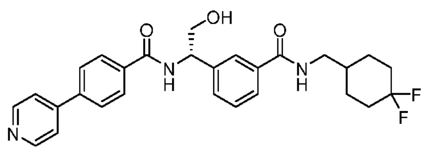

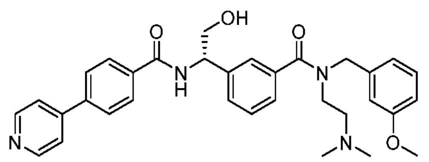
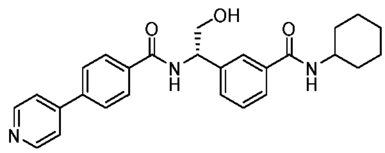

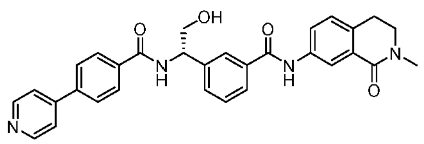
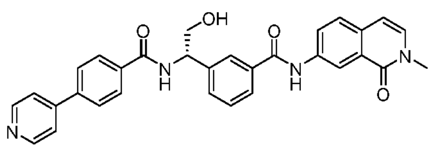

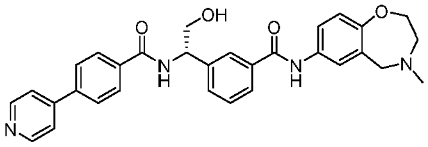
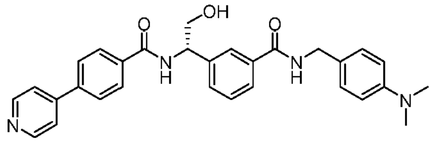

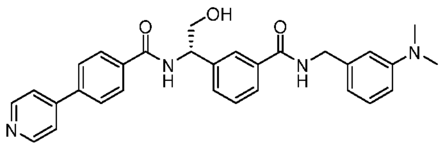
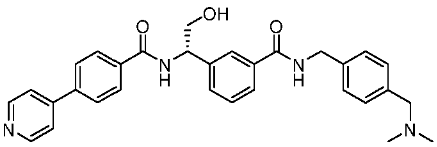

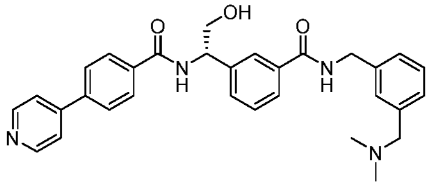

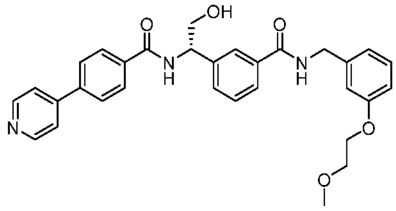

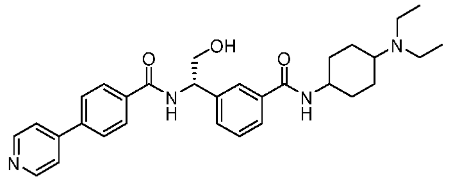

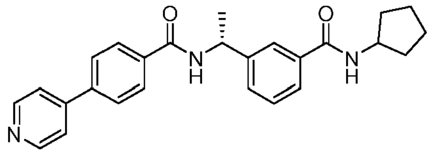

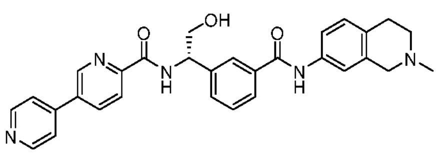

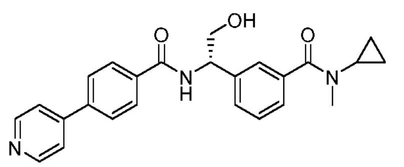

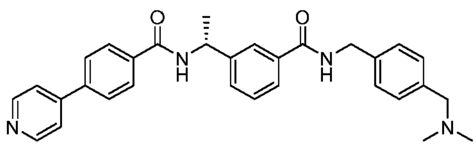

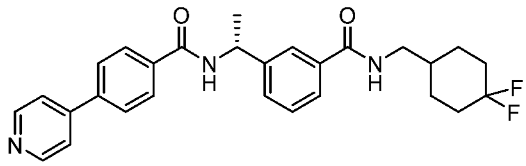

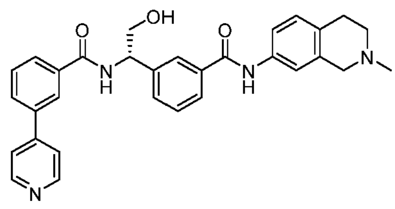

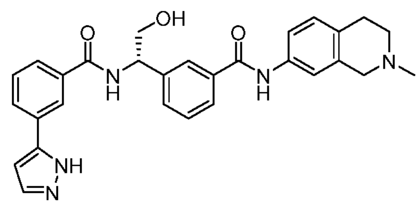

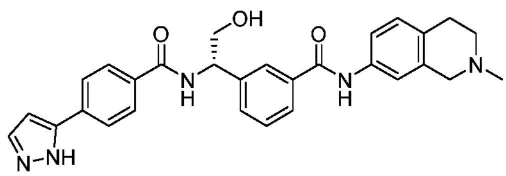

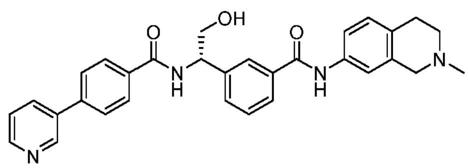

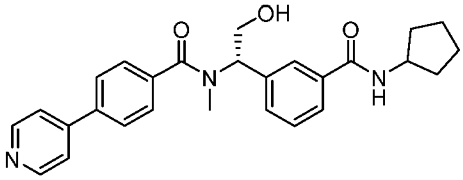

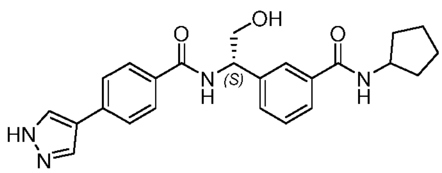

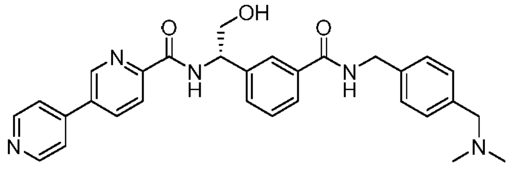

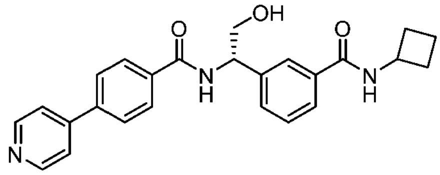

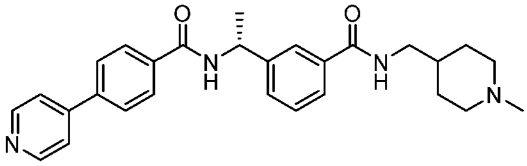

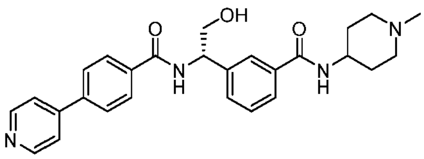

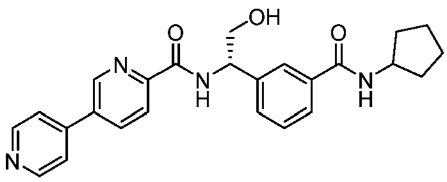

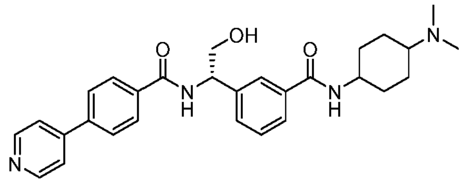

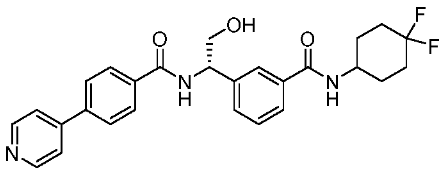

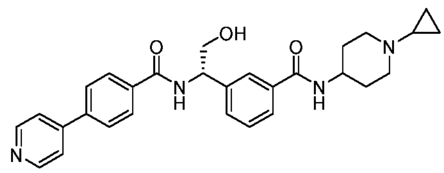

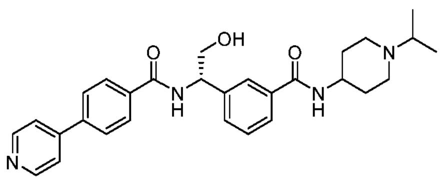

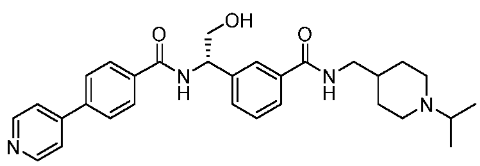

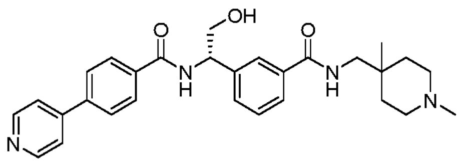

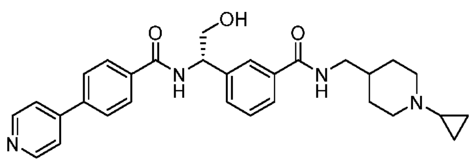

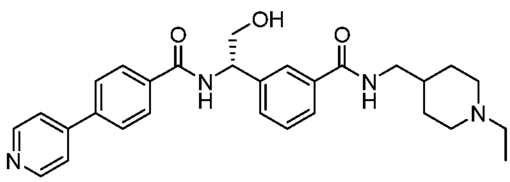

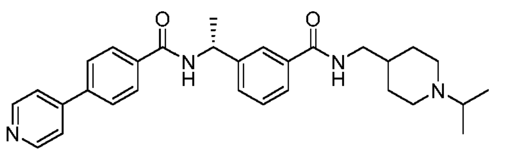

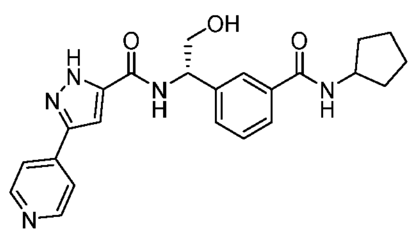

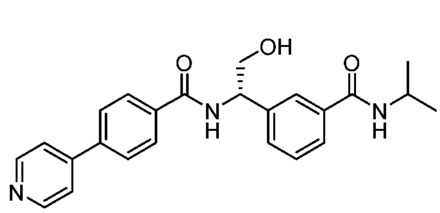

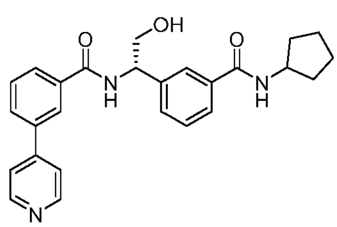

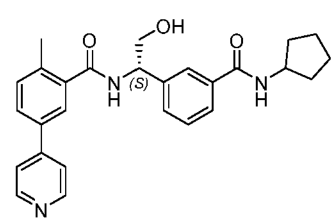

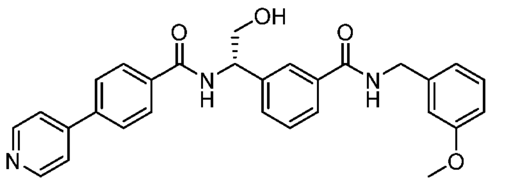

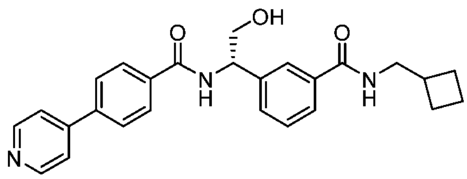

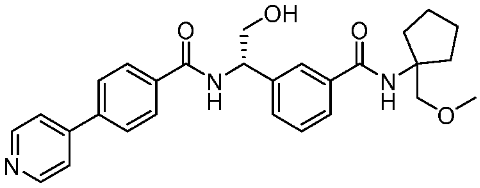

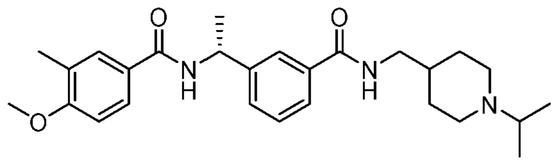

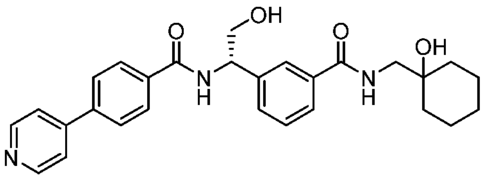

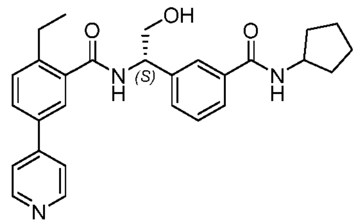

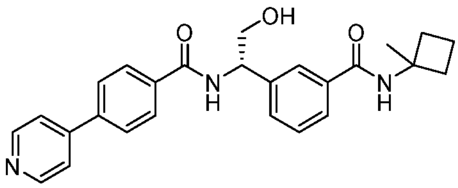

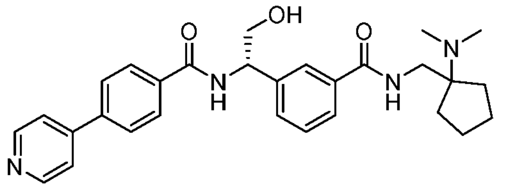

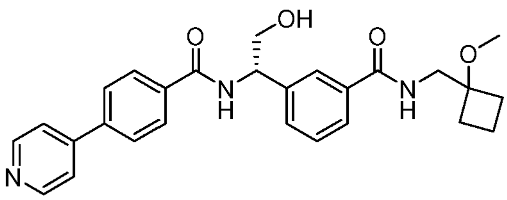

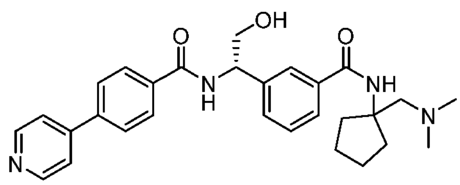

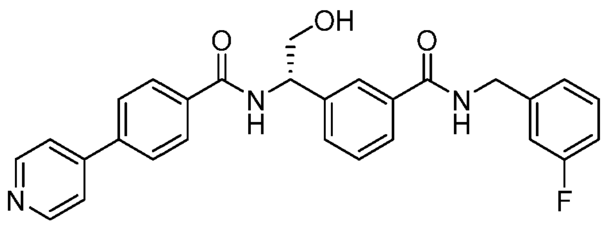

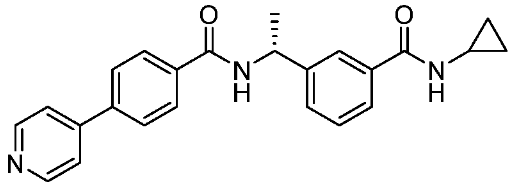

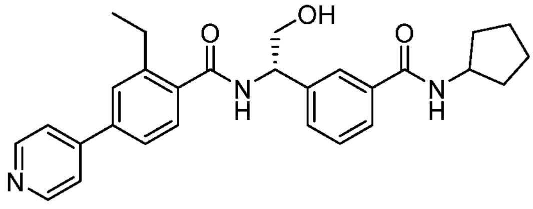

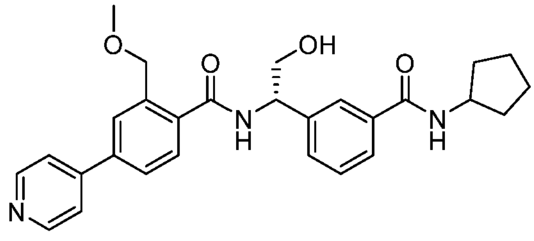

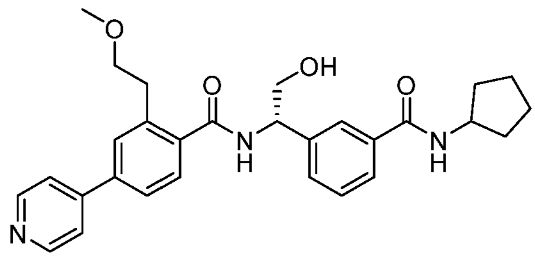

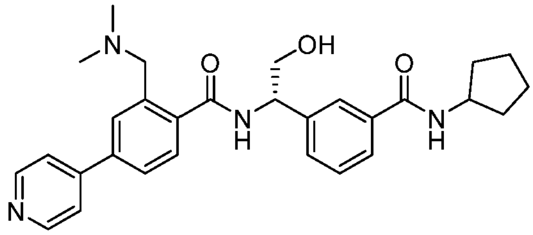

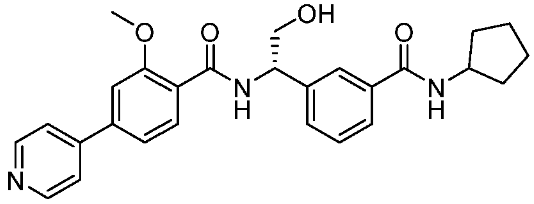

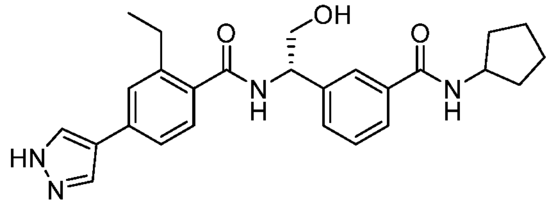

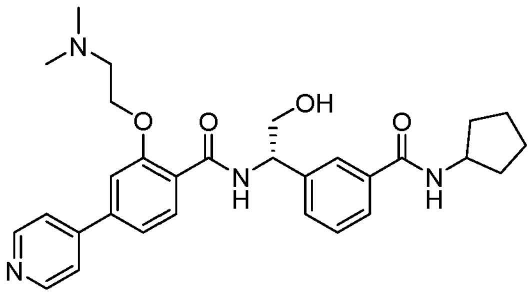

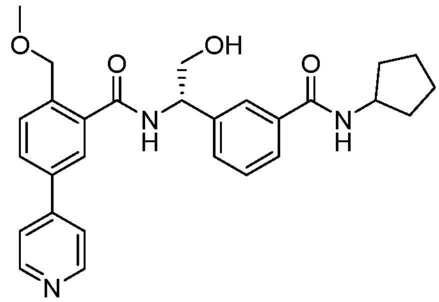

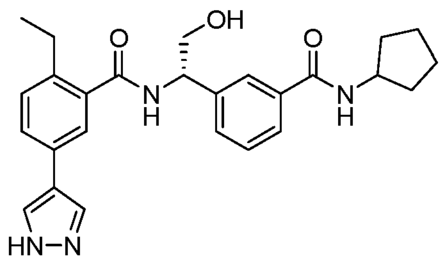

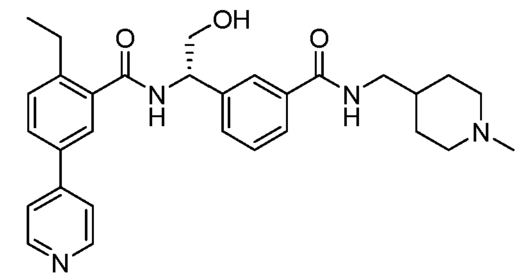

.

In some aspects, the present invention relates to a pharmaceutical composition comprising a compound disclosed herein and a pharmaceutically acceptable carrier.

The disclosure provides a method of treating a disease associated with Rho-associated protein kinase modulation in a subject in need thereof, comprising administering to the subject an effective amount of a compound disclosed herein. The compound may inhibit a Rho-associated protein kinase. The compound may inhibit ROCK1. The compound may inhibit ROCK2.

The disease may be cancer, neurodegenerative disease, neurodevelopmental disorder, inflammatory or autoimmune disease, infection, metabolic disease, hematologic disease, cardiovascular disease, or vascular smooth muscle dysfunction.

The cancer may be cutaneous T-cell lymphoma (CTCL), peripheral T-cell lymphoma (PTCL), angioimmunoblastic T-cell lymphoma (AITL), multiple myeloma, leukemia, lymphoma, or lung, ovarian, breast, prostate, pancreatic, hepatocellular, or renal cancer.

The neurodegenerative disease may be Alzheimer's, Huntington's, Parkinson's, Amyotrophic Lateral Sclerosis, or spinal muscular atrophy.

The neurodevelopmental disorder may be Rett syndrome or Niemann-Pick type C.

The inflammatory or autoimmune disease may be asthma, cardiovascular inflammation, renal inflammation,

arteriosclerosis, rheumatoid arthritis, spondylitis arthritis, psoriatic arthritis, psoriasis, atopic dermatitis, eczema, multiple sclerosis, systemic lupus erythematosus, inflammatory bowel diseases, Crohn's disease, graft versus host disease, transplant rejection, or fibrotic disease.

The cardiovascular disease or vascular smooth muscle dysfunction may be hypertension, atherosclerosis, restenosis, cardiac hypertrophy, ocular hypertension, cerebral ischemia, cerebral vasospasm, or erectile dysfunction.

The disclosure also provides a method of treating a subject suffering from a hematologic malignant neoplastic disorder comprising: identifying the subject in need of therapy for the hematologic malignant neoplastic disorder; and administering to the subject in need of such treatment an effective amount of a compound disclosed herein or a salt thereof to treat the hematologic malignant neoplastic disorder. The hematologic malignant neoplastic disorder may be characterized by deregulated FLT3 receptor tyrosine kinase activity and the malignant neoplastic disorder is selected from the group consisting of leukemia, myeloma, myeloproliferative disease, myelodysplastic syndrome, Hodgkin's disease, myeloma, acute lymphocytic leukemia (ALL), acute myeloid leukemia (AML), acute promyelocytic leukemia (APL), chronic lymphocytic leukemia (CLL), chronic myeloid leukemia (CML), chronic neutrophilic leukemia (CNL), acute undifferentiated leukemia (AUL), anaplastic large-cell lymphoma (ALCL), prolymphocytic leukemia (PML), juvenile myelomonocytic leukemia (JMML), adult T-cell ALL, AML, with trilineage myelodysplasia (AMLITMDS), mixed lineage leukemia (MLL), myelodysplastic syndromes (MDSs), myeloproliferative disorders (MPD), and multiple myeloma (MM).

In one embodiment, the present invention provides a compound as disclosed herein for use in treating or preventing a disease associated with Rho-associated protein kinase modulation. The disclosure also describes use of a compound as disclosed herein in the manufacture of a medicament for treating or preventing a disease associated with Rho-associated protein kinase modulation.

## DETAILED DESCRIPTION

As used herein, halo groups include any halogen. Examples include but are not limited to -F, -Cl, -Br, or -I.

A -$C_1$-$C_6$ alkyl group includes any straight or branched, saturated or unsaturated, substituted or unsubstituted hydrocarbon comprised of between one and six carbon atoms. Examples of -$C_1$-$C_6$ alkyl groups include, but are not limited to methyl, ethyl, propyl, isopropyl, butyl, sec- butyl, tert-butyl, pentyl, isopentyl, neopentyl, hexyl, isohexyl, neohexyl, ethylenyl, propylenyl, 1-butenyl, 2-butenyl, 1-pentenyl, 2-pentenyl, 1-hexenyl, 2-hexenyl, 3-hexenyl, acetylenyl, pentynyl, 1-butynyl, 2-butynyl, 1-pentynyl, 2-pentynyl, 1-hexynyl, 2-hexynyl and 3-hexynyl groups. Substituted -$C_1$-$C_6$ alkyl groups may include any applicable chemical moieties. Examples of groups that may be substituted onto any of the above listed -$C_1$-$C_6$ alkyl groups include but are not limited to the following examples: halo, -$C_1$-$C_6$ alkyl, -O-($C_1$-$C_6$ alkyl), $C_3$-$C_7$ cycloalkyl, -OH, -CN, -COOR', -OC(O)R', -NHR', $N(R')_2$ ,-NHC(O)R' or -C(O)NHR' groups. The groups denoted R' above may be -H, any -$C_1$-$C_6$ alkyl, or two R' may, optionally with a nitrogen or an oxygen atom which they are bound to, form a 3-, 4-, 5-, 6-, 7- membered ring system when the substitution is -$N(R')_2$;

An aryl group includes any unsubstituted or substituted phenyl or napthyl group. Examples of groups that may be substituted onto ay aryl group include, but are not limited to: halo, -$C_1$-$C_6$ alkyl, -O-($C_1$-$C_6$ alkyl), -OH, -CN, -COOR', -OC(O)R', NHR', N (R') 2,- NHC(O), R', or -C(O)NEtR'. The group denoted R' may be -H or any -$C_1$-$C_6$ alkyl.

A $C_3$-$C_7$ cycloalkyl group includes any 3-, 4-, 5-, 6-, or 7-membered substituted or unsubstituted non-aromatic carbocyclic ring. Examples of $C_3$-$C_7$ cycloalkyl groups include, but are not limited to, cyclopropyl, cyclobutyl, cyclopentyl, cyclopentadienyl, cyclohexyl, cyclohexenyl, cycloheptyl, cycloheptanyl, 1,3-cyclohexadienyl,-1, 4- cyclohexadienyl,-!, 3-cycloheptadienyl, and-1,3, 5-cycloheptatrienyl groups. Examples of groups that may be substituted onto $C_3$-$C_7$ cycloalkyl groups include, but are not limited to: -halo, -$C_1$-$C_6$ alkyl, -O-($C_1$-$C_6$ alkyl), -OH, -CN, -COOR', -OC(O) R', NHR', N(R')2, -NHC(O)R' or -C(O)NHR' groups. The groups denoted R' above include an -H or any unsubstituted -$C_1$-$C_6$ alkyl, examples of which are listed above. Halo groups include any halogen. Examples include but are not limited to -F, -Cl, -Br, or -I.

A heterocycle may be any optionally substituted saturated, unsaturated or aromatic cyclic moiety wherein said cyclic moiety is interrupted by at least one heteroatom selected from oxygen (O) , sulfur (S) or nitrogen (N) . Heterocycles may be monocyclic or polycyclic rings. For example, suitable substituents include halogen, halogenated C1-6 alkyl, halogenated C1-6 alkoxy, amino, amidino, amido, azido, cyano, guanidino, hydroxyl , nitro, nitroso, urea, $OS(O)_2R$; $OS(O)_2OR$, $S(O)_2OR$ $S(O)_{0-2}R$, C(O)OR wherein R may be H, $C_1$-$C_6$ alkyl, aryl or 3 to 10 membered heterocycle) $OP(O)OR_1OR_2$, $P(O)OR_1OR_2$, $SO_2NR_1R_2$, $NR_1SO_2R_2$ $C(R_1)NR_2$ $C(R_1)NOR_2$, R1 and R2 may be independently H, $C_1$-$C_6$ alkyl, aryl or 3 to 10 membered heterocycle), $NR_1C(O)R_2$, $NR_1C(O)OR_2$, $NR_3C(O)NR_2R_1$, $C(O)NR_1R_2$, $OC(O)NR_1R_2$. For these groups, $R_1$, $R_2$ and $R_3$ are each independently selected from H, $C_1$-$C_6$ alkyl, aryl or 3 to 10 membered heterocycle or $R_1$ and $R_2$ are taken together with the atoms to which they are attached to form a 3 to 10 membered heterocycle.

Possible substituents of heterocycle groups include halogen (Br, Cl, I or F), cyano, nitro, oxo, amino, C1-4 alkyl (e.g., $CH_3$, $C_2H_5$, isopropyl) $C_{1-4}$ alkoxy (e.g., $OCH_3$, $OC_2H_5$), halogenated $C_{1-4}$ alkyl (e.g., $CF_3$, $CHF_2$), halogenated $C_{14}$ alkoxy (e.g., $OCF_3$, $OC_2F_5$), COOH, COO-$C_{1-4}$ alkyl, CO-$C_{1-4}$ alkyl, $C_{1-4}$ alkyl -S- (e.g., $CH_3S$, $C_2H_5S$), halogenated $C_{1-4}$ alkyl -S-(e.g., $CF_3S$, $C_2F_5S$), benzyloxy, and pyrazolyl.

Examples of heterocycles include but are not limited to azepinyl, aziridinyl, azetyl, azetidinyl, diazepinyl, dithiadiazinyl , dioxazepinyl, dioxolanyl, dithiazolyl, furanyl, isooxazolyl, isothiazolyl, imidazolyl, morpholinyl, morpholino, oxetanyl , oxadiazolyl, oxiranyl, oxazinyl, oxazolyl, piperazinyl, pyrazinyl, pyridazinyl, pyrimidinyl, piperidyl, piperidino, pyridyl, pyranyl, pyrazolyl, pyrrolyl, pyrrolidinyl, thiatriazolyl, tetrazolyl, thiadiazolyl, triazolyl, thiazolyl, thienyl, tetrazinyl, thiadiazinyl , triazinyl, thiazinyl, thiopyranyl furoisoxazolyl, imidazothiazolyl, thienoisothiazolyl, thienothiazolyl, imidazopyrazolyl, cyclopentapyrazolyl, pyrrolopyrrolyl, thienothienyl, thiadiazolopyrimidinyl, thiazolothiazinyl, thiazolopyrimidinyl, thiazolopyridinyl, oxazolopyrimidinyl, oxazolopyridyl, benzoxazolyl, benzisothiazolyl, benzothiazolyl, imidazopyrazinyl, purinyl, pyrazolopyrimidinyl, imidazopyridinyl, benzimidazolyl, indazolyl, benzoxathiolyl, benzodioxolyl, benzodithiolyl, indolizinyl, indolinyl, isoindolinyl, furopyrimidinyl, furopyridyl, benzofuranyl, isobenzofuranyl, thienopyrimidinyl, thienσpyridyl, benzothienyl, cyclopentaoxazinyl, cyclopentafuranyl, benzoxazinyl, benzothiazinyl, quinazolinyl, naphthyridinyl, quinolinyl, isoquinolinyl, benzopyranyl, pyridopyridazinyl and pyridopyrimidinyl groups.

The invention further encompasses any other physiochemical or sterochemical form that the compound may assume. Such forms include diastereomers, racemates, isolated enantiomers, hydrated forms, solvated forms, any known or yet to be disclosed crystalline or amorphous form including all polymorphic crystalline forms. Amorphous forms lack a distinguishable crystal lattice and therefore lack an orderly arrangement of structural units. Many pharmaceutical compounds have amorphous forms. Methods of generating such chemical forms will be well known by one skilled in the art.

Another aspect of the invention is that the carbon atom bearing R or -CH2OH or R4 in Formula I, II, or III may have "S" or "R" configuration. All the diastereomers, racemates, isolated enantiomers are within the scope of the invention.

Racemates, individual enantiomers, or diasteromers of the compound may be prepared by specific synthesis or resolution through any method now known or yet to be disclosed. For example, the compound may be resolved into it enantiomers by the formation of diasteromeric pairs through salt formation using an optically active acid. Enantiomers are fractionally crystallized and the free base regenerated. In another example, enantiomers may be separated by chromatography. Such chromatography may be any appropriate method now known or yet to be disclosed that is appropriate to separate enantiomers such as HPLC on a chiral column.

In some aspects of the invention the compound is in the form of a pharmaceutically acceptable salt. Pharmaceutically acceptable salts include any salt derived from an organic or inorganic acid. Examples of such salts include but are not limited to the following: salts of hydrobromic acid, hydrochloric acid, nitric acid, phosphoric acid, and sulphuric acid. Organic acid addition salts include, for example, salts of acetic acid, benzenesulphonic acid, benzoic acid, camphorsulphonic acid, citric acid, 2- (4-chlorophenoxy)-2- methylpropionic acid, 1, 2-ethanedisulphonic acid, ethanesulphonic acid, ethylenediaminetetraacetic acid (EDTA), fumaric acid, glucoheptonic acid, gluconic acid, glutamic acid, N-glycolylarsanilic acid, 4-hexylresorcinol, hippuric acid, 2- (4-hydroxybenzoyl) benzoicacid, 1-hydroxy-2-naphthoicacid, 3-hydroxy- 2-naphthoic acid, 2-hydroxyethanesulphonic acid, lactobionic acid, n-dodecyl sulphuric acid, maleic acid, malic acid, mandelic acid, methanesulphonic acid, methyl sulpuric acid, mucic acid, 2-naphthalenesulphonic acid, pamoic acid, pantothenic acid, phosphanilic acid ((4-aminophenyl) phosphonic acid), picric acid, salicylic acid, stearic acid, succinic acid, tannic acid, tartaric acid, terephthalic acid, p-toluenesulphonic acid, 10-undecenoic acid or any other such acid now known or yet to be disclosed. It will be appreciated that such salts, provided that they are pharmaceutically acceptable, may be used in therapy. Such salts may be prepared by reacting the compound with a suitable acid in a manner known by those skilled in the art.

Examples that represent different aspects of the invention follow. Such examples should not be construed as limiting the scope of the disclosure. Alternative mechanistic pathways and analogous structures within the scope of the invention would be apparent to those skilled in the art.

Elements and acts in the examples are intended to illustrate the invention for the sake of simplicity and have not necessarily been rendered according to any particular sequence or embodiment.

The articles "a" and "an" are used in this disclosure to refer to one or more than one (e.g., to at least one) of the grammatical object of the article. By way of example, "an element" means one element or more than one element.

The term "and/or" is used in this disclosure to mean either "and" or "or" unless indicated otherwise.

The term "optionally substituted" is understood to mean that a given chemical moiety (e.g., an alkyl group) can (but is not required to) be bonded other substituents (e.g., heteroatoms). For instance, an alkyl group that is optionally substituted can be a fully saturated alkyl chain (e.g., a pure hydrocarbon). Alternatively, the same optionally substituted alkyl group can have substituents different from hydrogen. For instance, it can, at any point along the chain be bounded to a halogen atom, a hydroxyl group, or any other substituent described herein. Thus the term "optionally substituted" means that a given chemical moiety has the potential to contain other functional groups, but does not necessarily have any further functional groups.

The term "aryl" refers to cyclic, aromatic hydrocarbon groups that have 1 to 2 aromatic rings, including monocyclic or bicyclic groups such as phenyl, biphenyl or naphthyl. Where containing two aromatic rings (bicyclic, etc.), the aromatic rings of the aryl group may be joined at a single point (e.g., biphenyl), or fused (e.g., naphthyl). The aryl group may be optionally substituted by one or more substituents, e.g., 1 to 5 substituents, at any point of attachment. Exemplary substituents include, but are not limited to, -H, -halogen, -0-Ci-$C_6$ alkyl, -C $C_6$ alkyl, -$OC_2$-$C_6$ alkenyl, -$OC_2$-$C_6$ alkynyl,

$-C_2-C_6$ alkenyl, $-C_2-C_6$ alkynyl, -OH, $-OP(0)(OH)_2$, - OC(0)Ci-$C_6$ alkyl, -C(0)Ci-$C_6$ alkyl, -OC(0)OCi-$C_6$ alkyl, - $H_2$, - H(Ci-$C_6$ alkyl), -N(Ci-$C_6$ alkyl$)_2$, $-S(0)_2$-Ci-$C_6$ alkyl, -S(0) HCi-$C_6$ alkyl, and -S(0)N(Ci-$C_6$ alkyl$)_2$. The substituents can themselves be optionally substituted. Furthermore when containing two fused rings the aryl groups herein defined may have an unsaturated or partially saturated ring fused with a fully saturated ring. Exemplary ring systems of these aryl groups include indanyl, indenyl, tetrahydronaphthalenyl, and tetrahydrobenzoannulenyl.

Unless otherwise specifically defined, "heteroaryl" means a monovalent monocyclic aromatic radical of 5 to 24 ring atoms or a polycyclic aromatic radical, containing one or more ring heteroatoms selected from N, S, P, and O, the remaining ring atoms being C. Heteroaryl as herein defined also means a bicyclic heteroaromatic group wherein the heteroatom is selected from N, S, P, and O. The aromatic radical is optionally substituted independently with one or more substituents described herein. Examples include, but are not limited to, furyl, thienyl, pyrrolyl, pyridyl, pyrazolyl, pyrimidinyl, imidazolyl, isoxazolyl, oxazolyl, oxadiazolyl, pyrazinyl, indolyl, thiophen-2-yl, quinolyl, benzopyranyl, isothiazolyl, thiazolyl, thiadiazole, indazole, benzimidazolyl, thieno[3,2-b]thiophene, triazolyl, triazinyl, imidazo[1,2-b]pyrazolyl, furo[2,3-c]pyridinyl, imidazo[1,2-a]pyridinyl, indazolyl, pyrrolo[2,3-c]pyridinyl, pyrrolo[3,2-c]pyridinyl, pyrazolo[3,4-c]pyridinyl, thieno[3,2-c]pyridinyl, thieno[2,3-c]pyridinyl, thieno[2,3-b]pyridinyl, benzothiazolyl, indolyl, indolinyl, indolinonyl, dihydrobenzothiophenyl, dihydrobenzofuranyl, benzofuran, chromanyl, thiochromanyl, tetrahydroquinolinyl, dihydrobenzothiazine, dihydrobenzoxanyl, quinolinyl, isoquinolinyl, 1,6-naphthyridinyl, benzo[de]isoquinolinyl, pyrido[4,3-b][1,6]naphthyridinyl, thieno[2,3-b]pyrazinyl, quinazolinyl, tetrazolo[1,5-a]pyridinyl, [1,2,4]triazolo[4,3-a]pyridinyl, isoindolyl, pyrrolo[2,3-b]pyridinyl, pyrrolo[3,4-b]pyridinyl, pyrrolo[3,2-b]pyridinyl, imidazo[5,4-b]pyridinyl, pyrrolo[1,2-a] pyrimidinyl, tetrahydro pyrrolo[1,2-a]pyrimidinyl, 3,4-dihydro-2H-$1^2$-pyrrolo[2,1-b] pyrimidine, dibenzo[b,d] thiophene, pyridin-2-one, furo[3,2-c]pyridinyl, furo[2,3-c]pyridinyl, 1H-pyrido[3,4-b][1,4] thiazinyl, benzooxazolyl, benzoisoxazolyl, furo[2,3-b]pyridinyl, benzothiophenyl, 1,5-naphthyridinyl, furo[3,2-b]pyridine, [1,2,4]triazolo[1,5-a]pyridinyl, benzo [ 1 ,2,3 Jtriazolyl, imidazo[ 1 ,2-a]pyrimidinyl, [ 1 ,2,4]triazolo[4,3 -b]pyridazinyl, benzo[c] [ 1 ,2, 5]thiadiazolyl, benzo[c] [ 1 ,2, 5]oxadiazole, 1 ,3 -dihydro-2H-benzo[d]imidazol-2-one, 3,4-dihydro-2H-pyrazolo [1,5-b][1,2]oxazinyl, 4,5,6,7-tetrahydropyrazolo[1,5-a]pyridinyl, thiazolo[5,4-d]thiazolyl, imidazo[2,1-b][1,3,4]thiadiazolyl, thieno[2,3-b]pyrrolyl, 3H-indolyl, and derivatives thereof.

Furthermore when containing two fused rings the heteroaryl groups herein defined may have an unsaturated or partially saturated ring fused with a fully saturated ring. Exemplary ring systems of these heteroaryl groups include indolinyl, indolinonyl, dihydrobenzothiophenyl, dihydrobenzofuran, chromanyl, thiochromanyl, tetrahydroquinolinyl, dihydrobenzothiazine, 3,4-dihydro-1H~isoquinolinyl, 2,3 -dihydrobenzofuran, indolinyl, indolyl, and dihydrobenzoxanyl.

"Alkyl" refers to a straight or branched chain saturated hydrocarbon. Ci-$C_6$ alkyl groups contain 1 to 6 carbon atoms. Examples of a Ci-$C_6$ alkyl group include, but are not limited to, methyl, ethyl, propyl, butyl, pentyl, isopropyl, isobutyl, sec-butyl and tert-butyl, isopentyl and neopentyl.

The term "alkenyl" means an aliphatic hydrocarbon group containing a carbon- carbon double bond and which may be straight or branched having about 2 to about 6 carbon atoms in the chain. Alkenyl groups can have 2 to about 4 carbon atoms in the chain. Branched means that one or more lower alkyl groups such as methyl, ethyl, or propyl are attached to a linear alkenyl chain. Exemplary alkenyl groups include ethenyl, propenyl, n-butenyl, and i-butenyl. A $C_2-C_6$ alkenyl group is an alkenyl group containing between 2 and 6 carbon atoms.

The term "alkynyl" means an aliphatic hydrocarbon group containing a carbon- carbon triple bond and which may be straight or branched having about 2 to about 6 carbon atoms in the chain. Alkynyl groups can have 2 to about 4 carbon atoms in the chain. Branched means that one or more lower alkyl groups such as methyl, ethyl, or propyl are attached to a linear alkynyl chain. Exemplary alkynyl groups include ethynyl, propynyl, n-butynyl, 2-butynyl, 3-methylbutynyl, and n-pentynyl. A $C_2-C_6$ alkynyl group is an alkynyl group containing between 2 and 6 carbon atoms.

The term "cycloalkyl" means monocyclic or polycyclic saturated carbon rings containing 3-18 carbon atoms. Examples of cycloalkyl groups include, without limitations, cyclopropyl, cyclobutyl, cyclopentyl, cyclohexyl, cycloheptanyl, cyclooctanyl, norboranyl, norborenyl, bicyclo[2.2.2]octanyl, or bicyclo[2.2.2]octenyl. A $C_3-C_8$ cycloalkyl is a cycloalkyl group containing between 3 and 8 carbon atoms. A cycloalkyl group can be fused (e.g., decalin) or bridged (e.g., norbornane).

The term "cycloalkenyl" means monocyclic, non-aromatic unsaturated carbon rings containing 3-18 carbon atoms. Examples of cycloalkenyl groups include, without limitation, cyclopentenyl, cyclohexenyl, cycloheptenyl, cyclooctenyl, and norborenyl. A $C_3-C_8$ cycloalkenyl is a cycloalkenyl group containing between 3 and 8 carbon atoms.

The terms "heterocyclyl" or "heterocycloalkyl" or "heterocycle" refer to monocyclic or polycyclic 3 to 24-membered rings containing carbon and heteroatoms taken from oxygen, nitrogen, or sulfur and wherein there is not delocalized $\pi$ electrons (aromaticity) shared among the ring carbon or heteroatoms. Heterocyclyl rings include, but are not limited to, oxetanyl, azetadinyl, tetrahydrofuranyl, pyrrolidinyl, oxazolinyl, oxazolidinyl, thiazolinyl, thiazolidinyl, pyranyl, thiopyranyl, tetrahydropyranyl, dioxalinyl, piperidinyl, morpholinyl, thiomorpholinyl, thiomorpholinyl S-oxide, thiomorpholinyl S-dioxide, piperazinyl, azepinyl, oxepinyl, diazepinyl, tropanyl, and homotropanyl. A heterocyclyl or heterocycloalkyl ring can also be fused or bridged, e.g., can be a bicyclic ring.

As used herein, the term "halo" or "halogen" means fluoro, chloro, bromo, or iodo.

The term "carbonyl" refers to a functional group composing a carbon atom double-bonded to an oxygen atom. It can be abbreviated herein as "oxo", as C(O), or as C=0.

"Spirocycle" or "spirocyclic" means carbogenic bicyclic ring systems with both rings connected through a single atom. The ring can be different in size and nature, or identical in size and nature. Examples include spiropentane, spirohexane, spiroheptane, spirooctane, spirononane, or spirodecane. One or both of the rings in a spirocycle can be fused to another ring carbocyclic, heterocyclic, aromatic, or heteroaromatic ring. One or more of the carbon atoms in the spirocycle can be substituted with a heteroatom (e.g., O, N, S, or P). A C3-$C_{12}$ spirocycle is a spirocycle containing between 5 and 12 carbon atoms. One or more of the carbon atoms can be substituted with a heteroatom.

The term "spirocyclic heterocycle" or "spiroheterocycle" is understood to mean a spirocycle wherein at least one of the rings is a heterocycle (e.g., at least one of the rings is furanyl, morpholinyl, or piperadinyl).

The disclosure also includes pharmaceutical compositions comprising an effective amount of a disclosed compound and a pharmaceutically acceptable carrier. Representative "pharmaceutically acceptable salts" include, e.g., water-soluble and water-insoluble salts, such as the acetate, amsonate (4,4-diaminostilbene-2,2-disulfonate), benzenesulfonate, benzonate, bicarbonate, bisulfate, bitartrate, borate, bromide, butyrate, calcium, calcium edetate, camsylate, carbonate, chloride, citrate, clavulariate, dihydrochloride, edetate, edisylate, estolate, esylate, fumarate, gluceptate, gluconate, glutamate, glycollylarsanilate, hexafluorophosphate, hexylresorcinate, hydrabamine, hydrobromide, hydrochloride, hydroxynaphthoate, iodide, isethionate, lactate, lactobionate, laurate, magnesium, malate, maleate, mandelate, mesylate, methyl bromide, methyl nitrate, methyl sulfate, mucate, napsylate, nitrate, N-methylglucamine ammonium salt, 3 -hydroxy -2-naphthoate, oleate, oxalate, palmitate, pamoate (1,1-methene-bis-2-hydroxy-3-naphthoate, einbonate), pantothenate, phosphate/diphosphate, picrate, polygalacturonate, propionate, p-toluenesulfonate, salicylate, stearate, subacetate, succinate, sulfate, subsalicylate, suramate, tannate, tartrate, teoclate, tosylate, triethiodide, and valerate salts.

The term "stereoisomers" refers to the set of compounds which have the same number and type of atoms and share the same bond connectivity between those atoms, but differ in three dimensional structure. The term "stereoisomer" refers to any member of this set of compounds.

The term "diastereomers" refers to the set of stereoisomers which cannot be made superimposable by rotation around single bonds. For example, cis- and trans- double bonds, endo- and exo- substitution on bicyclic ring systems, and compounds containing multiple stereogenic centers with different relative configurations are considered to be diastereomers. The term "diastereomer" refers to any member of this set of compounds. In some examples presented, the synthetic route may produce a single diastereomer or a mixture of diastereomers. In some cases these diastereomers were separated and in other cases a wavy bond is used to indicate the structural element where configuration is variable.

The term "enantiomers" refers to a pair of stereoisomers which are non-superimposable mirror images of one another. The term "enantiomer" refers to a single member of this pair of stereoisomers. The term "racemic" refers to a 1 : 1 mixture of a pair of enantiomers.

The term "tautomers" refers to a set of compounds that have the same number and type of atoms, but differ in bond connectivity and are in equilibrium with one another. A "tautomer" is a single member of this set of compounds. Typically a single tautomer is drawn but it is understood that this single structure is meant to represent all possible tautomers that might exist. Examples include enol-ketone tautomerism. When a ketone is drawn it is understood that both the enol and ketone forms are part of the disclosure.

An "effective amount" when used in connection with a compound is an amount effective for treating or preventing a disease in a subject as described herein.

The term "carrier," as used in this disclosure, encompasses carriers, excipients, and diluents and means a material, composition or vehicle, such as a liquid or solid filler, diluent, excipient, solvent or encapsulating material, involved in carrying or transporting a pharmaceutical agent from one organ, or portion of the body, to another organ, or portion of the body of a subject.

The term "treating" with regard to a subject, refers to improving at least one symptom of the subject's disorder. Treating includes curing, improving, or at least partially ameliorating the disorder.

The term "disorder" is used in this disclosure to mean, and is used interchangeably with, the terms disease, condition, or illness, unless otherwise indicated.

The term "administer," "administering," or "administration" as used in this disclosure refers to either directly administering a disclosed compound or pharmaceutically acceptable salt of the disclosed compound or a composition to a subject, or administering a prodrug derivative or analog of the compound or pharmaceutically acceptable salt of the compound or composition to the subject, which can form an equivalent amount of active compound within the subject's body.

The term "prodrug," as used in this disclosure, means a compound which is convertible in vivo by metabolic means (e.g., by hydrolysis) to a disclosed compound. Furthermore, as used herein a prodrug is a drug which is inactive in the body, but is transformed in the body typically either during absorption or after absorption from the gastrointestinal tract into the active compound. The conversion of the prodrug into the active compound in the body may be done

chemically or biologically (e.g., using an enzyme).

The term "solvate" refers to a complex of variable stoichiometry formed by a solute and solvent. Such solvents for the purpose of the disclosure may not interfere with the biological activity of the solute. Examples of suitable solvents include, but are not limited to, water, MeOH, EtOH, and AcOH. Solvates wherein water is the solvent molecule are typically referred to as hydrates. Hydrates include compositions containing stoichiometric amounts of water, as well as compositions containing variable amounts of water.

The term "isomer" refers to compounds that have the same composition and molecular weight but differ in physical and/or chemical properties. The structural difference may be in constitution (geometric isomers) or in the ability to rotate the plane of polarized light (stereoisomers). With regard to stereoisomers, the disclosed compounds may have one or more asymmetric carbon atom and may occur as racemates, racemic mixtures and as individual enantiomers or diastereomers.

A "patient" or "subject" is a mammal, e.g., a human, mouse, rat, guinea pig, dog, cat, horse, cow, pig, or non-human primate, such as a monkey, chimpanzee, baboon or rhesus.

As used herein, the terms "FLT3 mutated proliferative disorder(s)," "disorder related to FLT3," "disorders related to FLT3 receptor," "disorders related to FLT3 receptor tyrosine kinase," "a deregulated FLT3 receptor tyrosine kinase disease," or "FLT3 driven cell proliferative disorder" include diseases associated with or implicating FLT3 activity, for example, mutations leading to constitutive activation of FLT3. Examples of "FLT3 mutated proliferative disorder(s)" include disorders resulting from over stimulation of FLT3 due to mutations in FLT3, or disorders resulting from abnormally high amount of FLT3 activity due to abnormally high amount of mutations in FLT3. It is known that over-activity of FLT3 has been implicated in the pathogenesis of many diseases, including the following listed cell proliferative disorders, neoplastic disorders, and cancers. Non-limiting examples of proliferative disorders for treatment with the present invention include leukemia, myeloma, myeloproliferative disease, myelodysplastic syndrome, idiopathic hypereosinophilic syndrome (HES), bladder cancer, breast cancer, cervical cancer, CNS cancer, colon cancer, esophageal cancer, head and neck cancer, liver cancer, lung cancer, nasopharyngeal cancer, neuroendocrine cancer, ovarian cancer, pancreatic cancer, prostate cancer, renal cancer, salivary gland cancer, small cell lung cancer, skin cancer, stomach cancer, testicular cancer, thyroid cancer, uterine cancer, and hematologic malignancy.

As used herein, the terms "proliferative disorder(s)" and "cell proliferative disorder(s)" refer to excess cell proliferation of one or more subset of cells in a multicellular organism resulting in harm (i.e. discomfort or decreased life expectancy) to the multicellular organism. Cell proliferative disorders can occur in different types of animals and humans. As used herein, "cell proliferative disorders" include neoplastic disorders.

As used herein, the term "neoplastic disorder" refers to a tumor resulting from abnormal or uncontrolled cellular growth. Examples of neoplastic disorders include, but are not limited to the following disorders, for instance: the myeloproliferative disorders, such as thrombocytopenia, essential thrombocytosis (ET), agnogenic myeloid metaplasia, myelofibrosis (MF), myelofibrosis with myeloid metaplasia (MMM), chronic idiopathic myelofibrosis (UIMF), and polycythemia vera (PV), the cytopenias, and pre-malignant myelodysplastic syndromes; cancers such as glioma cancers, lung cancers, breast cancers, colorectal cancers, prostate cancers, gastric cancers, esophageal cancers, colon cancers, pancreatic cancers, ovarian cancers, and hematological malignancies, including myelodysplasia, multiple myeloma, leukemias, and lymphomas. Examples of hematological malignancies include, for instance, leukemias, lymphomas, Hodgkin’s disease, and myeloma. Also, acute lymphocytic leukemia (ALL), acute myeloid leukemia (AML), acute promyelocytic leukemia (APL), chronic lymphocytic leukemia (CLL), chronic myeloid leukemia (CML), chronic neutrophilic leukemia (CNL), acute undifferentiated leukemia (AUL), anaplastic large-cell lymphoma (ALCL), prolymphocytic leukemia (PML), juvenile myelomonocytic leukemia (JMML), adult T-cell ALL, AML, with trilineage myelodysplasia (AMLITMDS), mixed lineage leukemia (MLL), myelodysplastic syndromes (MDSs), myeloproliferative disorders (MPD), and multiple myeloma (MM). In certain embodiments, the present invention is directed at the use of a compound disclosed herein or a pharmaceutically acceptable salt thereof in an amount sufficient for the treatment of a neoplastic disorder.

Yet another embodiment of the present invention includes a method for specifically inhibiting a deregulated receptor tyrosine kinase comprising: obtaining a patient sample and determining which receptor tyrosine kinases are deregulated; and administering to a mammal in need of such treatment an effective amount of a compound disclosed herein or a salt thereof, wherein the deregulated receptor tyrosine kinase is a FLT3 receptor tyrosine kinase. In one aspect, the effective amount of a compound disclosed herein or a salt thereof is provided in an amount that decreases patient circulating peripheral blood blast count. In another aspect, the effective amount of a compound disclosed herein or a salt thereof is provided in an amount that decreases a patient bone marrow blast count. In another aspect, the proliferative disease is selected from at least one of a leukemia, myeloma, myeloproliferative disease, myelodysplastic syndrome, idiopathic hypereosinophilic syndrome (HES), bladder cancer, breast cancer, cervical cancer, CNS cancer, colon cancer, esophageal cancer, head and neck cancer, liver cancer, lung cancer, nasopharyngeal cancer, neuroendocrine cancer, ovarian cancer, pancreatic cancer, prostate cancer, renal cancer, salivary gland cancer, small cell lung cancer, skin cancer, stomach cancer, testicular cancer, thyroid cancer, uterine cancer, and hematologic malignancy.

In another embodiment of the disclosure, the disclosed compounds are enantiomers. In some embodiments

the compounds are the (S)-enantiomer. In other embodiments the compounds are the (R)-enantiomer. In some embodiment, the (R)- or (S)-enantiomeric configuration may be assigned to each molecule. In other embodiments, the (R)- or (S)-enantiomeric configuration may not be assigned to the molecules despite the enantiomeric purification or separation of the molecules. In yet other embodiments, the disclosed compounds may be (+) or (-) enantiomers.

It should be understood that all isomeric forms are included within the present disclosure, including mixtures thereof. If the compound contains a double bond, the substituent may be in the E or Z configuration or cis or trans configuration. If the compound contains a disubstituted cycloalkyl, the cycloalkyl substituent may have a cis- or trans configuration. All tautomeric forms are also intended to be included. In some embodiments, the cis or trans configuration may be assigned to each molecule. In other embodiments, the cis or trans configuration may not be assigned to the molecules despite the chemical purification or separation of the diastereomers.

**Table 1.** Non-limiting examples of kinase inhibitor compounds. Unless specified, example compounds with a chiral center represent racemic mixture of the corresponding *R* and *S* enantiomers and all racemates and isolated enantiomers are within the scope of the invention (unless indicated otherwise below).

| ID | Structure | M+1 |
|---|---|---|
| 1 (not according to the invention) | 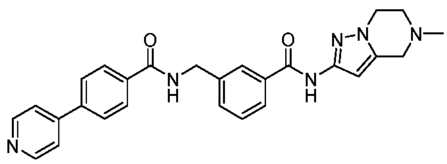 | 467 |
| 2 | 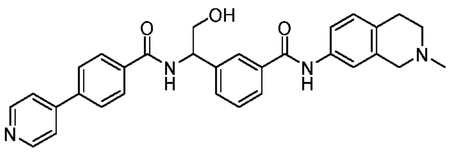 | 507 |
| 3 (not according to the invention) | 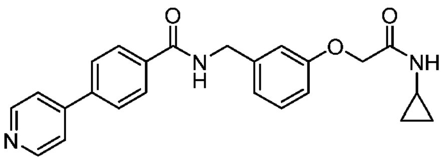 | 402 |
| 4 (not according to the invention) | 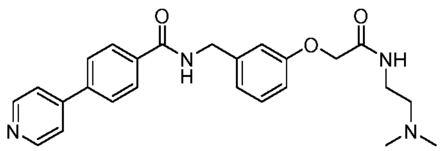 | 433 |
| 5 (not according to the invention) | 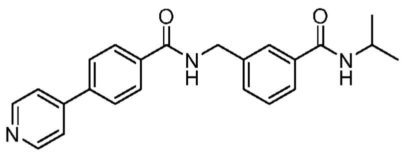 | 374 |
| 6 | 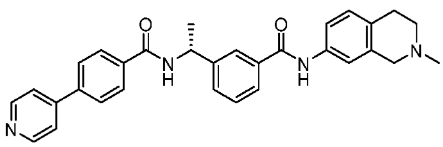 | 491 |
| 7 | 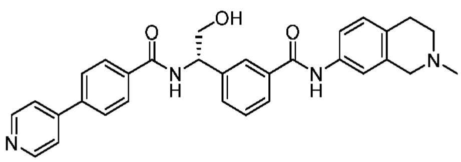 | 507 |

(continued)

| ID | Structure | M+1 |
|---|---|---|
| 8 | 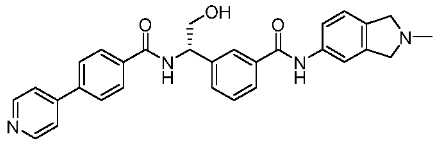 | 493 |
| 9 | 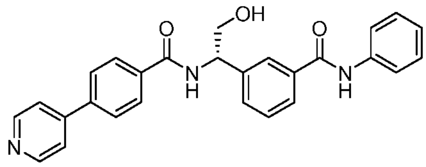 | 438 |
| 10 | 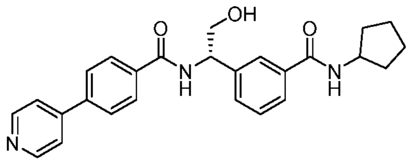 | 430 |
| 11 | 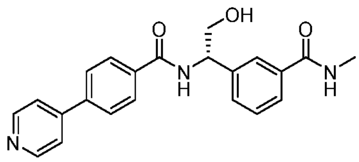 | 376 |
| 12 | 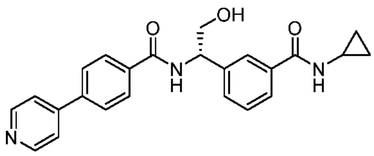 | 402 |
| 13 | 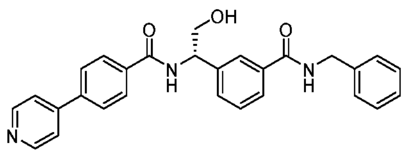 | 452 |
| 14 | 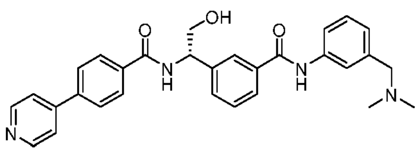 | 495 |
| 15 | 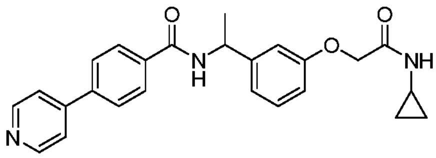 | 416 |
| 16 | 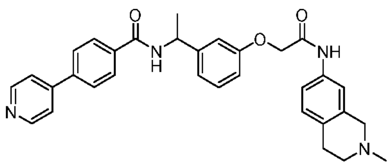 | 521 |

(continued)

| ID | Structure | M+1 |
|---|---|---|
| 17 (not according to the invention) | 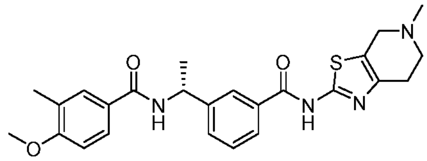 | 465 |
| 18 (not according to the invention) | 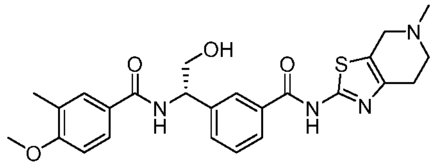 | 481 |
| 19 (not according to the invention) | 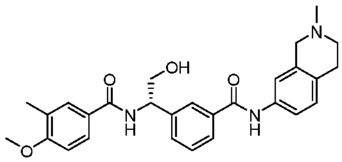 | 474 |
| 20 | 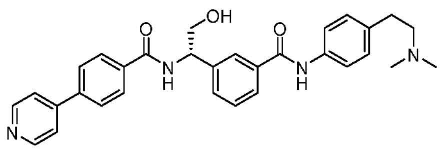 | 509 |
| 21 | 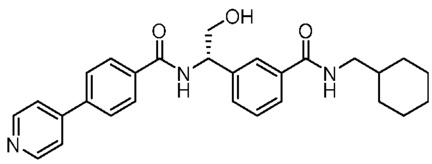 | 458 |
| 22 | 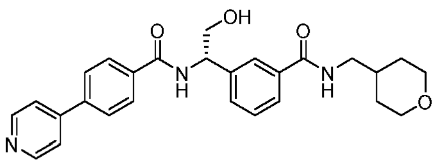 | 460 |
| 23 | 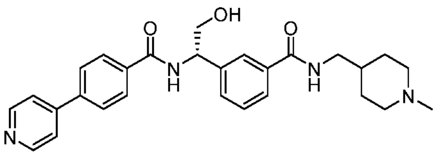 | 473 |
| 24 | 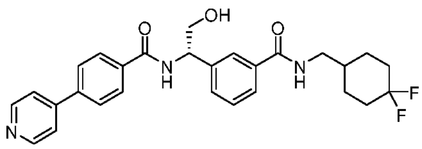 | 494 |
| 25 | 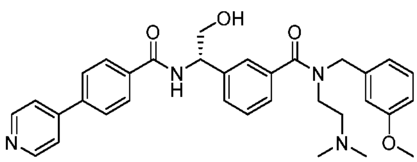 | 553 |

(continued)

| ID | Structure | M+1 |
|---|---|---|
| 26 | 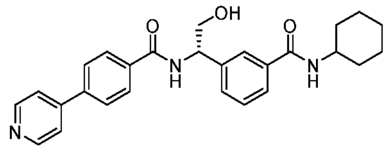 | 444 |
| 27 | 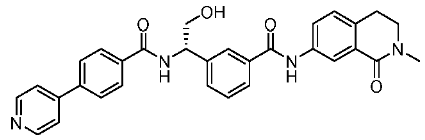 | 521 |
| 28 | 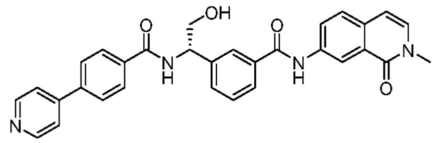 | 519 |
| 29 | 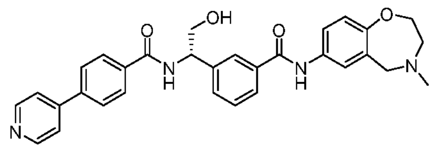 | 523 |
| 30 | 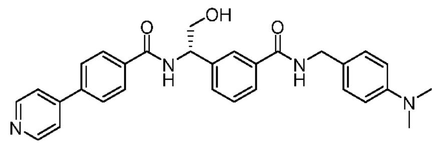 | 495 |
| 31 | 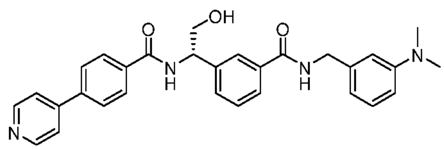 | 495 |
| 32 | 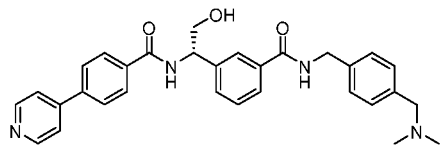 | 509 |
| 33 | 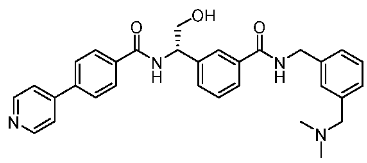 | 509 |
| 34 | 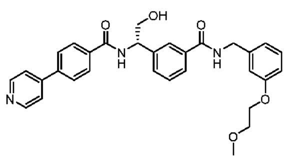 | 526 |

(continued)

| ID | Structure | M+1 |
|---|---|---|
| 35 | 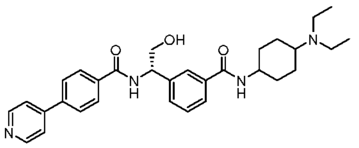 | 515 |
| 36 | 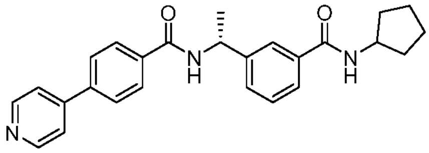 | 413 |
| 37 | 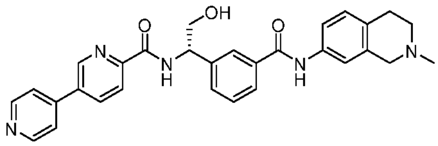 | 508 |
| 38 | 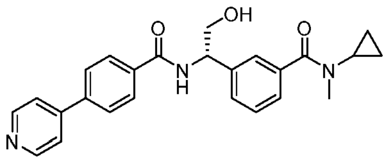 | 416 |
| 39 | 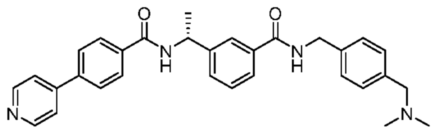 | 493 |
| 40 | 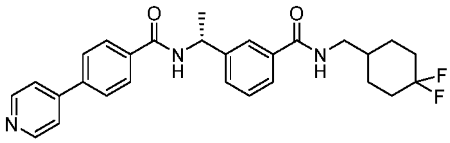 | 478 |
| 41 | 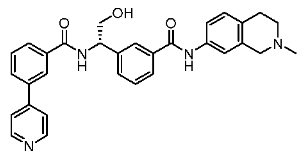 | 507 |
| 42 | 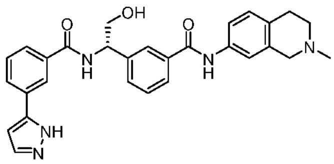 | 496 |
| 43 | 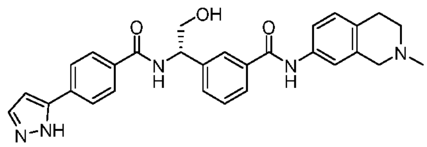 | 496 |

(continued)

| ID | Structure | M+1 |
|---|---|---|
| 44 | 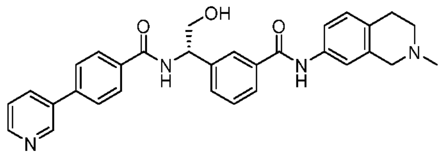 | 507 |
| 45 | 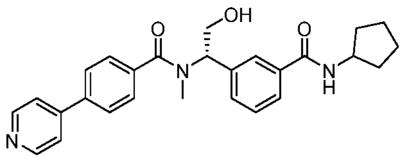 | 444 |
| 46 | 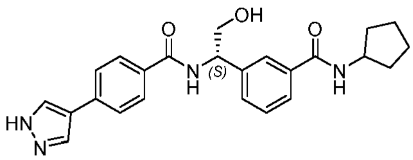 | 418 |
| 47 | 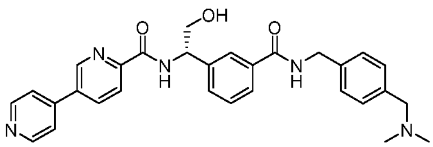 | 510 |
| 48 | 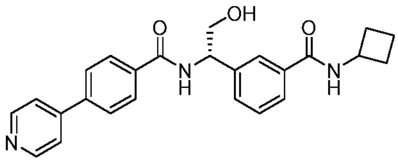 | 416 |
| 49 | 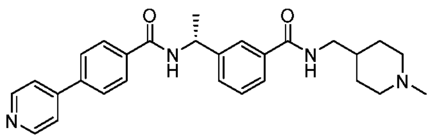 | 457 |
| 50 | 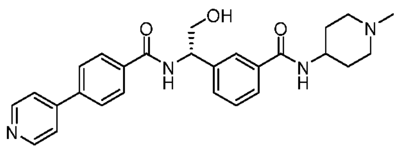 | 459 |
| 51 | 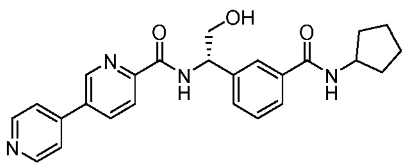 | 431 |
| 52 | 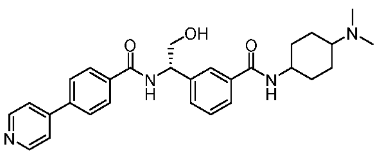 | 487 |

(continued)

| ID | Structure | M+1 |
|---|---|---|
| 53 | 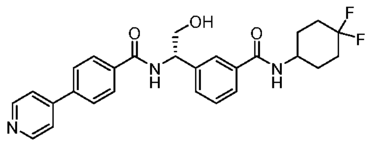 | 480 |
| 54 | 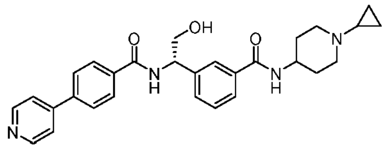 | 485 |
| 55 | 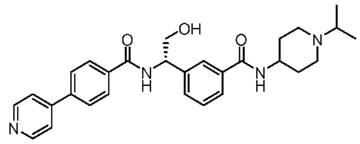 | 487 |
| 56 | 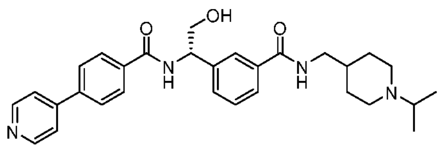 | 501 |
| 57 | 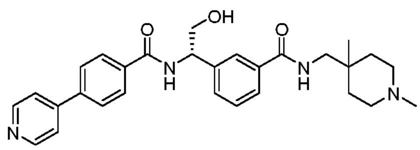 | 487 |
| 58 | 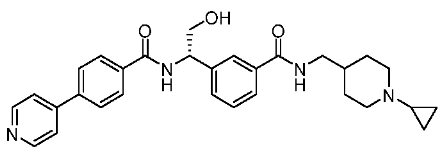 | 499 |
| 59 | 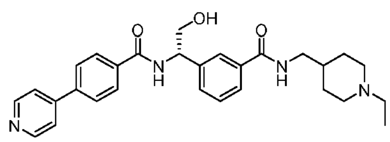 | 487 |
| 60 | 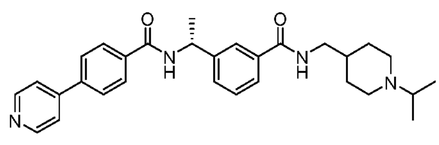 | 485 |
| 61 | 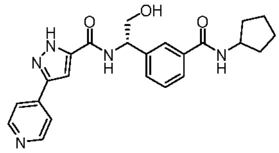 | 420 |
| 62 | 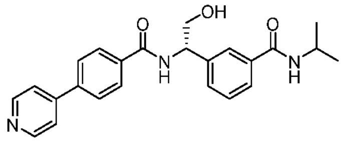 | 404 |

(continued)

| ID | Structure | M+1 |
|---|---|---|
| 63 | 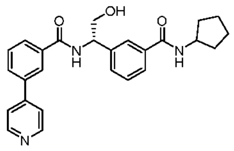 | 430 |
| 64 | 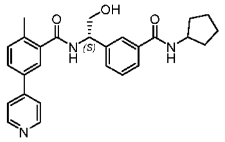 | 444 |
| 65 | 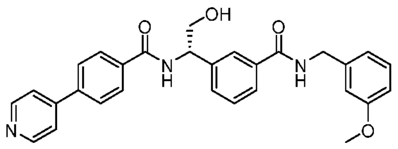 | 482 |
| 66 | 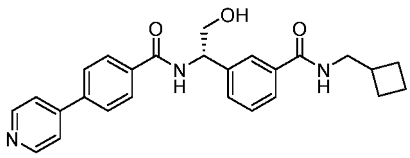 | 430 |
| 67 | 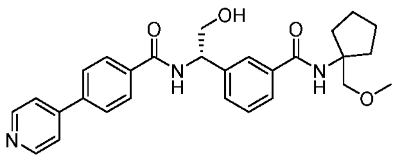 | 474 |
| 68 | 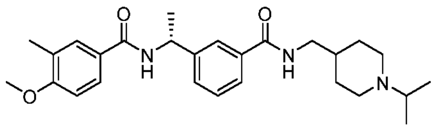 | 452 |
| 69 | 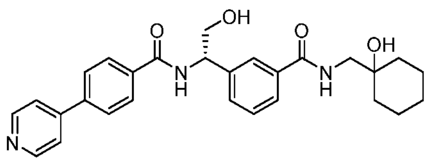 | 474 |
| 70 | 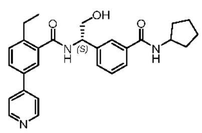 | 458 |
| 71 | 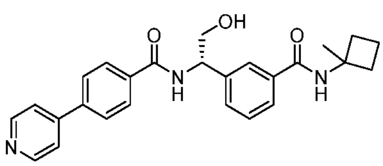 | 430 |

(continued)

| ID | Structure | M+1 |
|---|---|---|
| 72 | 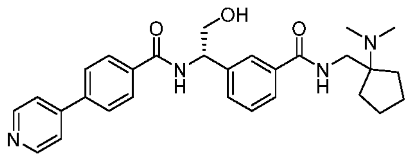 | 487 |
| 73 | 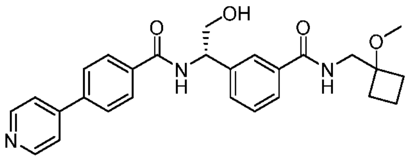 | 460 |
| 74 | 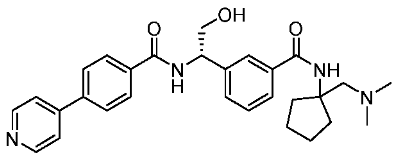 | 487 |
| 75 | 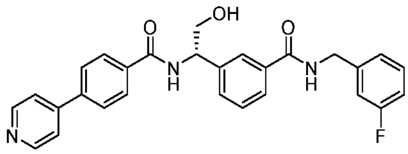 | 470 |
| 76 | 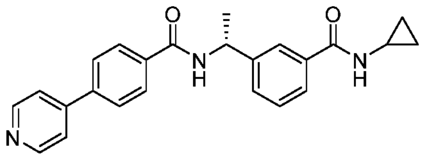 | 386 |

Additional non-limiting examples are:

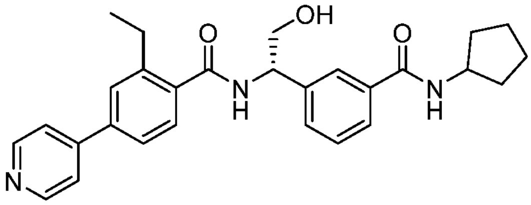

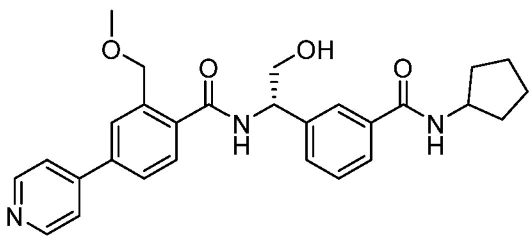

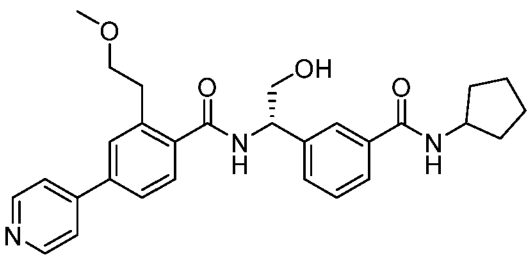

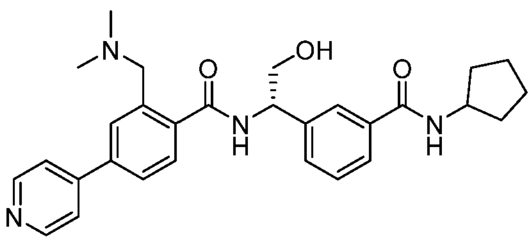

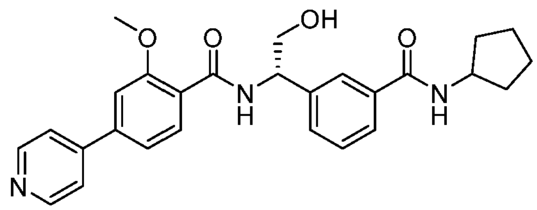

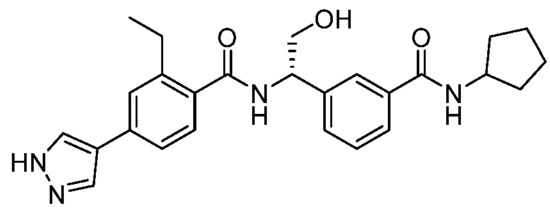

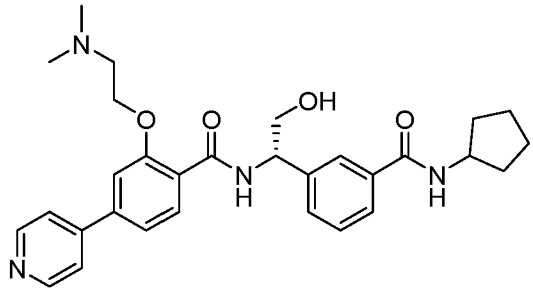

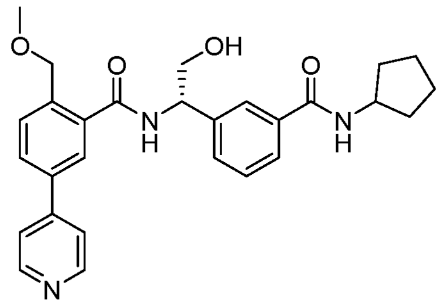

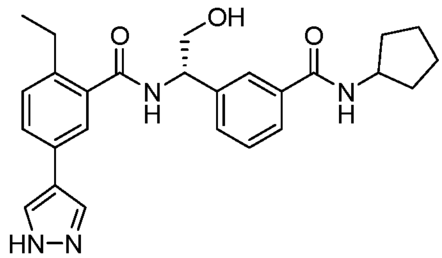

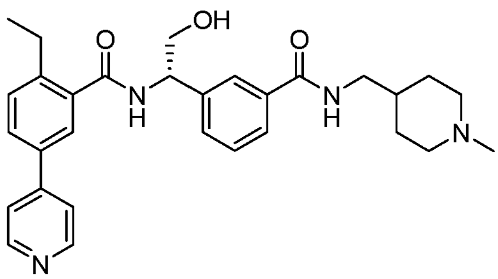

.

The compounds of the present invention demonstrate improved ROCK enzyme inhibiting activity, inhibition of cancer cell growth and viability, and FLT3-ITD mutant selectivity. In addition, the compounds demonstrate superior pharmacokinetic properties. As points of reference, two previously characterized compounds (i.e., Compounds A and B in **Table 2**) were analyzed. Additionally, the compounds disclosed herein generally demonstrate greater ROCK inhibition than Ripasudil, a clinical ROCK inhibitor, with relatively weak activity (ROCK1 $IC_{50}$ = 51 nM and ROCK2 $IC_{50}$ = 19 nM).

The compounds of the present disclosure may be made by a variety of methods, including standard chemistry. Suitable synthetic routes are depicted in the schemes given below.

The compounds may be prepared by methods known in the art of organic synthesis as set forth in part by the following synthetic schemes and examples. In the schemes described below, it is well understood that protecting groups for sensitive or reactive groups are employed where necessary in accordance with general principles or chemistry. Protecting groups are manipulated according to standard methods of organic synthesis (T. W. Greene and P. G. M. Wuts, "Protective Groups in Organic Synthesis", Third edition, Wiley, New York 1999). These groups are removed at a convenient stage of the compound synthesis using methods that are readily apparent to those skilled in the art. The selection processes, as well as the reaction conditions and order of their execution, shall be consistent with the preparation of compounds of Formula I.

Those skilled in the art will recognize if a stereocenter exists in the compounds of Formula I. Accordingly, the present disclosure includes both possible stereoisomers (unless specified in the synthesis) and includes not only racemic compounds but the individual enantiomers and/or diastereomers as well. When a compound is desired as a single enantiomer or diastereomer, it may be obtained by stereospecific synthesis or by resolution of the final product or any convenient intermediate. Resolution of the final product, an intermediate, or a starting material may be affected by any suitable method known in the art. See, for example, "Stereochemistry of Organic Compounds" by E. L. Eliel, S. H. Wilen, and L. N. Mander (Wiley -Interscience, 1994).

The compounds described herein may be made from commercially available starting materials or synthesized using known organic, inorganic, and/or enzymatic processes.

Another aspect of the disclosure relates to a method of treating a disease associated with modulation of Rho-kinases, e.g., ROCK-1, ROCK2, in a subject in need thereof. The method involves administering to a patient in need of

treatment for diseases or disorders associated with modulation of Rho-kinases, e.g., ROCK-1, ROCK2, an effective amount of a compound of Formula I. In an embodiment, the disease can be, but is not limited to, cancer, neurodegenerative disease, neurodevelopmental disease, inflammatory or autoimmune disease, infection, metabolic disease, hematologic disease, or cardiovascular disease.

Another aspect of the disclosure is directed to a method of inhibiting Rho-kinases, e.g., ROCK-1, ROCK2. The method involves administering to a subject in need thereof an effective amount of a disclosed compound.

The present disclosure relates to compositions capable of modulating (e.g., inhibiting) the activity of Rho-kinases, e.g., ROCK-1, ROCK2. The present disclosure also relates to the therapeutic use of such compounds.

One therapeutic use of the compounds of the present disclosure is to treat proliferative diseases or disorders such as cancer. Cancer can be understood as abnormal or unregulated cell growth within a patient and can include, but is not limited to lung cancer, ovarian cancer, breast cancer, prostate cancer, pancreatic cancer, hepatocellular cancer, renal cancer, and leukemia's such as acute myeloid leukemia and acute lymphoblastic leukemia. Additional cancer types include T-cell lymphoma (e.g., cutaneous T-cell lymphoma, peripheral T-cell lymphoma, angioimmunoblastic) and multiple myeloma.

One therapeutic use of the compounds of the present disclosure is to treat neurological diseases or disorders or neurodegeneration. Neurological disorders are understood as disorders of the nervous system (e.g., the brain and the spinal cord). Neurological disorders or neurodegenerative diseases can include, but are not limited to epilepsy, attention deficit disorder (ADD), Alzheimer' s disease, Parkinson's Disease, Huntington's Disease, amyotrophic lateral sclerosis, spinal muscular atrophy, essential tremor, central nervous system trauma caused by tissue injury, oxidative stress-induced neuronal or axomal degeneration, and multiple sclerosis.

Another therapeutic use of the compounds of the present disclosure is to treat neurodevelopmental disorders. Neurodevelopmental disorders can include, but are not limited to, Rett syndrome.

Another therapeutic use of the compounds of the present disclosure is to treat inflammatory diseases or disorders. Inflammation can be understood as a host's response to an initial injury or infection. Symptoms of inflammation can include but are not limited to redness, swelling, pain, heat, and loss of function. Inflammation may be caused by the upregulation of pro-inflammatory cytokines such as IL-Iβ, and increased expression of the FOXP3 transcription factor.

Another therapeutic use of the compounds of the present disclosure is also to treat autoimmune diseases or disorders. Autoimmune disorders are understood as disorders wherein a host's own immune system responds to tissues and substances occurring naturally in the host's body. Autoimmune diseases can include, but are not limited to Rheumatoid arthritis, spondylitis arthritis, psoriatic arthritis, multiple sclerosis, systemic lupus erythematosus, inflammatory bowel disease, graft versus host disease, transplant rejection, fibrotic disease, Crohn's Disease, type-1 diabetes, Eczema, and psoriasis.

Another therapeutic use of the compounds of the present disclosure is to treat infectious diseases or disorders. Infections or infectious diseases are caused by the invasion of a foreign pathogen. The infection may be caused by, for instance, a bacterium, a fungus, or virus. For example, a bacterial infection may be caused by an E. coli.

Yet another therapeutic use of the compounds of the present disclosure is to treat metabolic diseases or disorders. Metabolic diseases can be characterized as abnormalities in the way that a subject stores energy. Metabolic disorders can include, but are not limited to metabolic syndrome, diabetes, obesity, high blood pressure, and heart failure.

Yet another therapeutic use of the compounds of the present disclosure is to treat hematologic disorders. Hematologic diseases primarily affect the blood. Hematologic disorders can include, but are not limited to anemia, lymphoma, and leukemia.

Yet another therapeutic use of the compounds of the present disclosure is also to treat cardiovascular diseases or disorders. Cardiovascular diseases affect the heart and blood vessels of a patient. Exemplary conditions include but are not limited to cardiovascular stress, pressure overload, chronic ischemia, infarction-reperfusion injury, hypertension, atherosclerosis, peripheral artery disease, and heart failure.

In certain aspects, the present invention provides a compound as disclosed herein for use in treating a disease related to upregulation of Rho kinase- signaling pathways.

In other aspects, the present invention is directed to a method of treating an autoimmune disorder in a subject comprising: administering to the subject a therapeutically effective amount of a compound disclosed herein. In one aspect, the autoimmune disorder is rheumatoid arthritis, multiple sclerosis, systemic lupus erythematosus (SLE), psoriasis, Crohn's disease, atopic dermatitis, eczema, or graft- versus-host disease (GVHD).

In some embodiments, the present invention provides a method of treating a cardiovascular disorder in a subject comprising: administering to the subject a therapeutically effective amount of a compound disclosed herein. In one embodiment, the cardiovascular disorder is hypertension, atherosclerosis, restenosis, cardiac hypertrophy, ocular hypertension, cerebral ischemia, cerebral vasospasm, or erectile dysfunction.

In other embodiments, the present invention provides a method of treating inflammation in a subject comprising: administering to the subject a therapeutically effective amount of a compound disclosed herein. In certain aspects, the inflammation is asthma, cardiovascular inflammation, renal inflammation or arteriosclerosis.

In certain aspects, the present invention provides a method of treating a central nervous system disorder in a subject comprising: administering to the subject a therapeutically effective amount of a compound disclosed herein. In one aspect, the central nervous system disorder is neuronal degeneration or spinal cord injury. In another aspect, the central nervous system disorder is Huntington's disease, Parkinson's Disease, Alzheimer's, Amyotrophic lateral sclerosis (ALS), or multiple sclerosis.

The present invention also provides a method of treating an arterial thrombotic disorder in a subject comprising: administering to the subject a therapeutically effective amount of a compound disclosed herein. In one embodiment, the arterial thrombotic disorder is platelet aggregation, or leukocyte aggregation

In other aspects, the present invention relates to a method of treating a fibrotic disorder in a subject comprising: administering to the subject a therapeutically effective amount of a compound disclosed herein. In one embodiment, the fibrotic disorder is liver fibrosis, lung fibrosis, or kidney fibrosis.

The present invention also relates to a method of treating glaucoma or regulating intraocular pressure in a subject comprising administering to the subject a therapeutically effective amount of a compound disclosed herein. In one aspect, the glaucoma is primary open-angle glaucoma, acute angle-closure glaucoma, pigmentary glaucoma, congenital glaucoma, normal tension glaucoma, or secondary glaucoma.

In some embodiments, the present invention is directed to a method of treating a neoplastic disease in a subject comprising: administering to the subject a therapeutically effective amount of a compound disclosed herein. In certain aspects, the neoplastic disorder is a lymphoma, carcinoma, leukemia, sarcoma, or blastoma. In other aspects, the neoplastic disorder is squamous cell cancer, small- cell king cancer, pituitary cancer, esophageal cancer, astrocytoma, soft tissue sarcoma, non- small cell lung cancer, adenocarcinoma of the king, squamous carcinoma of the king, cancer of the peritoneum, hepatocellular cancer, gastrointestinal cancer, pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney cancer, liver cancer, prostate cancer, vulval cancer, thyroid cancer, hepatic carcinoma, brain cancer, endometrial cancer, testis cancer, cholangiocarcinoma, gallbladder carcinoma, gastric cancer, melanoma, or head and neck cancer.

In yet other embodiments, the present invention provides a method of treating metabolic syndrome, insulin resistance, hyperinsulinemia, type 2 diabetes, or glucose intolerance in a subject comprising administering to the subject a therapeutically effective amount of a compound disclosed herein.

In one embodiment, the present invention relates to a method of treating osteoporosis or promoting bone formation in a subject comprising administering to the subject a therapeutically effective amount of a compound disclosed herein.

In another embodiment, the present invention relates to a method of treating an ocular disorder having an angiogenic component comprising administering to the subject a therapeutically effective amount of a compound disclosed herein and an angiogenesis inhibitor. In certain aspects, the ocular disorder is age related macular degeneration (AMD), choroidal neovascularization (CNV), diabetic macular edema (DME), iris neovascularization, uveitis, neo vascular glaucoma, or retinitis of prematurity (ROP).

Another aspect of the present disclosure relates to disclosed compounds, or a pharmaceutically acceptable salt, hydrate, solvate, prodrug, stereoisomer, or tautomer thereof, for use in treating or preventing a disease associated with Rho-kinase modulation. In some embodiments, the disease is cancer, neurodegenerative disease, neurodevelopmental disorder, inflammatory or autoimmune disease, infection, metabolic disease, hematologic disease, or cardiovascular disease. In some embodiments, the compound inhibits a Rho-kinase. In another embodiment, the compound inhibits ROCK-1. In another embodiment, the compound inhibits ROCK-2.

In another aspect, the present disclosure relates to the use of a disclosed compound, or a pharmaceutically acceptable salt, hydrate, solvate, prodrug, stereoisomer, or tautomer thereof, in the manufacture of a medicament for treating or preventing a disease associated with Rho-kinase modulation. In some embodiments, the disease is cancer, neurodegenerative disease, neurodevelopmental disorder, inflammatory or autoimmune disease, infection, metabolic disease, hematologic disease, or cardiovascular disease. In some embodiments, the compound inhibits a Rho-kinase. In another embodiment, the compound inhibits ROCK-1. In another embodiment, the compound inhibits ROCK-2.

In some embodiments, the cancer is cutaneous T-cell lymphoma, peripheral T-cell lymphoma, angioimmunoblastic T-cell lymphoma, multiple myeloma, leukemia, diffuse large B-cell lymphoma, lung, ovarian, breast, prostate, pancreatic, hepatocellular or renal cancer. In other embodiments, the neurodegenerative disease is Alzheimer's, Huntington's, Parkinson's, Amyotrophic Lateral Sclerosis, or spinal muscular atrophy. In other embodiments, the neurodevelopmental disorder is Rett syndrome and Nieman-Pick C disease. In yet other embodiments, the inflammatory or autoimmune disease is rheumatoid arthritis, spondylitis arthritis, psoriatic arthritis, psoriasis, multiple sclerosis, systemic lupus erythematosus, inflammatory bowel diseases, graft versus host disease, transplant rejection or fibrotic disease.

The disclosed compound can be administered in effective amounts to treat or prevent a disorder and/or prevent the development thereof in subjects.

Administration of the disclosed compounds can be accomplished via any mode of administration for therapeutic

agents. These modes include systemic or local administration such as oral, nasal, parenteral, transdermal, subcutaneous, vaginal, buccal, rectal or topical administration modes.

Depending on the intended mode of administration, the disclosed compositions can be in solid, semi-solid or liquid dosage form, such as, for example, injectables, tablets, suppositories, pills, time-release capsules, elixirs, tinctures, emulsions, syrups, powders, liquids, suspensions, or the like, sometimes in unit dosages and consistent with conventional pharmaceutical practices. Likewise, they can also be administered in intravenous (both bolus and infusion), intraperitoneal, subcutaneous or intramuscular form, all using forms well known to those skilled in the pharmaceutical arts.

Illustrative pharmaceutical compositions are tablets and gelatin capsules comprising a Compound of the Disclosure and a pharmaceutically acceptable carrier, such as a) a diluent, e.g., purified water, triglyceride oils, such as hydrogenated or partially hydrogenated vegetable oil, or mixtures thereof, corn oil, olive oil, sunflower oil, safflower oil, fish oils, such as EPA or DHA, or their esters or triglycerides or mixtures thereof, omega-3 fatty acids or derivatives thereof, lactose, dextrose, sucrose, mannitol, sorbitol, cellulose, sodium, saccharin, glucose and/or glycine; b) a lubricant, e.g., silica, talcum, stearic acid, its magnesium or calcium salt, sodium oleate, sodium stearate, magnesium stearate, sodium benzoate, sodium acetate, sodium chloride and/or polyethylene glycol; for tablets also; c) a binder, e.g., magnesium aluminum silicate, starch paste, gelatin, tragacanth, methylcellulose, sodium carboxymethylcellulose, magnesium carbonate, natural sugars such as glucose or beta-lactose, corn sweeteners, natural and synthetic gums such as acacia, tragacanth or sodium alginate, waxes and/or polyvinylpyrrolidone, if desired; d) a disintegrant, e.g., starches, agar, methyl cellulose, bentonite, xanthan gum, alginic acid or its sodium salt, or effervescent mixtures; e) absorbent, colorant, flavorant and sweetener; f) an emulsifier or dispersing agent, such as Tween 80, Labrasol, HPMC, DOSS, caproyl 909, labrafac, labrafil, peceol, transcutol, capmul MCM, capmul PG-12, captex 355, gelucire, vitamin E TGPS or other acceptable emulsifier; and/or g) an agent that enhances absorption of the compound such as cyclodextrin, hydroxypropyl-cyclodextrin, PEG400, PEG200.

Liquid, particularly injectable, compositions can, for example, be prepared by dissolution, dispersion, etc. For example, the disclosed compound is dissolved in or mixed with a pharmaceutically acceptable solvent such as, for example, water, saline, aqueous dextrose, glycerol, ethanol, and the like, to thereby form an injectable isotonic solution or suspension. Proteins such as albumin, chylomicron particles, or serum proteins can be used to solubilize the disclosed compounds.

The disclosed compounds can be also formulated as a suppository that can be prepared from fatty emulsions or suspensions; using polyalkylene glycols such as propylene glycol, as the carrier.

The disclosed compounds can also be administered in the form of liposome delivery systems, such as small unilamellar vesicles, large unilamellar vesicles, and multilamellar vesicles. Liposomes can be formed from a variety of phospholipids, containing cholesterol, stearylamine or phosphatidylcholines. In some embodiments, a film of lipid components is hydrated with an aqueous solution of drug to a form lipid layer encapsulating the drug, as described in U.S. Pat. No. 5,262,564.

Disclosed compounds can also be delivered by the use of monoclonal antibodies as individual carriers to which the disclosed compounds are coupled. The disclosed compounds can also be coupled with soluble polymers as targetable drug carriers. Such polymers can include, but are not limited to polyvinylpyrrolidone, pyran copolymer, polyhydroxypropylmethacrylamidephenol, polyhydroxyethylaspanamidephenol, or polyethyleneoxidepolylysine substituted with palmitoyl residues. Furthermore, the disclosed compounds can be coupled to a class of biodegradable polymers useful in achieving controlled release of a drug, for example, polylactic acid, polyepsilon caprolactone, polyhydroxy butyric acid, polyorthoesters, polyacetals, polydihydropyrans, polycyanoacrylates, and cross-linked or amphipathic block copolymers of hydrogels. In one embodiment, disclosed compounds are not covalently bound to a polymer, e.g., a polycarboxylic acid polymer, or a polyacrylate.

Parental injectable administration is generally used for subcutaneous, intramuscular or intravenous injections, and infusions. Injectables can be prepared in conventional forms, either as liquid solutions or suspensions or solid forms suitable for dissolving in liquid prior to injection.

Another aspect of the disclosure relates to a pharmaceutical composition comprising a disclosed compound and a pharmaceutically acceptable carrier. The pharmaceutically acceptable carrier can further include an excipient, diluent, or surfactant.

Compositions can be prepared according to conventional mixing, granulating or coating methods, respectively, and the present pharmaceutical compositions can contain from about 0.1% to about 99%, from about 5% to about 90%, or from about 1% to about 20% of the disclosed compound by weight or volume.

The dosage regimen utilizing the disclosed compound is selected in accordance with a variety of factors including type, species, age, weight, sex and medical condition of the patient; the severity of the condition to be treated; the route of administration; the renal or hepatic function of the patient; and the particular disclosed compound employed. A physician or veterinarian of ordinary skill in the art can readily determine and prescribe the effective amount of the drug required to prevent, counter or arrest the progress of the condition.

Effective dosage amounts of the disclosed compounds, when used for the indicated effects, range from about

0.5 mg to about 5000 mg of the disclosed compound as needed to treat the condition. Compositions for in vivo or in vitro use can contain about 0.5, 5, 20, 50, 75, 100, 150, 250, 500, 750, 1000, 1250, 2500, 3500, or 5000 mg of the disclosed compound, or, in a range of from one amount to another amount in the list of doses. In one embodiment, the compositions are in the form of a tablet that can be scored.

Without wishing to be bound by any particular theory, the compounds of the present disclosure can inhibit Rho-kinase such as ROCK-1 and ROCK-2.

**EXAMPLES**

The disclosure is further illustrated by the following examples and synthesis examples, that are not to be construed as limiting this disclosure in scope or spirit to the specific procedures herein described. It is to be understood that the examples are provided to illustrate certain embodiments and that no limitation to the scope of the disclosure is intended thereby. It is to be further understood that resort may be had to various other embodiments, modifications, and equivalents thereof which may suggest themselves to those skilled in the art without departing from the spirit of the present disclosure and/or scope of the appended claims.

**Example 1. Synthesis of Compound ID#1**

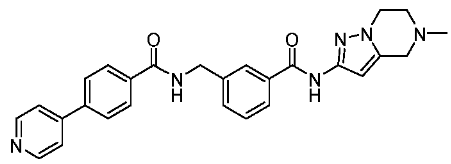

ID#1

*Step 1.*

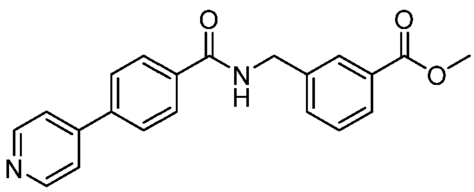

1A

To a solution of 4-(pyridin-4-yl)benzoic acid (100 mg, 0.50 mmol) and methyl 3-(aminomethyl)benzoate (99 mg, 0.6 mmol) in DMF were added diisopropylethylamine (DIEA, 0.26 mL, 1.5 mmol) and 1-[Bis(dimethylamino)methylene]-1*H*-1,2,3-triazolo[4,5-*b*]pyridinium 3-oxid hexafluorophosphate (HATU, 247 mg, 0.65 mmol). The reaction was stirred at room temperature for 2 hrs and diluted with water. The mixture was concentrated and purified by C-18 column chromatography to give 1A (141 mg).

*Step 2.*

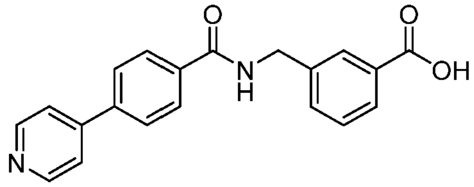

1B

To a solution of 1A (141 mg, 0.41 mmol) in THF was added LiOH solution (1 M, 2.0 mL). The reaction was stirred at room temperature overnight and neutralized with HCl solution. The mixture was concentrated and purified by C-18 column chromatography to give 1B (97 mg).

*Step 3.*

To a solution of 1B (7 mg, 0.02 mmol) and 5-methyl-4,5,6,7-tetrahydropyrazolo[1,5-a]pyrazin-2-amine (6 mg, 0.04 mmol) in DMF were added diisopropylethylamine (DIEA, 0.007 mL, 0.04 mmol) and HATU (15 mg, 0.04 mmol). The reaction was stirred at room temperature for 2 hrs and diluted with water. The mixture was concentrated and purified by C-18 column chromatography to give ID#1 (8 mg).

**Example 2. Synthesis of Compound ID#10**

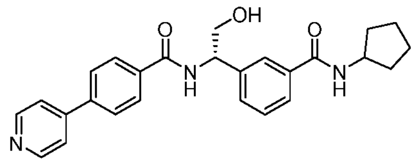

ID#10

*Step 1.*

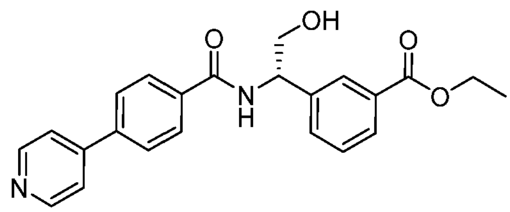

2A

To a solution of 4-(pyridin-4-yl)benzoic acid (50 mg, 0.25 mmol) and methyl 3-((1S)-1-amino-2-hydroxyethyl)benzoate HCl salt (75 mg, 0.3 mmol) in DMF were added diisopropylethylamine (DIEA, 0.13 mL, 0.75 mmol) and HATU (123 mg, 0.32 mmol). The reaction was stirred at room temperature for 2 hrs and diluted with water. The mixture was concentrated and purified by C-18 column chromatography to give 2A (68 mg).

*Step 2.*

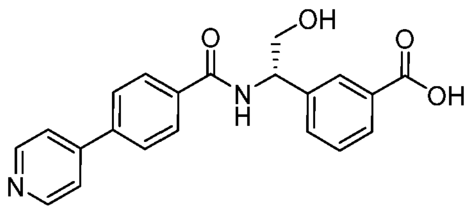

2B

To a solution of 1A (68 mg, 0.17 mmol) in THF was added LiOH solution (1 M, 0.85 mL). The reaction was stirred at room temperature overnight and neutralized with HCl solution. The mixture was concentrated and purified by C-18 column chromatography to give 2B (40 mg).

*Step 3.*

To a solution of 2B (5 mg, 0.014 mmol) and cyclopentylamine (3 mg, 0.035 mmol) in DMF were added diisopropylethylamine (DIEA, 5 microL, 0.028 mmol) and HATU (7 mg, 0.018 mmol). The reaction was stirred at room temperature for 2 hrs and diluted with water. The mixture was concentrated and purified by C-18 column chromatography to give ID#10 (5 mg).

**Example 3. Synthesis of Compound ID#3**

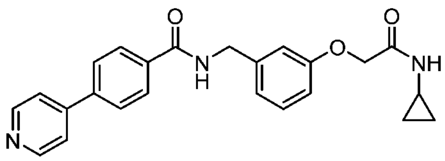

ID#3

*Step 1.*

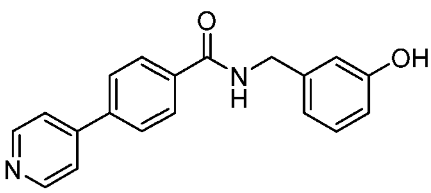

3A

To a solution of 4-(pyridin-4-yl)benzoic acid (50 mg, 0.25 mmol) and 3-(aminomethyl)phenol (37 mg, 0.3 mmol) in DMF were added diisopropylethylamine (DIEA, 0.13 mL, 0.75 mmol) and HATU (123 mg, 0.32 mmol). The reaction was stirred at room temperature for 2 hrs and diluted with water. The mixture was concentrated and purified by C-18 column chromatography to give 3A (62 mg).

*Step 2.*

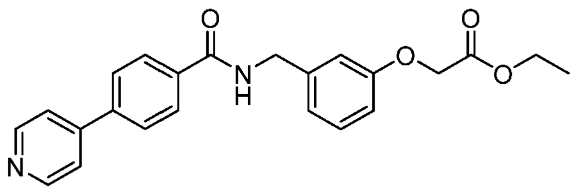

3B

To a mixture of 3A (62 mg, 0.20 mmol) and ethyl chloroacetate (25 mg, 0.20 mmol) in acetonitrile was added potassium carbonate (33 mg, 0.24 mmol). The reaction was heated to 55 C, stirred overnight, filtered and concentrated. The residue was purified by C-18 column chromatography to give 3B (55 mg).

*Step 3.*

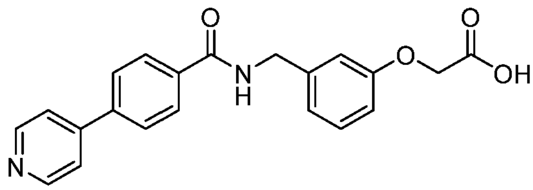

3C

To a solution of 3B (55 mg, 0.14 mmol) in THF was added LiOH solution (1 M, 0.7 mL). The reaction was stirred at room temperature overnight and neutralized with HCl solution. The mixture was concentrated and purified by C-18 column chromatography to give 3C (50 mg). Step 4.

To a solution of 3C (10 mg, 0.028 mmol) and cyclopropylamine (5 mg, 0.084 mmol) in DMF was added HATU (13 mg, 0.034 mmol). The reaction was stirred at room temperature for 2 hrs and diluted with water. The mixture was concentrated and purified by C-18 column chromatography to give ID#3 (10 mg).

**Example 4 - ROCK1 and ROCK2 Kinase Inhibition Assays**

The following assay protocol is for measuring the phosphorylation of a peptide substrate (FAM-KKLRRTLSVA-OH wherein FAM is carboxyfluorescein). The peptide is >98% purity by Capillary Electrophoresis. The peptide is phosphorylated by the protein kinase ROCK1 or ROCK2. The ROCK1 or ROCK2 enzyme, substrate, and cofactors (ATP and $Mg^{2+}$) are combined in a well of a microtiter plate and incubated for 3 hours at 25°C in the presence or absence of an inhibitor compound. At the end of the incubation, the reaction is quenched by the addition of an EDTA-containing buffer. The substrate and product are separated and quantified electrophoretically using the microfluidic-based LABCHIP® 3000 Drug Discovery System from Caliper Life Sciences (Hopkinton, Massachusetts).

The components of the assay mixture are:

100 mM HEPES, pH 7.5
0.1% BSA
0.01% Triton X-100
1 mM DTT
10 mM $MgCl_2$
10 μM Sodium Orthovanadate
10 μM Beta-Glycerophosphate
5 μM ATP (for ROCK1) or 7 μM ATP (for ROCK2)
1% DMSO (from compound)
1.25 μM FAM-KKLRRTLSVA-OH
3 nM ROCK1 or 2.5 nM ROCK2 enzyme

Substrate and product peptides present in each sample are separated electrophoretically using the LABCHIP® 3000 capillary electrophoresis instrument. As substrate and product peptides are separated two peaks of fluorescence

are observed. Change in the relative fluorescence intensity of the substrate and product peaks is the parameter measured reflecting enzyme activity. Capillary electrophoregramms (RDA acquisition files) are analyzed using HTS Well Analyzer software (Caliper Life Sciences, Hopkinton, Massachusetts). The kinase activity in each sample is determined as the product to sum ratio (PSR): P/(S+P), where P is the peak height of the product peptide and S is the peak height of the substrate peptide. For each compound, enzyme activity is measured at various concentrations (12 concentrations of compound spaced by 3X dilution intervals). Negative control samples (0%- inhibition in the absence of inhibitor) and positive control samples (100%-inhibition in the presence of 20 mM EDTA) are assembled in replicates of four and are used to calculate %-inhibition values for each compound at each concentration. Percent inhibition (Pinh) is determined using the following equation:

$$Pinh = (PSR0\% - PSRinh)/(PSR0\% - PSR100\%)*100$$

where PSRinh is the product sum ratio in the presence of inhibitor, PSR0% is the average product sum ratio in the absence of inhibitor, and PSR100% is the average product sum ratio in 100%-inhibition control samples. The $IC_{50}$ values of inhibitors are determined by fitting the inhibition curves (Pinh versus inhibitor concentration) by the 4-parameter sigmoidal dose-response model using XLfit 4 software (IBDS).

This assay can be used to test the activity of each of the exemplary compounds identified in Table 2. It is expected that each of these compounds will demonstrate inhibition of the protein kinase ROCK1 and/or ROCK2.

**Example 5. Cell Viability Assay**

Cell viability in the presence of varying concentrations of the above listed compounds at different time points was used to assess cytotoxicity and the effect of the compounds on cell proliferation. $IC_{50}$ (or percent activity) data for the compounds of the present invention in K562 or MV411 cell lines are summarized in Table 2.

*Cell Viability Assay*- Cell viability was measured by the CELLTITER-GlO® cell viability assay from Promega (Madison, WI). The CELLTITER-GlO® Luminescent Cell Viability Assay is a homogeneous method to determine the number of viable cells in culture based on quantitation of the ATP present, which signals the presence of metabolically active cells. Following treatment, CELLTITER-GlO® is added to treatment wells and incubated at 37° C. Luminescence values were measured at using a Molecular Devices Spectramax microplate reader.

*Experimental Design*

*Single Agent Studies*- Cells were grown to 70% confluency, trypsinized, counted, and seeded in 96 well flat-bottom plates at a final concentration of $2.5\times10^3$-$5\times10^3$ cells/well (Day 0). Cells were allowed to incubate in growth media for 24 hours. Treatment with the test agents or standard agents began on Day 1 and continued for 72 hours. At the 72-hour time point, treatment containing media was removed. Viable cell numbers were quantified by the CELLTITER-GLO® cell viability assay as described above. Results from these studies were used to calculate an $IC_{50}$ value (concentration of drug that inhibits cell growth by 50 percent of control) for each compound.

*Data Collection*- For single agent and combination studies, data from each experiment was collected and expressed as % Cell Growth using the following calculation:

$$\% \text{ Cell Growth} = (f_{test}/f_{vehicle}) \text{ x } 100$$

Where $f_{test}$ is the luminescence of the tested sample, and $f_{vehicle}$ is the luminescence of the vehicle in which the drug is dissolved. Dose response graphs and $IC_{50}$ values were generated using Prism 6 software (GraphPad) using the following equation:

$$Y = (Top\text{-}Bottom)/(1+10^{((logIC50\text{-}X)\text{-}HillSlope)})$$

Where X is the logarithm of the concentration and Y is the response. Y starts at the Bottom and goes to the Top with a sigmoid shape.

**Example 7. ROCK1 and ROCK2 Kinase Inhibition and Cell Viability Assay Results**

In general, kinases regulate many important cellular activities including cell growth, signaling, metabolism, etc.

Different kinases have distinct functions and pathways. Selective inhibition of ROCK1 and ROCK2 avoids off target activity that may cause undesired side effects such as toxicity.

The protocols outlined in Examples 5 and 6 were followed to test ROCK1 and ROCK2 kinase inhibition and cancer cell viability with compounds from Table 1. As shown in Table 2, the compounds demonstrated inhibition of the ROCK1 and ROCK2 kinases and growth of cancer cells.

The experiments also evaluated the selectivity of the compounds for inhibiting growth of cancer cells carrying a mutation in the *Flt3* gene. The MV411 cell line expresses the mutant allele of *Flt3* with internal tandem duplications (ITD) of the gene. *See* Quentmeier et al., "FLT3 Mutations in Acute Myeloid Leukemia Cell Lines," Leukemia 17(1), 2003, 120-124. K562 is a chronic myeloid leukemia cell line that does not express FLT3 protein. *See* Grafone et al., "Monitoring of FLT3 Phosphorylation Status and Its Response to Drugs By Flow Cytometry in AML Blast Cells," Hematol Oncol. 26(3), 2008, 159-166. Compounds demonstrating FLT3-ITD$^+$ selectivity are identified by a greater K562/MV411 ratio in **Table 2.**

FLT3/ITD gene mutations have been detected by polymerase chain reaction (PCR) in acute myeloid leukemia (AML), acute lymphocytic leukemia (ALL), chronic myelogenous leukemia (CML) in chronic phase (CML-CP), CML cases in blast crisis (CML-BC), chronic lymphatic leukemia (CLL), myelodysplastic syndrome (MDS), chronic myelomonocytic leukemia (CMML), multiple myeloma (MM) cases and non-Hodgkin's lymphoma (NHL) with marrow infiltration. Patients with ITD-*FLT3*$^+$ acute myeloid leukemia (AML) experience an extremely poor prognosis.

Surprisingly, many of the compounds demonstrated greater efficacy with the MV11 cells than with the K562 cells, suggesting that these compounds could be used to effectively treat FLT3-ITD$^+$ cancers including ITD-*FLT3*$^+$ AML.

**Table 2.** ROCK1 and ROCK2 kinase inhibition and cell viability

| Compound ID | ROCK1 $IC_{50}$ (nM) | ROCK2 $IC_{50}$ (nM) | K562* (μM) | MV411* (μM) | FLT3-ITD$^+$ Selectivity (K562/MV411) |
|---|---|---|---|---|---|
| 1 | 11.6 | 6.0 | >100 | 8.9 | >11 |
| 2 | 2.8 | 2.8 | | | |
| 3 | 38.8 | 14.2 | >100 | 2.4 | >42 |
| 4 | 56.1 | 18.9 | | | |
| 5 | 21.7 | 6.7 | >100 | 1.5 | >68 |
| 6 | | 1.2 | 8.0 | 0.2 | 40 |
| 7 | | 1.5 | 20.4 | 0.06 | 333 |
| 8 | | 1.3 | | | |
| 9 | | 1.7 | 35 | 0.8 | 47 |
| 10 | 4.8 | 0.87 | 17 | 0.17 | 100 |
| 11 | | 2.7 | >100 | 12.3 | >8 |
| 12 | | 1.8 | >100 | 0.54 | >187 |
| 13 | | 1.7 | 27 | 3.0 | 9 |
| 14 | | 1.6 | 48 | 0.09 | 532 |
| 15 | | 9 | >100 | 4.0 | >25 |
| 16 | | 3.6 | 30 | 1.5 | 20 |
| 17 | | 22.5 | >100 | 13.5 | >7 |
| 18 | | 19.6 | >100 | 15.9 | >6 |
| 19 | | | 44 | 13.2 | 3 |
| 20 | | 1.2 | 16 | 0.01 | 1585 |
| 21 | 3.1 | 1.0 | 39 | 2.1 | 19 |
| 22 | | 0.57 | >100 | 3.8 | >27 |
| 23 | | 0.3 | 56 | 0.09 | 632 |

(continued)

| Compound ID | ROCK1 $IC_{50}$ (nM) | ROCK2 $IC_{50}$ (nM) | K562* (μM) | MV411* (μM) | FLT3-ITD⁺ Selectivity (K562/MV411) |
|---|---|---|---|---|---|
| 24 | 2.2 | 1.2 | 28 | 1.5 | 19 |
| 25 | | | | 18.9 | |
| 26 | 3.7 | 1.5 | 48 | 7.4 | 6 |
| 27 | | 0.39 | 25 | 4.4 | 6 |
| 28 | 1.6 | 1.1 | >100 | 2.4 | >42 |
| 29 | | | | 65.1 | |
| 30 | 2.9 | 1.5 | | | |
| 31 | 2.1 | 1.0 | | | |
| 32 | 1.4 | 0.9 | 37 | 0.06 | 619 |
| 33 | | 0.38 | | | |
| 34 | | 0.69 | | | |
| 35 | 1.6 | 1.2 | | | |
| 36 | | 1.2 | | | |
| 37 | | 3.0 | | 1.7 | |
| 38 | | 56 | | 20 | |
| 39 | | 0.3 | 9.4 | 0.01 | 940 |
| 40 | | 0.41 | | 3.4 | |
| 41 | | 6.53 | | | |
| 42 | | 72.6 | | | |
| 43 | | 9.3 | | | |
| 44 | | 240 | | | |
| 45 | | 257 | | | |
| 46 | | 1.88 | 11 | 0.09 | 122 |
| 47 | | 0.54 | 36 | 0.10 | 360 |
| 48 | | 1.01 | >100 | 0.44 | >230 |
| 49 | | 0.30 | >100 | 0.02 | >5050 |
| 50 | | 0.33 | >100 | 0.10 | >1010 |
| 51 | | 4.38 | >100 | 1.45 | >70 |
| 52 | | 0.61 | >100 | 0.12 | >842 |
| 53 | | 0.66 | >100 | 0.22 | >459 |
| 54 | | 0.54 | >100 | 0.07 | >1443 |
| 55 | | 0.47 | >100 | 0.12 | >842 |
| 56 | | 0.31 | >100 | 0.09 | > 1122 |
| 57 | | 0.63 | >100 | 0.09 | > 1122 |
| 58 | | 0.44 | >100 | 0.04 | >2525 |
| 59 | | 0.46 | >100 | 0.28 | >361 |
| 60 | | 0.53 | >100 | 0.04 | >2525 |

(continued)

| Compound ID | ROCK1 $IC_{50}$ (nM) | ROCK2 $IC_{50}$ (nM) | K562* (μM) | MV411* (μM) | FLT3-ITD+ Selectivity (K562/MV411) |
|---|---|---|---|---|---|
| 61 | | 2.12 | >100 | 2.5 | >40 |
| 62 | | 2.85 | 65 | 1.03 | 63 |
| 63 | 14 | 4.1 | >100 | 0.14 | >721 |
| 64 | 16.8 | 5.2 | >100 | 1.49 | >68 |
| 65 | | 0.6 | 28 | 0.07 | 393 |
| 66 | | 0.6 | 36 | 0.13 | 279 |
| 67 | | 9.1 | 41 | 2.67 | 15 |
| 68 | | 104 | | | |
| 69 | | 0.7 | 52 | 0.15 | 350 |
| 70 | 3.84 | 0.9 | 85 | 0.31 | 275 |
| 71 | | 9.0 | 34 | 0.91 | 37 |
| 72 | | 0.5 | 89 | 0.10 | 891 |
| 73 | | 0.6 | 27 | 0.08 | 337 |
| 74 | | 27.1 | 74 | 9.12 | 8 |
| 75 | | 0.5 | 33 | 0.16 | 207 |
| 76 | 2.4 | 1.2 | 58 | 0.48 | 120 |
| Compound A** | 3.3 | 2.8 | 0.8 | 0.7 | 1 |
| Compound B*** | 9.9 | 8.7 | 20 | 1.3 | 15 |

* MV411 is ITD-*FLT3*+, and K562 does not express *ITD-FLT3.* Rho-associated kinase may be manipulated for the treatment of ITD-*FLT3*+ AML as reported in Onish et al., "Internal Tandem Duplication Mutations in FLT3 Gene Augment Chemotaxis to Cxcl12 Protein by Blocking the Down-regulation of the Rho-associated Kinase via the Cxcl12/Cxcr4 Signaling Axis," J. Biol. Chem. 289 (45), 2014, 31053-31065.
** Compound A was a positive control (shown below and is described by Schirok et al., "Design and Synthesis of Potent and Selective Azaindole-Based Rho Kinase (ROCK) Inhibitors," ChemMedChem 3, 2008, 1893 - 1904).
*** Compound B was another positive control (shown below and is described by Cook et al, "RHO KINASE INHIBITORS" US Patent No. 8,697,911).

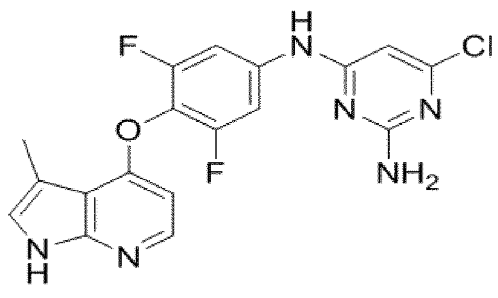

*Compound A*

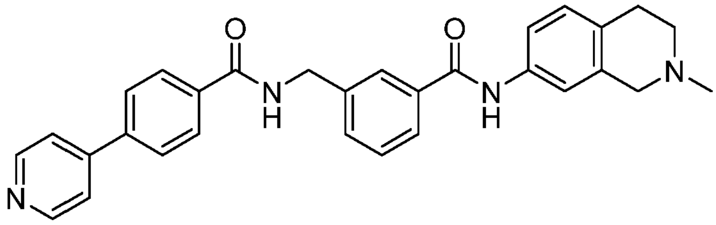

*Compound B*

**Example 8. Pharmacokinetic activity**

Compounds were individually and accurately weighed out to produce a stock solution in Dimethyl-sulfoxide (DMSO) at a concentration of 2 mg/mL and stored at -20⁰C. From the stock solution, a working stock was prepared by diluting it in Methanol-Water 50:50 (v/v) at 20 $\mu$g/mL for calibration curve preparation and stored at 4⁰C. Standards used for quantitation and quality control samples (QCs) were prepared on the same day of sample processing using blank plasma obtained from non-treated mice. For each analyte, standards were prepared through serial dilutions of the working stock at concentrations of 0.1, 0.5, 1.0, 10, 50, 100, 500, and 1000 ng/mL; QCs were prepared at intermediate concentrations of 0.75, 7.5, 75, and 750 ng/mL. Plasma samples were stored at -80⁰C until ready for analysis and then placed on ice for thawing. An aliquot of 20 $\mu$L samples, standards, or QCs, were transferred into a 96-well extraction plate according to a pre-defined layout. An appropriate volume of Methanol-Formic Acid 99.9-0.1 containing internal standard (Verapamil at 25 ng/mL) was added to each well and the samples were extracted under vacuum. Eluent was then transferred into a LCMS plate for analysis using a suitable column for separation and MRM detection. Concentration of analytes was calculated based on the calibration curve and analyzed for pharmacokinetic parameters by using Noncompartmental analysis (NCA). Parameters such as Cmax, Tmax, half-life, AUC(0-last), AUC(0-$\infty$), volume of distribution (Vss), and clearance (Cl/F) were reported.

**Table 3. Pharmacokinetic activity of 7, 10 and 36 in mice**

| PK parameters | 7 | | 10 | | 36 | |
|---|---|---|---|---|---|---|
| | IV - 1 mg/kg | PO-5 mg/kg | IV - 1 mg/kg | PO - 5 mg/kg | IV - 1 mg/kg | PO - 5 mg/kg |
| R-sq | 0.88 | 0.47 | 0.90 | 0.93 | 0.72 | 1.00 |
| Half life (hr) | 6.43 | N/A | 3.86 | 2.32 | 2.59 | 2.96 |
| Tmax (hr) | 0.25 | 1.00 | 0.25 | 1.00 | 0.25 | 1.00 |
| Cmax (ng/mL) | 190.02 | 5.30 | 4179.80 | 13132.60 | 4344.78 | 2501.58 |
| C0 (ng/mL) | 411.06 | | 7001.17 | | 5905.04 | |
| $AUC_{0-\infty}$ (hr*ng/mL) | 234.71 | 81.60 | 5346.72 | 51413.43 | 5854.70 | 8887.72 |
| $AUC_{0-inf}$ (hr*ng/mL) | 242.69 | N/A | 5367.78 | 51459.94 | 6067.28 | 10491.23 |
| AUC %Extrap | 3.29 | N/A | 0.39 | 0.09 | 0.35 | 0.33 |
| Vss (L/kg) | 16.31 | | 0.32 | | 0.27 | |
| Vz/F obs (L/kg) | | N/A | | 0.30 | | 2.04 |
| Cl/F obs (L/hr/kg) | 4.12 | N/A | 0.17 | 0.09 | 0.16 | 0.48 |
| %F[a] | | N/A | | *> 100* | | *34.58* |

Unless defined otherwise, all technical and scientific terms herein have the same meaning as commonly understood by one of ordinary skill in the art to which this invention belongs.

## Claims

**1.** A compound of Formula (I):

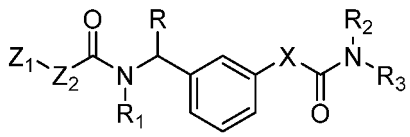

**I**

wherein:

$Z_1$ is pyridine or pyrazole, wherein the pyridine or pyrazole is optionally substituted with halo, -OH, -CN, -COOR', -OR', -SR', -OC(O)R', -NHR', -NR'R", -NHC(O)R', -NHC(O)NR'R", -C(O)NR'R", -NS(O)$_2$R', -S(O)$_2$NR'R", -S(O)$_2$R', guanidino, nitro, nitroso, C1-C6 alkyl, aryl, C3-C7 cycloalkyl, or a 3- to 10-membered heterocycle, wherein the C1-C6 alkyl, aryl, C3-C7 cycloalkyl, or 3- to 10-membered heterocycle is unsubstituted or substituted with one or more of the following: halo, -OH, -CN, -COOR', -OR', -SR', -OC(O)R', -NHR', -NR'R", -NHC(O)R', - NHC(O)NR'R", -C(O)NR'R", -NS(O)$_2$R', -S(O)$_2$NR'R", -S(O)$_2$R', guanidino, nitro, nitroso, Cl-C6 alkyl, aryl, or C3-C7 cycloalkyl;

$Z_2$ is phenyl, naphthyl, or C5-C10 membered heterocycle, wherein the phenyl, naphthyl, or C5-C10 membered heterocycle is optionally substituted with halo, -OH, -CN, -COOR', -OR', - SR', -OC(O)R', -NHR', -NR'R", -NHC(O)R', -NHC(O)NR'R", -C(O)NR'R", -NS(O)$_2$R', - S(O)$_2$NR'R", -S(O)$_2$R', guanidino, nitro, nitroso, C1-C6 alkyl, aryl, C3-C7 cycloalkyl, or a 3- to 10-membered heterocycle, wherein the C1-C6 alkyl, aryl, C3-C7 cycloalkyl, or 3- to 10-membered heterocycle is unsubstituted or substituted with one or more of the following: halo, -OH, -CN, - COOR', -OR', -SR', -OC(O)R', -NHR', -NR'R", -NHC(O)R', -NHC(O)NR'R", -C(O)NR'R", - NS(O)$_2$R', -S(O)$_2$NR'R", -S(O)$_2$R', guanidino, nitro, nitroso, C1-C6 alkyl, aryl, or C3-C7 cycloalkyl;

R is -C1-C6 alkyl, optionally substituted with one or more -OH;

$R_1$ is H, C1-C6 alkyl, or C3-C7 cycloalkyl, wherein the C1-C6 alkyl, or C3-C7-cycloalkyl is optionally substituted with one or more of the following: halo, -OH, -CN, -COOR', -OR', -SR', -OC(O)R', -NHR', -NR'R", -NHC(O)R', -NHC(O)NR'R", -C(O)NR'R", -NS(O)$_2$R', - S(O)$_2$NR'R", or -S(O)$_2$R';

X is a bond or -O(C1-C6 alkyl); and

$R_2$ and $R_3$ are independently H, C1-C6 alkyl or C3-C7 cycloalkyl, aryl, or C5-C10 membered heterocycle,

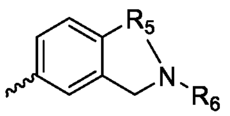,

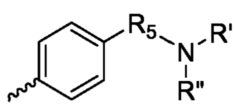,

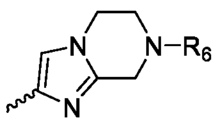,

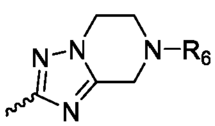, or

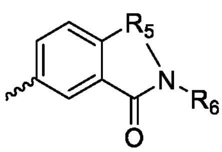,

wherein the C1-C6 alkyl, C3-C7 cycloalkyl, aryl, or C5-C10 membered heterocycle is optionally substituted with halo, -OH, -CN, -COOR', -OR', - SR', -OC(O)R', -NHR', -NR'R", -CNR'R", -NHC(O)R', -NHC(O)NR'R", -C(O)NR'R", - NS(O)$_2$R', -S(O)$_2$NR'R", -S(O)$_2$R', guanidino, nitro, nitroso, C1-C6 alkyl, aryl, C3-C7 cycloalkyl, or 3- to 10-membered heterocycle, wherein the C1-C6 alkyl, aryl, C3-C7 cycloalkyl, or 3- to 10-membered heterocycle is unsubstituted or substituted with one or more of the following: halo, - OH, -CN, -COOR', -OR', -SR', -OC(O)R', -NHR', -NR'R", -NHC(O)R', -NHC(O)NR'R", - C(O)NR'R", -NS(O)$_2$R', -S(O)$_2$NR'R", -S(O)$_2$R', guanidino, nitro, nitroso, C1-C6 alkyl, aryl, or C3-C7 cycloalkyl;

wherein $R_5$ is C1-C6 alkyl, $-OCH_2CH_2-$, $-NR_6CH_2CH_2-$, or $-NC(O)CH_2CH_2-$; and $R_6$ is H, C1-C6 alkyl or C3-C7 cycloalkyl; and

wherein R' and R" are independently H or C1-C6 alkyl; and wherein R' and R" together, optionally attaching to N or O atom, may form a 4- to 8-membered cyclic structure.

2. The compound of Claim 1, wherein R is $-CH_3$ or $-CH_3OH$.

3. The compound of Claim 1, having a structure of Formula (III): wherein:

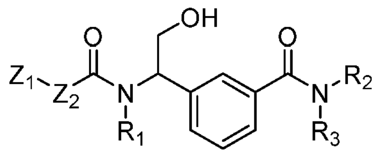

**III**

,

$Z_1$ is pyridine or pyrazole, wherein the pyridine or pyrazole is optionally substituted with halo, -OH, -CN, -COOR', -OR', -SR', -OC(O)R', -NHR', -NR'R", -NHC(O)R', - NHC(O)NR'R", -C(O)NR'R", $-NS(O)_2R'$, $-S(O)_2NR'R"$, $-S(O)_2R'$, guanidino, nitro, nitroso, Cl-C6 alkyl, aryl, C3-C7 cycloalkyl, or a 3- to 10-membered heterocycle, wherein the C1-C6 alkyl, aryl, C3-C7 cycloalkyl, or 3- to 10-membered heterocycle is unsubstituted or substituted with one or more of the following: halo, -OH, -CN, -COOR', -OR', -SR', -OC(O)R', -NHR', -NR'R", -NHC(O)R', -NHC(O)NR'R", -C(O)NR'R", $-NS(O)_2R'$, $-S(O)_2NR'R"$, $-S(O)_2R'$, guanidino, nitro, nitroso, C1-C6 alkyl, aryl, or C3-C7 cycloalkyl;

$Z_2$ is phenyl, naphthyl, or a C5-C10 membered heterocycle, wherein the phenyl, naphthyl, or C5-C10 membered heterocycle is optionally substituted with halo, -OH, -CN, -COOR', -OR', -SR', -OC(O)R', -NHR', -NR'R", -NHC(O)R', -NHC(O)NR'R", -C(O)NR'R", $-NS(O)_2R'$, - $S(O)_2NR'R"$, $-S(O)_2R'$, guanidino, nitro, nitroso, C1-C6 alkyl, aryl, C3-C7 cycloalkyl, or a 3- to 10-membered heterocycle, wherein the C1-C6 alkyl, aryl, C3-C7 cycloalkyl, or 3- to 10-membered heterocycle is unsubstituted or substituted with one or more of the following: halo, -OH, -CN, - COOR', -OR', -SR', -OC(O)R', -NHR', -NR'R", -NHC(O)R', -NHC(O)NR'R", -C(O)NR'R", - $NS(O)_2R'$, $-S(O)_2NR'R"$, $-S(O)_2R'$, guanidino, nitro, nitroso, C1-C6 alkyl, aryl, or C3-C7 cycloalkyl;

$R_1$ is H, C1-C6 alkyl or C3-C7 cycloalkyl, wherein the C1-C6 alkyl or C3-C7 cycloalkyl is optionally substituted with one or more of the following: halo, -OH, -CN, -COOR', -OR', -SR', - OC(O)R', -NHR', -NR'R", -NHC(O)R', -NHC(O)NR'R", -C(O)NR'R", $-NS(O)_2R'$, - $S(O)_2NR'R"$, or $-S(O)_2R'$; and

$R_2$ and $R_3$ are independently H, C1-C6 alkyl, C3-C7 cycloalkyl, aryl, or C5-C10 membered heterocycle,

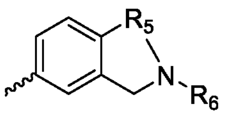 , 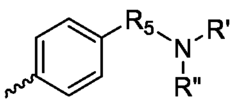 , 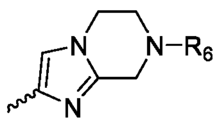 , 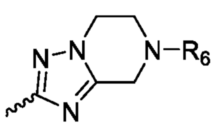 ,

or

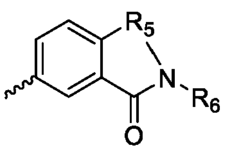

,

wherein the $C_1$-$C_6$ alkyl, $C_3$-$C_7$ cycloalkyl, aryl, or C5-C10 membered heterocycle is optionally substituted with halo, -OH, -CN, -COOR', -OR', -SR', -OC(O)R', - NHR', -NR'R", -CNR'R", -NHC(O)R', -NHC(O)NR'R", -C(O)NR'R", $-NS(O)_2R'$, - $S(O)_2NR'R"$, $-S(O)_2R'$, guanidino, nitro, nitroso, C1-C6 alkyl, aryl, C3-C7 cycloalkyl, or a 3- to 10-membered heterocycle, wherein the C1-C6 alkyl, aryl, C3-C7 cycloalkyl, or 3- to 10-membered heterocycle is unsubstituted or substituted with one or more of the following: halo, -OH, -CN, - COOR', -OR', -SR', -OC(O)R', -NHR', -NR'R", -NHC(O)R', -NHC(O)NR'R", -C(O)NR'R", - $NS(O)_2R'$, $-S(O)_2NR'R"$, $-S(O)_2R'$, guanidino, nitro, nitroso, C1-C6 alkyl, aryl, or C3-C7 cycloalkyl;

wherein $R_5$ is C1-C6 alkyl, $-OCH_2CH_2-$, $-NR_6CH_2CH_2-$, or $-NC(O)CH_2CH_2-$; and $R_6$ is H, C1-C6 alkyl or C3-C7 cycloalkyl; and

wherein R' and R" are independently H or C1-C6 alkyl; or R' and R" together, optionally attaching to N or O atom, may form a 4- to 8- membered cyclic structure.

**4.** The compound of Claim 3, having a structure of Formula (IV):

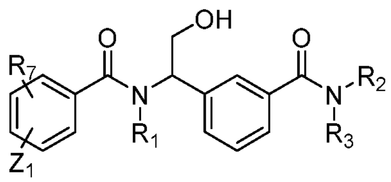

**IV**

wherein:

$Z_1$ is pyridine or pyrazole, wherein the pyridine or pyrazole is optionally substituted with halo, -OH, -CN, -COOR', -OR', -SR', -OC(O)R', -NHR', -NR'R", -NHC(O)R', -NHC(O)NR'R", -C(O)NR'R", $-NS(O)_2R'$, $-S(O)_2NR'R"$, $-S(O)_2R'$, guanidino, nitro, nitroso, C1-C6 alkyl, aryl, C3-C7 cycloalkyl, or a 3- to 10-membered heterocycle, wherein the C1-C6 alkyl, aryl, C3-C7 cycloalkyl, or 3- to 10-membered heterocycle is unsubstituted or substituted with one or more of the following: halo, -OH, -CN, -COOR', -OR', -SR', -OC(O)R', -NHR', -NR'R", -NHC(O)R', - NHC(O)NR'R", -C(O)NR'R", $-NS(O)_2R'$, $-S(O)_2NR'R"$, $-S(O)_2R'$, guanidino, nitro, nitroso, Cl-C6 alkyl, aryl, or $C_3$-$C_7$ cycloalkyl;

$R_1$ is H, C1-C6 alkyl or C3-C7 cycloalkyl, wherein the C1-C6 alkyl or C3-C7 cycloalkyl is optionally substituted with one or more of the following: halo, -OH, -CN, -COOR', -OR', -SR', - OC(O)R', -NHR', -NR'R", -NHC(O)R', -NHC(O)NR'R", -C(O)NR'R", $-NS(O)_2R'$, - $S(O)_2NR'R"$, or $-S(O)_2R'$; and

$R_2$ and $R_3$ are independently H, C1-C6 alkyl, C3-C7 cycloalkyl, aryl, or C5-C10 membered heterocycle,

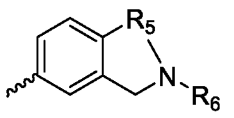,
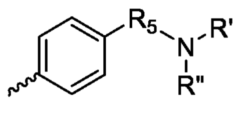,
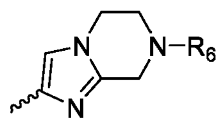,
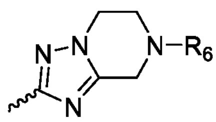,

or

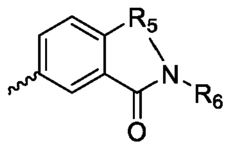,

wherein the $C_1$-$C_6$ alkyl, $C_3$-$C_7$ cycloalkyl, aryl, or C5-C10 membered heterocycle is optionally substituted with halo, -OH, -CN, -COOR', -OR', -SR', -OC(O)R', - NHR', -NR'R", -CNR'R", -NHC(O)R', -NHC(O)NR'R", -C(O)NR'R", $-NS(O)_2R'$, - $S(O)_2NR'R"$, $-S(O)_2R'$, guanidino, nitro, nitroso, C1-C6 alkyl, aryl, C3-C7 cycloalkyl, or a 3- to 10-membered heterocycle, wherein the C1-C6 alkyl, aryl, C3-C7 cycloalkyl or 3- to 10-membered heterocycle is unsubstituted or substituted with one or more of the following: halo, -OH, -CN, - COOR', -OR', -SR', -OC(O)R', -NHR', -NR'R", -NHC(O)R', -NHC(O)NR'R", -C(O)NR'R", - $NS(O)_2R'$, $-S(O)_2NR'R"$, $-S(O)_2R'$, guanidino, nitro, nitroso, C1-C6 alkyl, aryl, or C3-C7 cycloalkyl;

wherein $R_5$ is C1-C6 alkyl, $-OCH_2CH_2-$, $-NR_6CH_2CH_2-$, or $-NC(O)CH_2CH_2-$; and $R_6$ is H, C1-C6 alkyl or C3-C7 cycloalkyl;

wherein R' and R" are independently H or C1-C6 alkyl; or R' and R" together, optionally attaching to N or O atom, may form a 4- to 8- membered cyclic structure; and

wherein $R_7$ is H, halo, -OH, -CN, -COOR', -OR', -SR', -OC(O)R', -NHR', -NR'R", - NHC(O)R', -NHC(O)NR'R", -C(O)NR'R", $-NS(O)_2R'$, $-S(O)_2NR'R"$, $-S(O)_2R'$, C1-C6 alkyl, C3-C7 cycloalkyl, aryl, or a C5-C10 membered heterocycle, wherein the C1-C6 alkyl, C3-C7 cycloalkyl, aryl, or C5-C10 membered heterocycle is optionally substituted with halo, -OH, -CN, -COOR', -OR', -SR', -OC(O)R', -NHR', -NR'R", -NHC(O)R', -NHC(O)NR'R", -C(O)NR'R", - $NS(O)_2R'$, $-S(O)_2NR'R"$, $-S(O)_2R'$, guanidino, nitro, nitroso, C1-C6 alkyl, aryl, C3-C7 cycloalkyl, or a 3- to 10-membered heterocycle.

**5.** The compound of any one of Claims 1-4, wherein $R_1$ is H.

**6.** The compound of any one of Claims 1-5, wherein $R_2$ is H.

**7.** The compound of any one of Claims 1-6, wherein R is methyl with *R* configuration or hydroxymethyl with *S* configuration.

**8.** The compound of any one of Claims 1-7, wherein $R_3$ is H, C3-C7 cycloalkyl or C3-C7 cycloalkyl methyl, and wherein the C3-C7 cycloalkyl or C3-C7 cycloalkyl methyl is optionally substituted with halo, -OH, -CN, -COOR', -OR', -SR', -OC(O)R', -NHR', -NR'R", - CNR'R", -NHC(O)R', -NHC(O)NR'R", -C(O)NR'R", -NS(O)$_2$R', -S(O)$_2$NR'R", -S(O)$_2$R', guanidino, nitro, nitroso, C1-C6 alkyl, aryl, C3-C7 cycloalkyl, or a 3- to 10-membered heterocycle.

**9.** The compound of any one of Claims 1-7, wherein $R_3$ is H, cyclohexyl, cyclopentyl, cyclobutyl, cyclopropyl, cyclohexyl methyl, cyclopentyl methyl, cyclobutyl methyl, cyclopropyl methyl, phenyl, or benzyl, wherein the cyclohexyl, cyclopentyl, cyclobutyl, cyclopropyl, cyclohexyl methyl, cyclopentyl methyl, cyclobutyl methyl, cyclopropyl methyl, phenyl, or benzyl is optionally substituted with halo, -OH, -CN, -COOR', -OR', -SR', - OC(O)R', -NHR', -NR'R", -CNR'R", -NHC(O)R', -NHC(O)NR'R", -C(O)NR'R", -NS(O)$_2$R', -S(O)$_2$NR'R", -S(O)$_2$R', guanidino, nitro, nitroso, C1-C6 alkyl, aryl, C3-C7 cycloalkyl, or a 3- to 10-membered heterocycle.

**10.** The compound of any one of Claims 1, 2, and 5-9, wherein $Z_2$ is pyridine or pyrazole.

**11.** The compound of Claim 1, selected from the group consisting of:

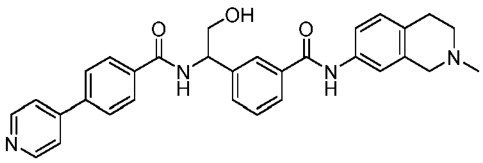 (2), 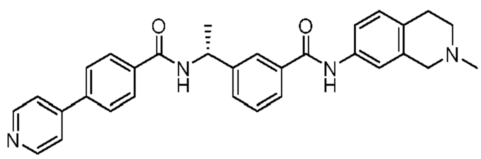 (6),

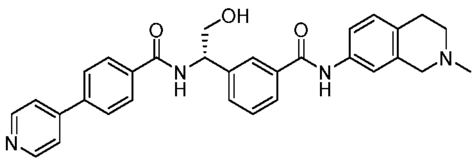 (7), 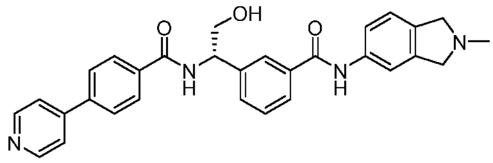 (8),

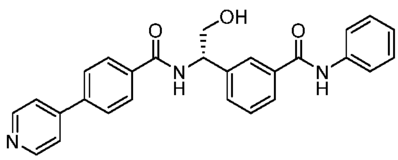 (9), 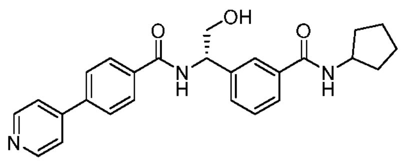 (10),

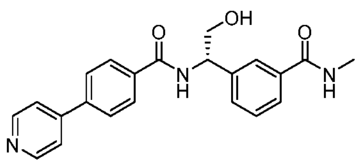 (11), 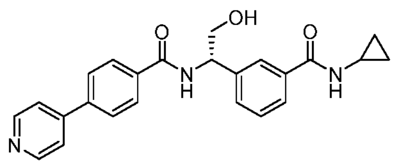 (12),

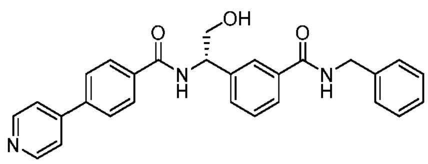 (13), 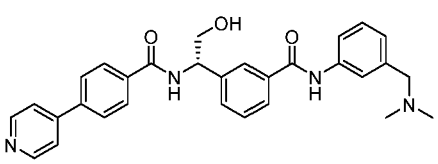 (14),

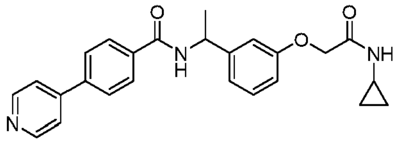 (15), 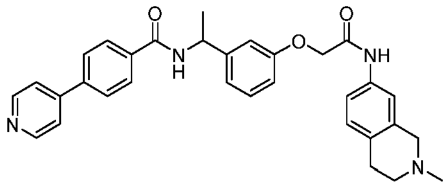 (16),

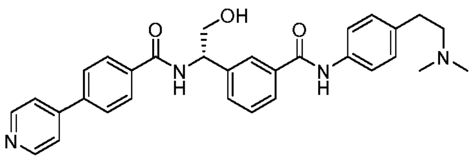 (20), 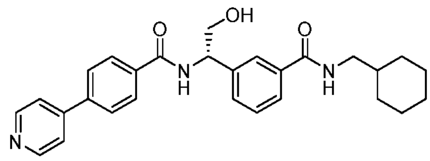 (21),

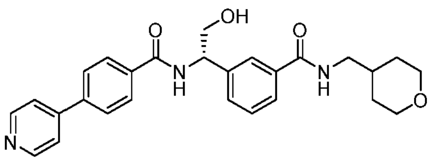 (22), 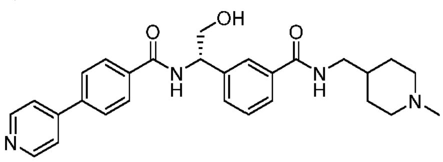 (23),

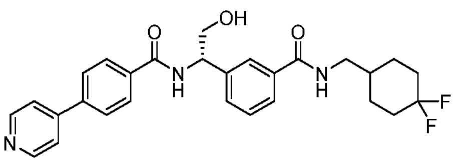 (24), 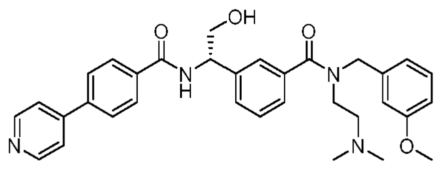 (25),

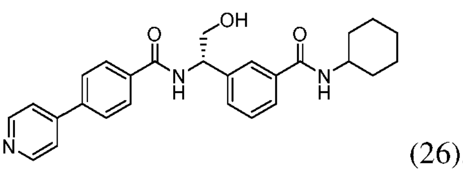 (26), 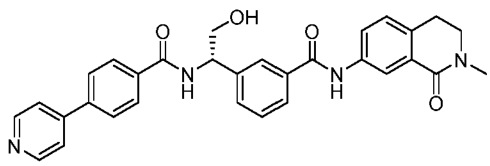 (27),

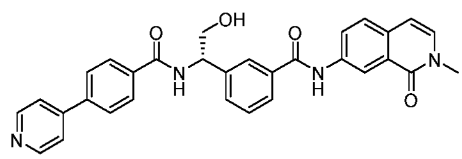 (28), 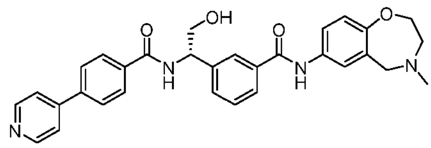 (29),

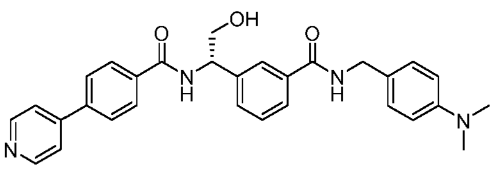 (30), 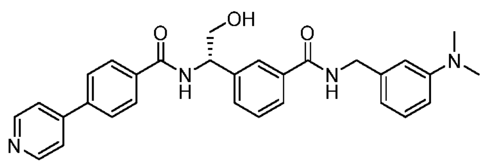 (31),

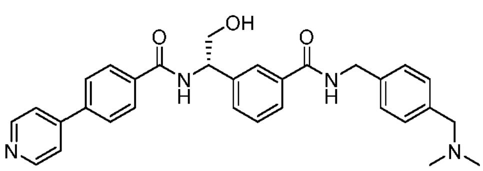 (32), 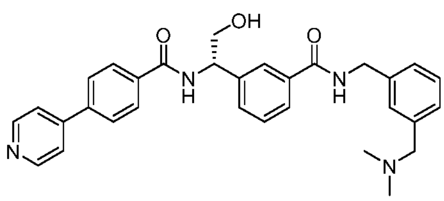 (33),

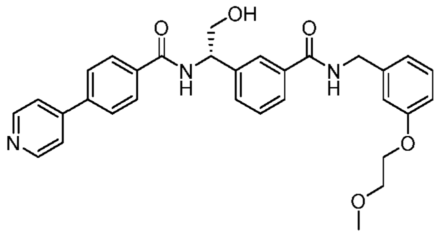 (34), 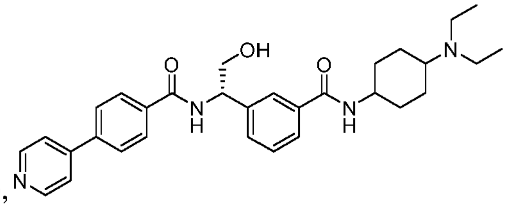 (35),

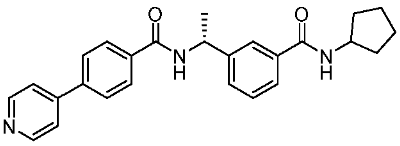 (36), 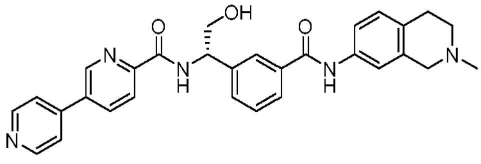 (37),

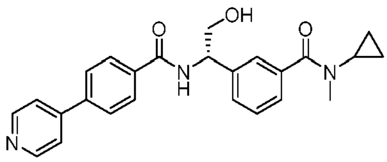 (38), 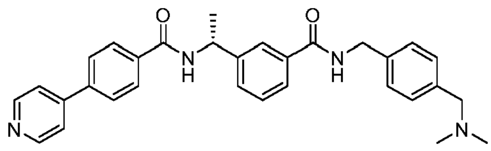 (39),

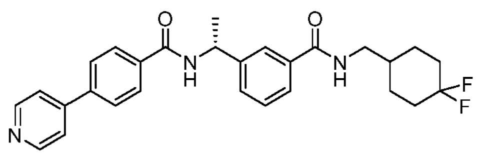 (40), 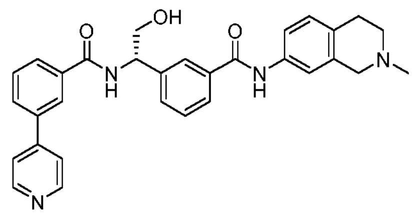 (41),

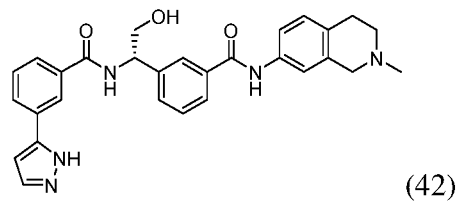 (42), 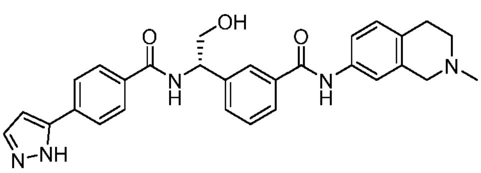 (43),

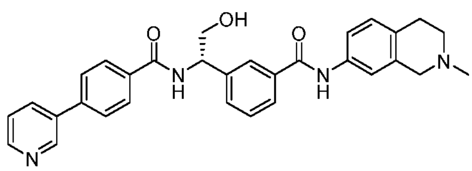 (44), 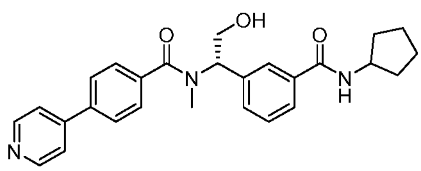 (45),

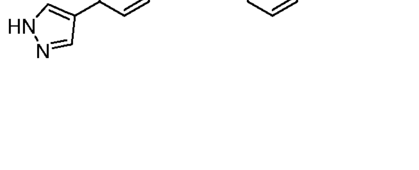 (46), 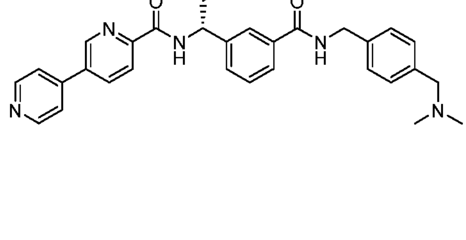 (47),

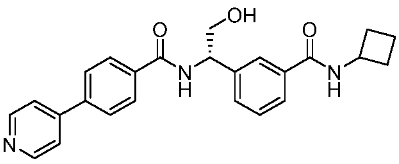 (48), 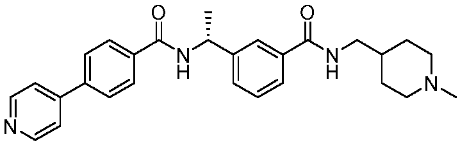 (49),

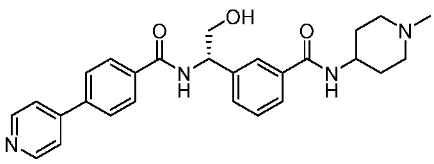 (50), 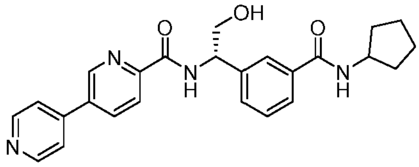 (51),

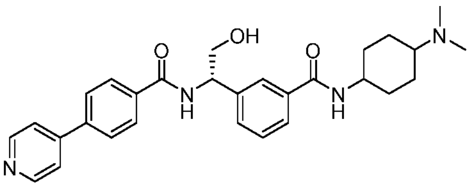 (52), 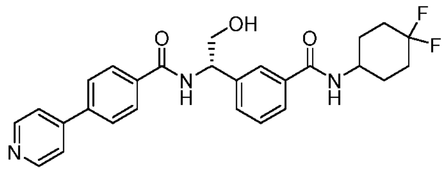 (53),

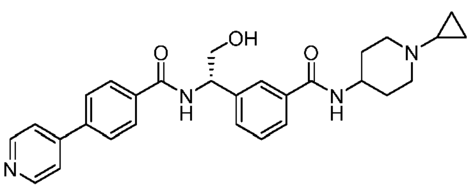 (54), 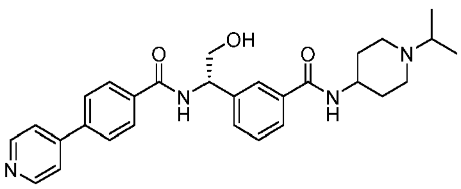 (55),

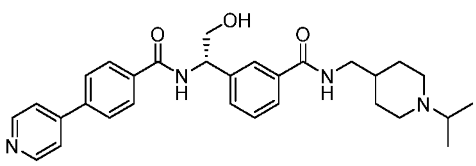 (56), 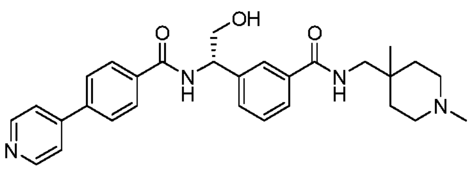 (57),

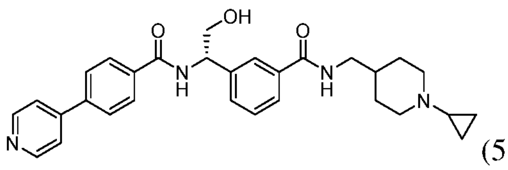 (58), 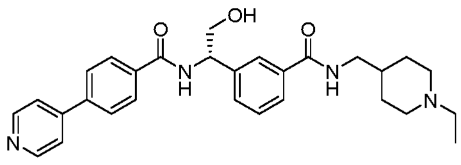 (59),

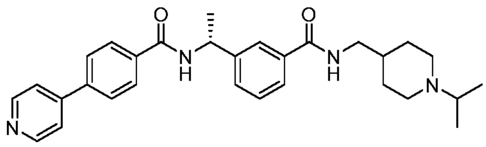 (60), 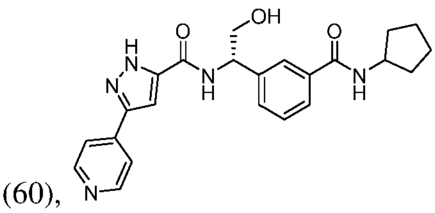 (61),

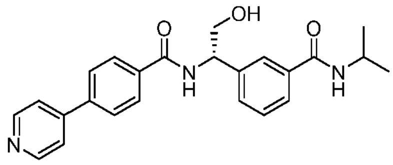 (62), 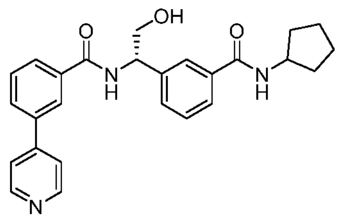 (63),

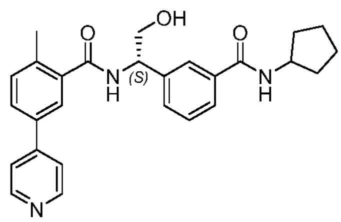 (64), 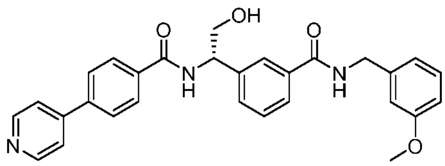 (65),

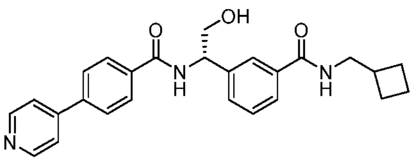 (66), 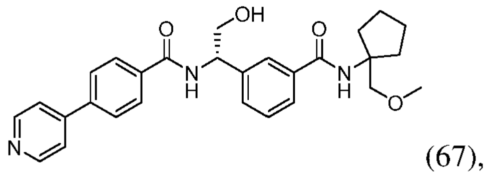 (67),

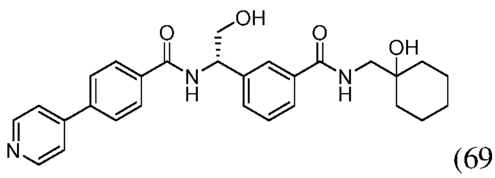 (69), 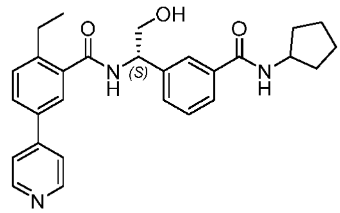 (70),

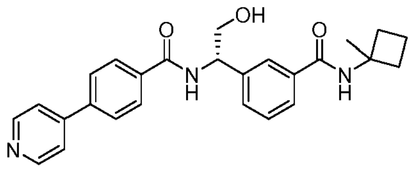 (71), 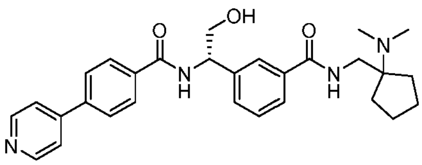 (72),

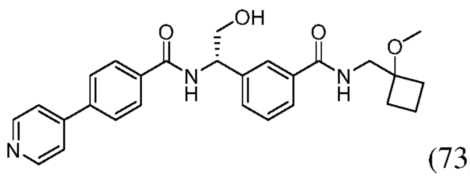 (73), 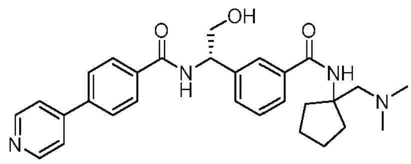 (74),

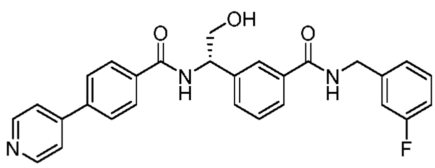 (75), 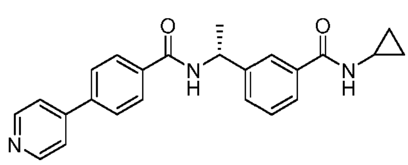 (76),

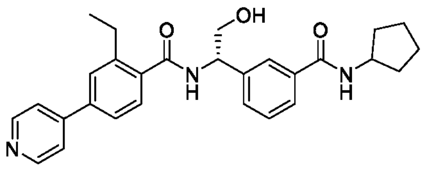, 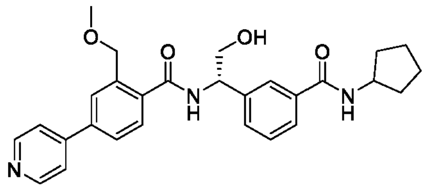,

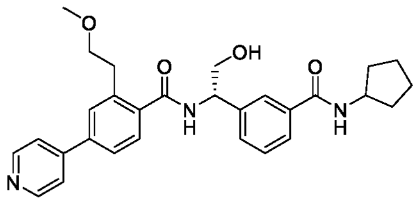, 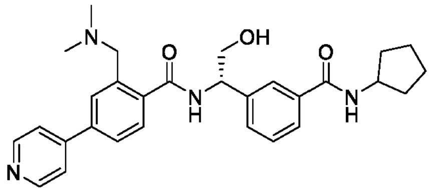,

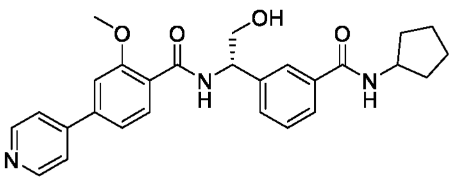, 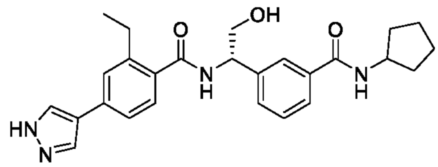,

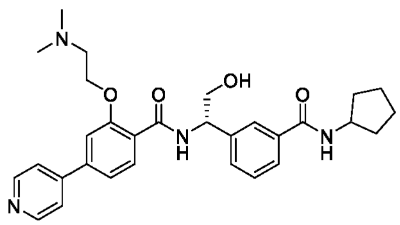, 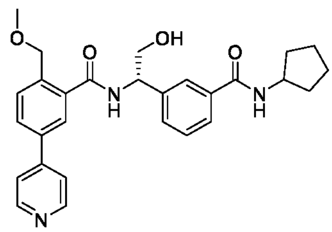, 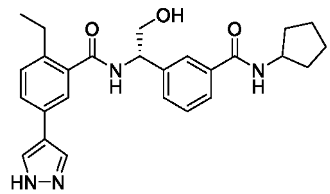,

and

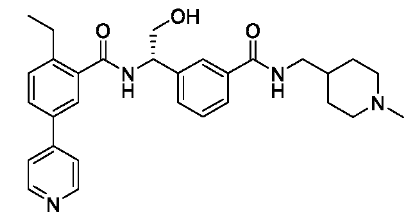.

**12.** The compound of Claim 11, selected from the group consisting of:

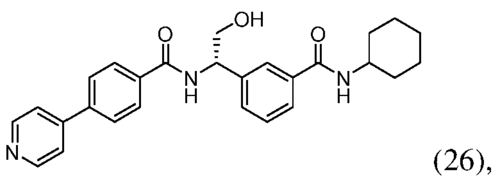 (26), 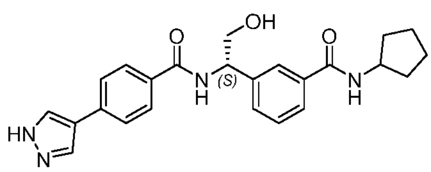 (46),

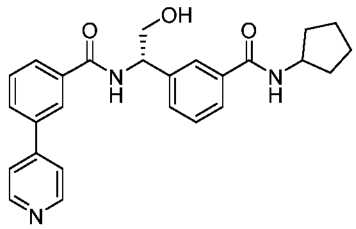
(63), and
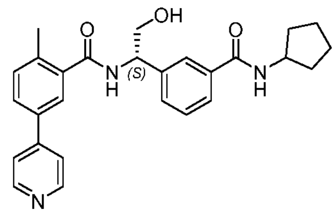
(64).

**13.** The compound of Claim 11, wherein the compound consists of

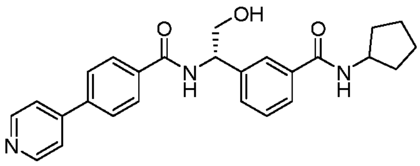
(10).

**14.** A pharmaceutical composition comprising a compound according to any one of Claims 1 to 13 and a pharmaceutically acceptable carrier.

**15.** The compound of any one of Claims 1 to 13, or the pharmaceutical composition of Claim 14 for use in the prevention or treatment of a disease associated with Rho-associated protein kinase modulation in a subject, wherein

the disease is selected from the group consisting of: cancer, a neurodegenerative disease, a neurodevelopmental disorder, an inflammatory or autoimmune disease, an infection, a metabolic disease, a cardiovascular disease or vascular smooth muscle dysfunction, a hematologic disease, a hematologic malignant neoplastic disorder, and an ocular disorder; wherein
cancer is selected from the group consisting of: cutaneous T-cell lymphoma (CTCL), peripheral T-cell lymphoma (PTCL), angioimmunoblastic T-cell lymphoma (AITL), multiple myeloma, leukemia, lymphoma, lung cancer, ovarian cancer, breast cancer, gastric cancer, prostate cancer, pancreatic cancer, hepatocellular cancer, and renal cancer;
the neurodegenerative disease is selected from the group consisting of: Alzheimer's, Huntington's, Parkinson's, Amyotrophic Lateral Sclerosis, and spinal muscular atrophy;
the neurodevelopmental disorder is Rett syndrome or Niemann-Pick disease type C;
the inflammatory or autoimmune disease is selected from the group consisting of: asthma, cardiovascular inflammation, renal inflammation, arteriosclerosis, rheumatoid arthritis, spondylitis arthritis, psoriatic arthritis, psoriasis, atopic dermatitis, eczema, multiple sclerosis, systemic lupus erythematosus, inflammatory bowel diseases, Crohn's disease, graft versus host disease, transplant rejection, and fibrotic disease;
the cardiovascular disease or vascular smooth muscle dysfunction is selected from the group consisting of: hypertension, atherosclerosis, restenosis, cardiac hypertrophy, ocular hypertension, cerebral ischemia, cerebral vasospasm, and erectile dysfunction;
the hematologic malignant neoplastic disorder is **characterized by** deregulated FLT3 receptor tyrosine kinase activity, and is selected from the group consisting of: leukemia, myeloma, myeloproliferative disease, myelodysplastic syndrome, Hodgkin's disease, myeloma, acute lymphocytic leukemia (ALL), acute myeloid leukemia (AML), acute promyelocytic leukemia (APL), chronic lymphocytic leukemia (CLL), chronic myeloid leukemia (CML), chronic neutrophilic leukemia (CNL), acute undifferentiated leukemia (AUL), anaplastic large-cell lymphoma (ALCL), prolymphocytic leukemia (PML), juvenile myelomonocytic leukemia (JMML), adult T-cell ALL, AML, with trilineage myelodysplasia (AMLITMDS), mixed lineage leukemia (MLL), myelodysplastic syndromes (MDSs), myeloproliferative disorders (MPD), and multiple myeloma (MM); and
the ocular disorder is selected from the group consisting of: age related macular degeneration (AMD), choroidal neovascularization (CNV), diabetic macular edema (DME), iris neovascularization, uveitis, neo vascular glaucoma, and retinitis of prematurity (ROP).

## Patentansprüche

**1.** Verbindung der Formel (I):

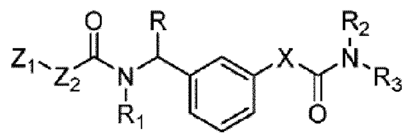

I

wobei:

$Z_1$ Pyridin oder Pyrazol ist, wobei das Pyridin oder das Pyrazol optional mit Halogen, - OH, -CN, -COOR’, -OR’, -SR’, -OC(O)R’, -NHR’, -NR’R", -NHC(O)R’, -NHC(O)NR’R", - C(O)NR’R", $-NS(O)_2R'$, $-S(O)_2NR'R''$, $-S(O)_2R'$, Guanidino, Nitro, Nitroso, C1-C6-Alkyl, Aryl, C3-C7-Cycloalkyl oder einem 3- bis 10-gliedrigen Heterocyclus substituiert ist, wobei das C1-C6-Alkyl, Aryl, C3-C7-Cycloalkyl oder ein 3- bis 10-gliedriger Heterocyclus unsubstituiert oder mit einem oder mehreren der Folgenden substituiert ist: Halogen, -OH, -CN, -COOR’, -OR’, -SR’, - OC(O)R’, -NHR’, -NR’R", -NHC(O)R’, -NHC(O)NR’R", -C(O)NR’R", $-NS(O)_2R'$, $-S(O)_2NR'R''$, - $S(O)_2R'$, Guanidino, Nitro, Nitroso, C1-C6-Alkyl, Aryl oder C3-C7-Cycloalkyl;

$Z_2$ Phenyl, Naphthyl oder ein C5-C10-gliedriger Heterocyclus ist, wobei das Phenyl, Naphthyl oder der C5-C10-gliedrige Heterocyclus optional mit Halogen, -OH, -CN, -COOR’, - OR’, -SR’, -OC(O)R’, -NHR’, -NR’R", -NHC(O)R’, -NHC(O)NR’R", -C(O)NR’R", $-NS(O)_2R'$, - $S(O)_2NR'R''$, $-S(O)_2R'$, Guanidino, Nitro, Nitroso, C1-C6-Alkyl, Aryl, C3-C7-Cycloalkyl oder einem 3- bis 10- gliedrigen Heterocyclus substituiert ist, wobei das C1-C6-Alkyl, Aryl, C3-C7-Cycloalkyl oder der 3- bis 10-gliedrige Heterocyclus unsubstituiert oder mit einem oder mehreren der Folgenden substituiert ist: Halogen, -OH, -CN, -COOR’, -OR’, -SR’, -OC(O)R’, -NHR’, - NR’R", -NHC(O)R’, -NHC(O)NR’R", -C(O)NR’R", $-NS(O)_2R'$, $-S(O)_2NR'R''$, $-S(O)_2R'$, Guanidino, Nitro, Nitroso, C1-C6-Alkyl, Aryl oder C3-C7-Cycloalkyl;

R-C1-C6-Alkyl ist, optional substituiert mit einem oder mehreren -OH;

$R_1$ H, C1-C6-Alkyl oder C3-C7-Cycloalkyl ist, wobei das C1-C6-Alkyl oder C3-C7-Cycloalkyl optional mit einem oder mehreren der Folgenden substituiert ist: Halogen, -OH, -CN, -COOR’, -OR’, -SR’, -OC(O)R’, -NHR’, -NR’R", -NHC(O)R’, -NHC(O)NR’R", -C(O)NR’R", - $NS(O)_2R'$, $-S(O)_2NR'R''$ oder $-S(O)_2R'$;

X eine Bindung oder -O(C1-C6-Alkyl) ist; und

$R_2$ und $R_3$ unabhängig H, C1-C6-Alkyl oder C3-C7-Cycloalkyl, Aryl oder C5-C10-gliedriger Heterocyclus,

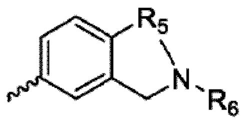,
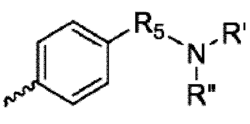,
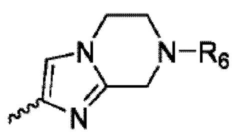,

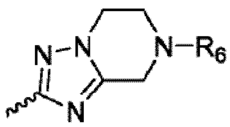
oder
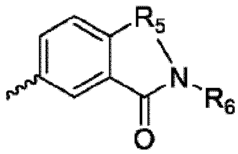

sind, wobei das C1-C6-Alkyl, C3-C7-Cycloalkyl, Aryl- oder der C5-C10-gliedrige Heterocyclus optional mit Halogen, -OH, -CN, -COOR’, -OR’, -SR’, -OC(O)R’, -NHR’, -NR’R", -CNR’R", - NHC(O)R’, -NHC(O)NR’R", -C(O)NR’R", $-NS(O)_2R'$, $-S(O)_2NR'R''$, $-S(O)_2R'$, Guanidino, Nitro, Nitroso, C1-C6-Alkyl, Aryl, C3-C7-Cycloalkyl oder 3- bis 10-gliedrigem Heterocyclus substituiert ist, wobei das C1-C6-Alkyl, Aryl, C3-C7-Cycloalkyl oder der 3- bis 10-gliedrige Heterocyclus unsubstituiert oder mit einem oder mehreren der Folgenden substituiert ist: Halogen, -OH, -CN, -COOR’, -OR’, -SR’, -OC(O)R’, -NHR’, -NR’R", -NHC(O)R’, -NHC(O)NR’R", - C(O)NR’R", $-NS(O)_2R'$, $-S(O)_2NR'R''$, $-S(O)_2R'$, Guanidino, Nitro, Nitroso, C1-C6-Alkyl, Aryl oder C3-C7-Cycloalkyl; wobei $R_5$ C1-C6-Alkyl, $-OCH_2CH_2-$, $-NR_6CH_2CH_2-$ oder $-NC(O)CH_2CH_2-$ ist; und $R_6$ H, C1-C6-Alkyl oder C3-C7-Cycloalkyl ist; und

wobei R’ und R" unabhängig H oder C1-C6-Alkyl sind; und wobei R’ und R" zusammen, optional an ein N- oder O-Atom gebunden, eine 4- bis 8-gliedrige zyklische Struktur bilden können.

**2.** Verbindung nach Anspruch 1, wobei R $-CH_3$ oder $-CH_3OH$ ist.

**3.** Verbindung nach Anspruch 1 mit einer Struktur der Formel (III):

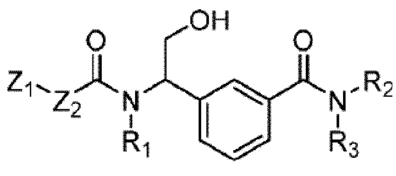

III ,

wobei:

$Z_1$ Pyridin oder Pyrazol ist, wobei das Pyridin oder Pyrazol optional mit Halogen, -OH, - CN, -COOR', -OR', -SR', -OC(O)R', -NHR', -NR'R", -NHC(O)R', -NHC(O)NR'R", -C(O)NR'R", -NS(O)$_2$R', -S(O)$_2$NR'R", -S(O)$_2$R', Guanidino, Nitro, Nitroso, C1-C6-Alkyl, Aryl, C3-C7-Cycloalkyl oder einem 3- bis 10-gliedrigen Heterocyclus substituiert ist, wobei das C1-C6-Alkyl, Aryl, C3-C7-Cycloalkyl oder der 3- bis 10-gliedrige Heterocyclus unsubstituiert oder mit einem oder mehreren der Folgenden substituiert ist: Halogen, -OH, -CN, -COOR', -OR', -SR', -OC(O)R', -NHR', -NR'R", -NHC(O)R', -NHC(O)NR'R", -C(O)NR'R", -NS(O)$_2$R', -S(O)$_2$NR'R", -S(O)$_2$R', Guanidino, Nitro, Nitroso, C1-C6-Alkyl, Aryl oder C3-C7-Cycloalkyl;

$Z_2$ Phenyl, Naphthyl oder ein C5-C10-gliedriger Heterocyclus ist, wobei das Phenyl, Naphthyl oder der C5-C10-gliedrige Heterocyclus optional mit Halogen, -OH, -CN, -COOR', - OR', -SR', -OC(O)R', -NHR', -NR'R", -NHC(O)R', -NHC(O)NR'R", -C(O)NR'R", -NS(O)$_2$R', - S(O)$_2$NR'R", -S(O)$_2$R', Guanidino, Nitro, Nitroso, C1-C6-Alkyl, Aryl, C3-C7-Cycloalkyl oder einem 3- bis 10 -gliedrigen Heterocyclus substituiert ist, wobei das C1-C6-Alkyl, Aryl, C3-C7-Cycloalkyl oder der 3- bis 10-gliedrige Heterocyclus unsubstituiert oder mit einem oder mehreren der Folgenden substituiert ist: Halogen, -OH, -CN, -COOR', -OR', -SR', -OC(O)R', -NHR', - NR'R", -NHC(O)R', -NHC(O)NR'R", -C(O)NR'R", -NS(O)$_2$R', -S(O)$_2$NR'R", -S(O)$_2$R', Guanidino, Nitro, Nitroso, C1-C6-Alkyl, Aryl oder C3-C7-Cycloalkyl;

$R_1$ H, C1-C6-Alkyl oder C3-C7-Cycloalkyl ist, wobei das C1-C6-Alkyl oder C3-C7-Cycloalkyl optional mit einem oder mehreren der Folgenden substituiert ist: Halogen, -OH, -CN, -COOR', - OR', -SR', -OC(O)R', -NHR', -NR'R", -NHC(O)R', -NHC(O)NR'R", -C(O)NR'R", - NS(O)$_2$R', -S(O)$_2$NR'R" oder -S(O)$_2$R'; und

$R_2$ und $R_3$ unabhängig H, C1-C6-Alkyl, C3-C7-Cycloalkyl, Aryl oder C5-C10-gliedriger Heterocyclus,

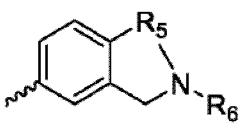 , 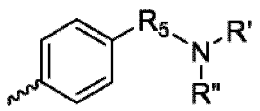 , 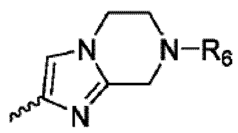 , 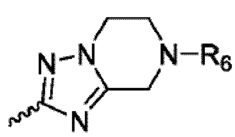

oder

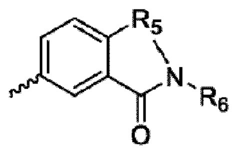

sind, wobei das $C_1$-$C_6$-Alkyl-, $C_3$-$C_7$-Cycloalkyl-, Aryl- oder der C5-C10-gliedrige Heterocyclus optional mit Halogen, -OH, -CN, -COOR', -OR', -SR', - OC(O)R', -NHR', -NR'R", -CNR'R", -NHC(O)R', -NHC(O)NR'R", -C(O)NR'R", -NS(O)$_2$R', -S(O)$_2$NR'R", - S(O)$_2$R', Guanidino, Nitro, Nitroso, C1-C6-Alkyl, Aryl, C3-C7-Cycloalkyl oder einem 3- bis 10-gliedrigen Heterocyclus substituiert ist, wobei das C1-C6-Alkyl, Aryl, C3-C7-Cycloalkyl oder der 3- bis 10-gliedrige Heterocyclus unsubstituiert oder mit einem oder mehreren der Folgenden substituiert ist: Halogen, -OH, -CN, -COOR', -OR', -SR', -OC(O)R', -NHR', -NR'R", -NHC(O)R', -NHC(O)NR'R", -C(O)NR'R", -NS(O)$_2$R', -S(O)$_2$NR'R", -S(O)$_2$R', Guanidino, Nitro, Nitroso, C1-C6-Alkyl, Aryl oder C3-C7-Cycloalkyl;

wobei $R_5$ C1-C6-Alkyl, -$OCH_2CH_2$-, -$NR_6CH_2CH_2$- oder -NC(O)$CH_2CH_2$- ist; und $R_6$ H, C1-C6-Alkyl oder C3-C7-Cycloalkyl ist; und

wobei R' und R" unabhängig H oder C1-C6-Alkyl sind; oder R' und R" zusammen, optional an ein N- oder O-Atom gebunden, eine 4- bis 8-gliedrige cyclische Struktur bilden können.

**4.** Verbindung nach Anspruch 3 mit einer Struktur der Formel (IV):

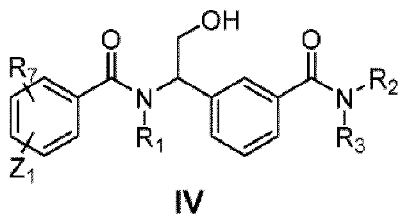

**IV**

wobei:

$Z_1$ Pyridin oder Pyrazol ist, wobei das Pyridin oder Pyrazol optional mit Halogen, -OH, - CN, -COOR', -OR', -SR', -OC(O)R', -NHR', -NR'R", -NHC(O)R', -NHC(O)NR'R", -C(O)NR'R", -NS(O)$_2$R', -S(O)$_2$NR'R", -S(O)$_2$R', Guanidino, Nitro, Nitroso, C1-C6-Alkyl, Aryl, C3-C7-Cycloalkyl oder einem 3- bis 10-gliedrigen Heterocyclus substituiert ist, wobei C1-C6-Alkyl, Aryl, C3- C7-Cycloalkyl oder ein 3- bis 10-gliedriger Heterocyclus unsubstituiert oder mit einem oder mehreren der Folgenden substituiert ist: Halogen, -OH, -CN, -COOR', -OR', -SR', -OC(O)R', -NHR', -NR'R", -NHC(O)R', -NHC(O)NR'R", -C(O)NR'R", -NS(O)$_2$R', -S(O)$_2$NR'R", -S(O)$_2$R', Guanidino, Nitro, Nitroso, C1-C6-Alkyl, Aryl oder $C_3$-$C_7$-Cycloalkyl;

$R_1$ H, C1-C6-Alkyl oder C3-C7-Cycloalkyl ist, wobei das C1-C6-Alkyl oder C3-C7-Cycloalkyl optional mit einem oder mehreren der Folgenden substituiert ist: Halogen, -OH, -CN, -COOR', -OR', -SR', -OC(O)R', -NHR', -NR'R", -NHC(O)R', -NHC(O)NR'R", -C(O)NR'R", - NS(O)$_2$R', -S(O)$_2$NR'R" oder -S(O)$_2$R'; und

$R_2$ und $R_3$ unabhängig H, C1-C6-Alkyl, C3-C7-Cycloalkyl, Aryl oder C5-C10-gliedriger Heterocyclus,

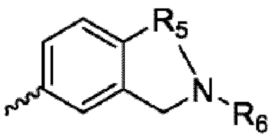 , 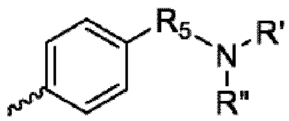 , 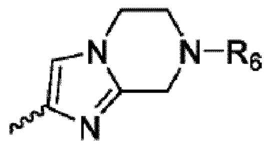 , 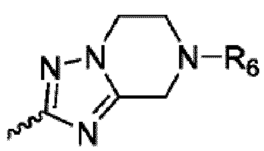 ,

oder

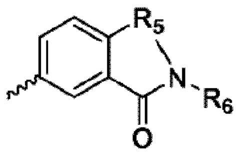

sind, wobei das $C_1$-$C_6$-Alkyl, $C_3$-$C_7$-Cycloalkyl, Aryl- oder der C5-C10-gliedrige Heterocyclus optional mit Halogen, -OH, -CN, -COOR', -OR', -SR', -OC(O)R', -NHR', - NR'R", -CNR'R", -NHC(O)R', -NHC(O)NR'R", -C(O)NR'R", -NS(O)$_2$R', -S(O)$_2$NR'R", -S(O)$_2$R', Guanidino, Nitro, Nitroso, C1-C6-Alkyl, Aryl, C3-C7-Cycloalkyl oder einem 3- bis 10-gliedrigen Heterocyclus substituiert ist, wobei das C1-C6-Alkyl, Aryl, C3-C7-Cycloalkyl oder der 3- bis 10-gliedrige Heterocyclus unsubstituiert oder mit einem oder mehreren der Folgenden substituiert ist: Halogen, -OH, -CN, -COOR', -OR', -SR', -OC(O)R', -NHR', -NR'R", -NHC(O)R', - NHC(O)NR'R", - C(O)NR'R", -NS(O)$_2$R', -S(O)$_2$NR'R", -S(O)$_2$R', Guanidino, Nitro, Nitroso, C1-C6-Alkyl, Aryl, oder C3-C7-Cycloalkyl;

wobei $R_5$ C1-C6-Alkyl, -OCH$_2$CH$_2$-, -NR$_6$CH$_2$CH$_2$- oder -NC(O)CH$_2$CH$_2$- ist; und $R_6$ H, C1-C6-Alkyl oder C3-C7-Cycloalkyl ist;

wobei R' und R" unabhängig H oder C1-C6-Alkyl sind; oder R' und R" zusammen, optional an ein N- oder O-Atom gebunden, eine 4- bis 8-gliedrige zyklische Struktur bilden können; und

wobei $R_7$ H, Halogen, -OH, -CN, -COOR', -OR', -SR', -OC(O)R', -NHR', -NR'R", - NHC(O)R', -NHC(O)NR'R", -C(O)NR'R", -NS(O)$_2$R', -S(O)$_2$NR'R", -S(O)$_2$R', C1-C6-Alkyl, C3-C7-Cycloalkyl, Aryl oder ein C5-C10-gliedriger Heterocyclus ist, wobei das C1-C6-Alkyl, C3-C7-Cycloalkyl, Aryl oder der C5-C10-gliedrige Heterocyclus optional mit Halogen, -OH, -CN, - COOR', -OR', -SR', -OC(O)R', -NHR', -NR'R", -NHC(O)R', -NHC(O)NR'R", -C(O)NR'R", - NS(O)$_2$R', -S(O)$_2$NR'R", -S(O)$_2$R', Guanidino, Nitro, Nitroso, C1-C6-Alkyl, Aryl, C3-C7-Cycloalkyl oder einem 3- bis 10-gliedrigen Heterocyclus substituiert ist.

**5.** Verbindung nach einem der Ansprüche 1-4, wobei $R_1$ H ist.

**6.** Verbindung nach einem der Ansprüche 1-5, wobei $R_2$ H ist.

**7.** Verbindung nach einem der Ansprüche 1-6, wobei R Methyl mit R-Konfiguration oder Hydroxymethyl mit S-Konfiguration ist.

**8.** Verbindung nach einem der Ansprüche 1-7, wobei $R_3$ H, C3-C7-Cycloalkyl oder C3-C7-Cycloalkylmethyl ist und wobei das C3-C7-Cycloalkyl oder C3-C7-Cycloalkylmethyl optional mit Halogen, - OH, -CN, -COOR', -OR', -SR', -OC(O)R', -NHR', -NR'R", -CNR'R", -NHC(O)R', -NHC(O)NR'R", -C(O)NR'R", -NS$(O)_2$R', -S$(O)_2$NR'R", -S$(O)_2$R', Guanidino, Nitro, Nitroso, C1-C6-Alkyl, Aryl, C3-C7-Cycloalkyl oder einem 3- bis 10-gliedrigen Heterocyclus substituiert ist.

**9.** Verbindung nach einem der Ansprüche 1-7, wobei $R_3$ H, Cyclohexyl, Cyclopentyl, Cyclobutyl, Cyclopropyl, Cyclohexylmethyl, Cyclopentylmethyl, Cyclobutylmethyl, Cyclopropylmethyl, Phenyl oder Benzyl ist, wobei das Cyclohexyl, Cyclopentyl, Cyclobutyl, Cyclopropyl, Cyclohexylmethyl, Cyclopentylmethyl, Cyclobutylmethyl, Cyclopropylmethyl, Phenyl oder Benzyl optional mit Halogen, -OH, -CN, -COOR', -OR', -SR', -OC(O)R', -NHR', - NR'R", -CNR'R", -NHC(O)R', -NHC(O)NR'R", -C(O)NR'R", -NS$(O)_2$R', -S$(O)_2$NR'R", -S$(O)_2$R', Guanidino, Nitro, Nitroso, C1-C6-Alkyl, Aryl, C3-C7-Cycloalkyl oder einem 3- bis 10-gliedrigen Heterocyclus substituiert ist.

**10.** Verbindung nach einem der Ansprüche 1, 2 und 5-9, wobei $Z_2$ Pyridin oder Pyrazol ist.

**11.** Verbindung nach Anspruch 1, ausgewählt aus der Gruppe bestehend aus:

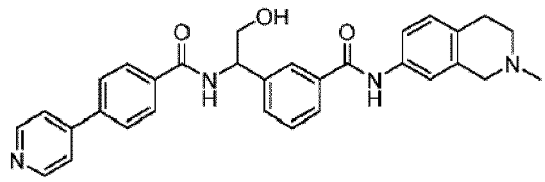
(2),
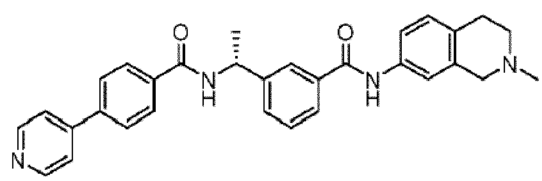
(6),

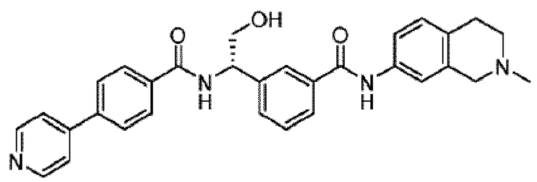
(7),
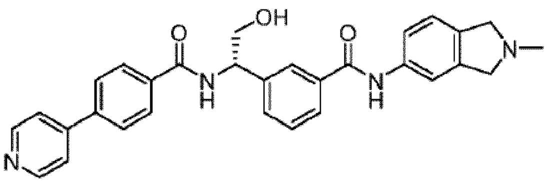
(8),

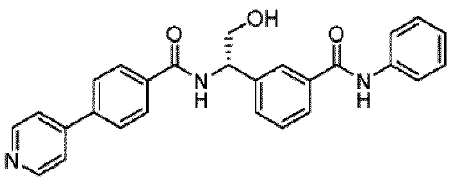
(9),
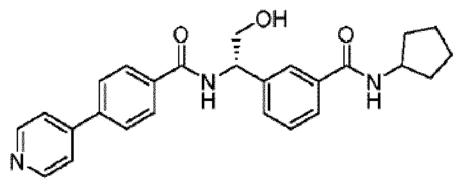
(10),

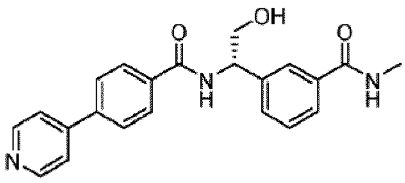
(11),
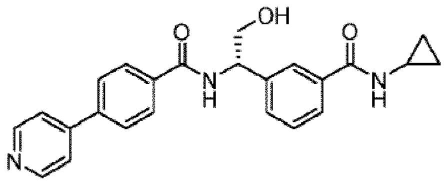
(12),

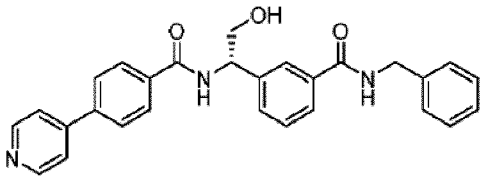 (13), 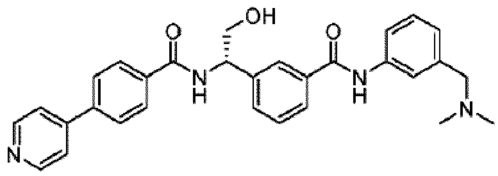 (14),

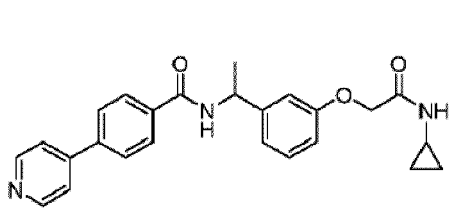 (15), 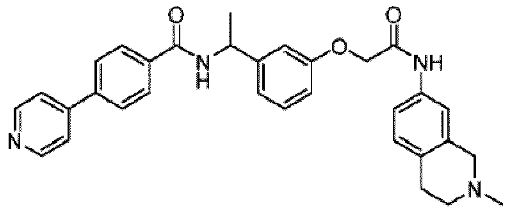 (16),

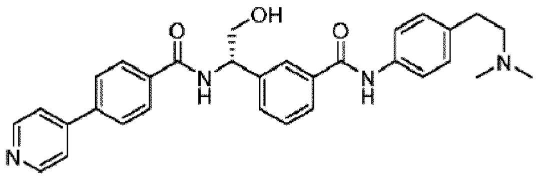 (20), 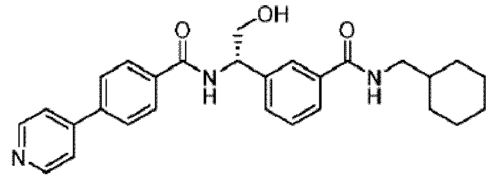 (21),

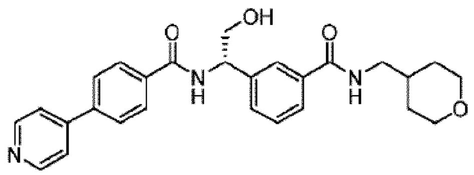 (22), 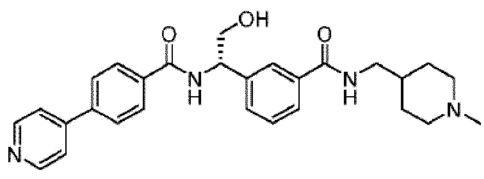 (23),

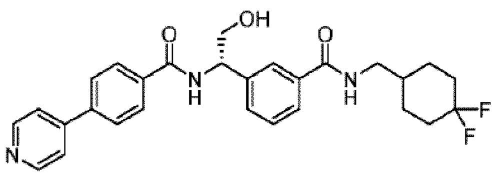 (24), 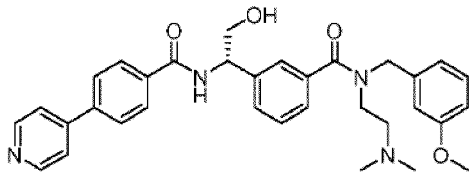 (25),

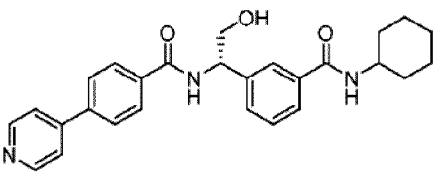 (26), 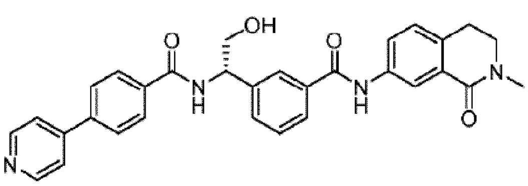 (27),

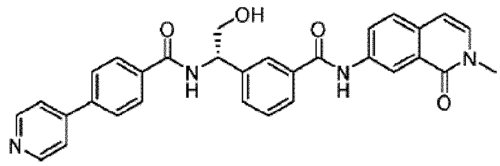 (28), 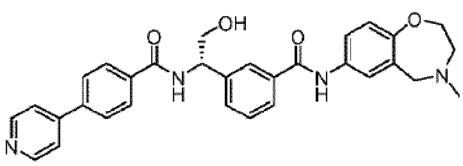 (29),

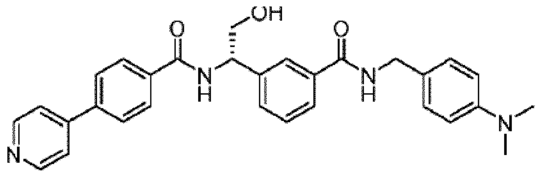 (30), 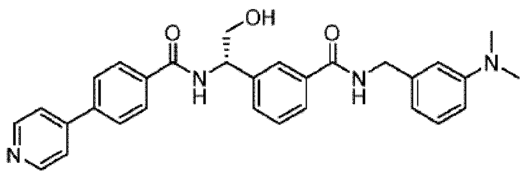 (31),

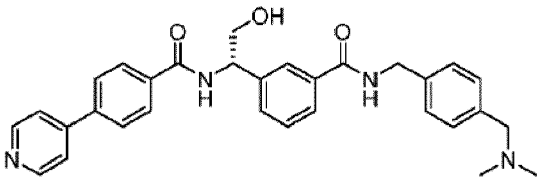 (32), 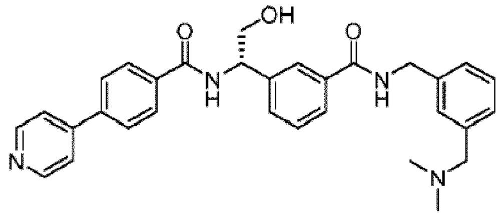 (33),

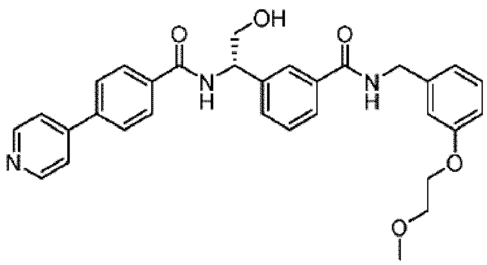 (34), 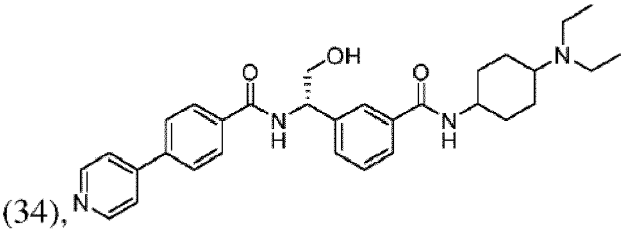 (35),

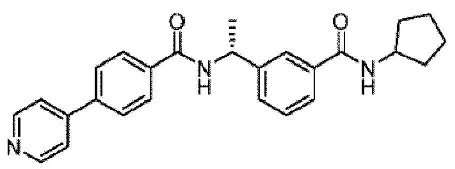 (36), 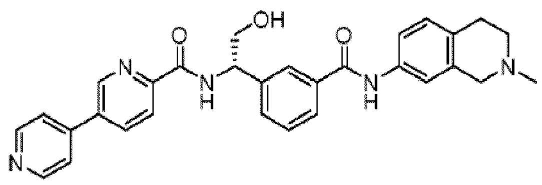 (37),

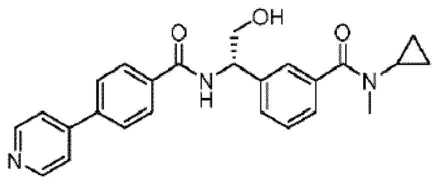 (38), 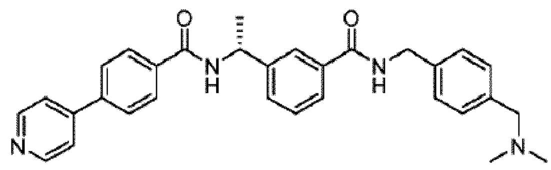 (39),

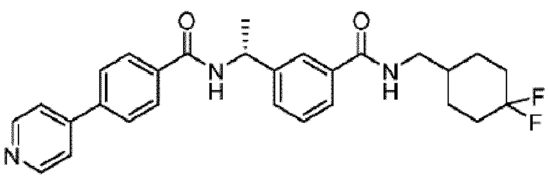 (40), 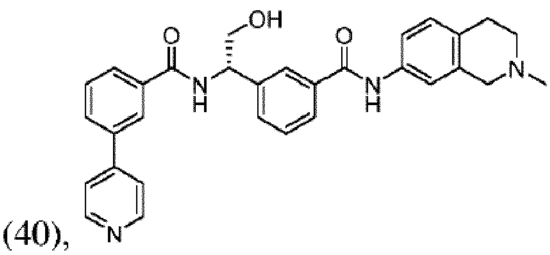 (41),

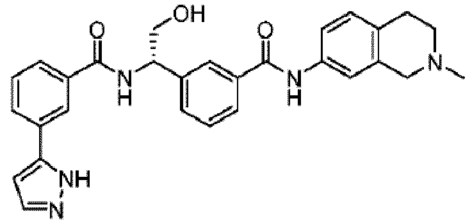
(42),
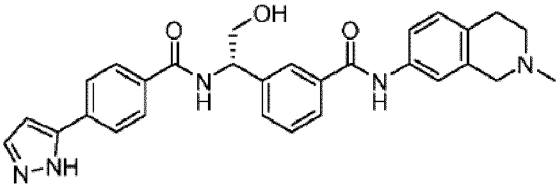
(43),
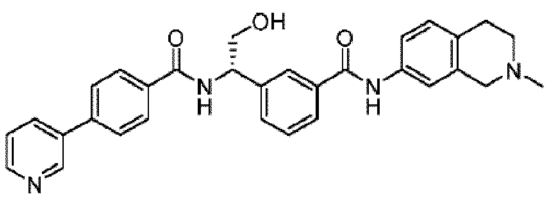
(44),
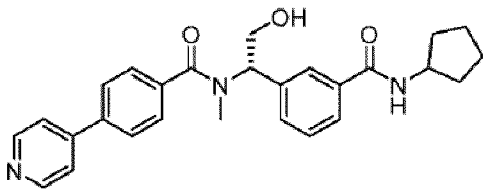
(45),
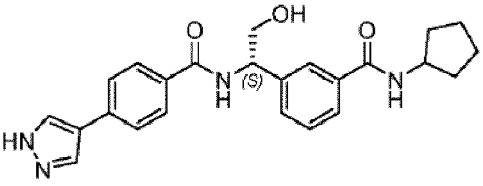
(46),
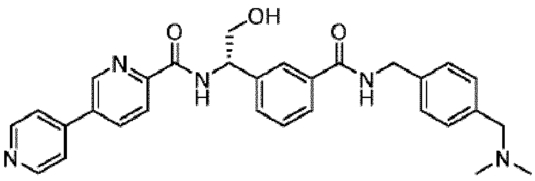
(47),
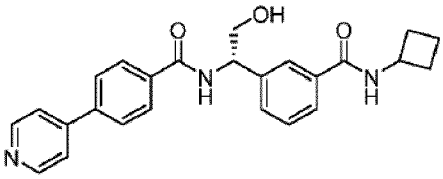
(48),
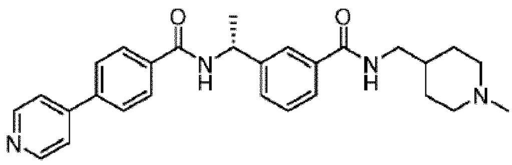
(49),
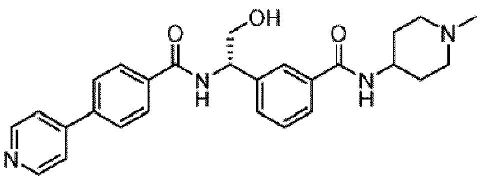
(50),
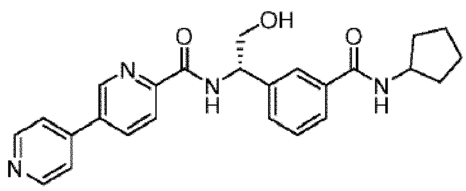
(51),
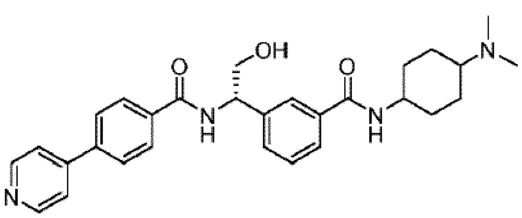
(52),
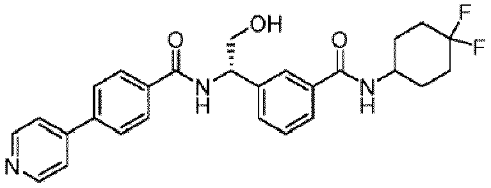
(53),
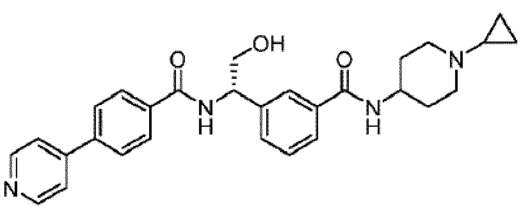
(54),
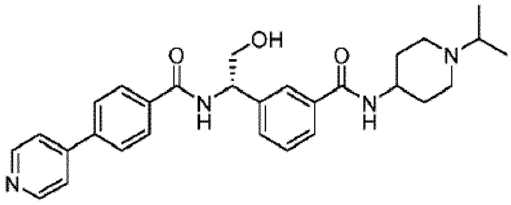
(55),

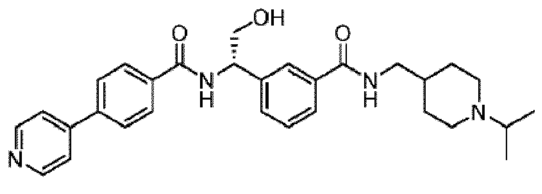
(56),
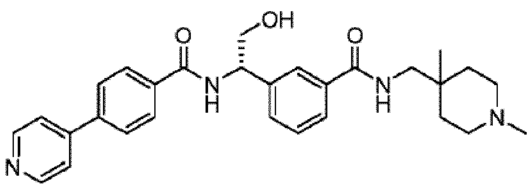
(57),

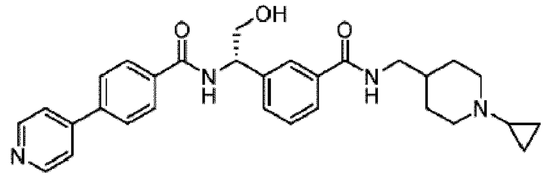
(58),
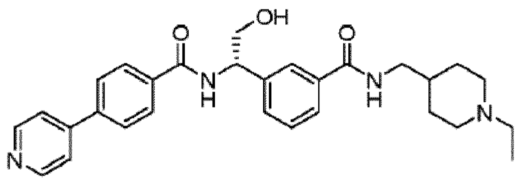
(59),

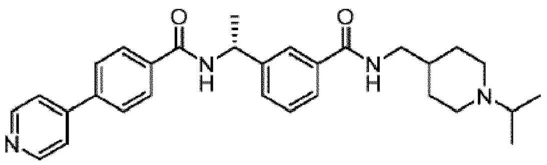
(60),
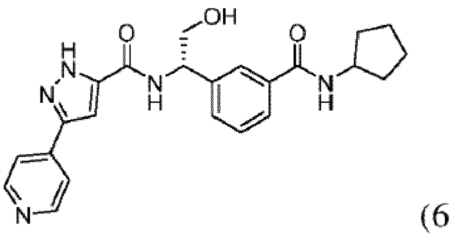
(61),

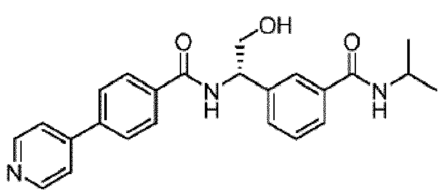
(62),
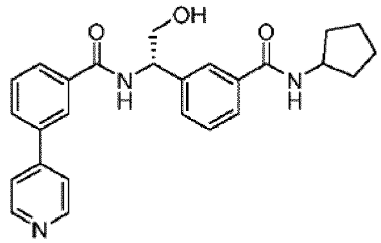
(63),

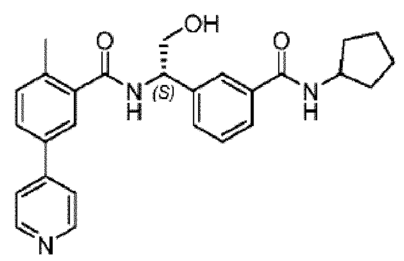
(64),
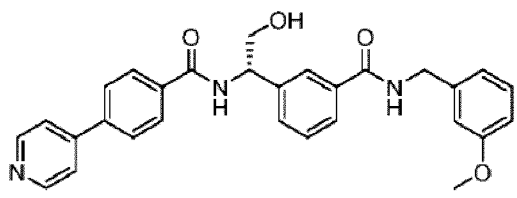
(65),

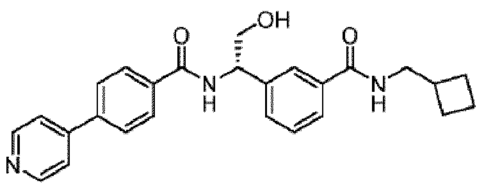
(66),
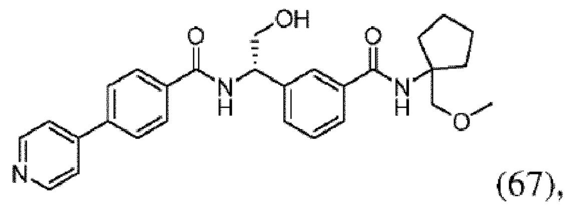
(67),

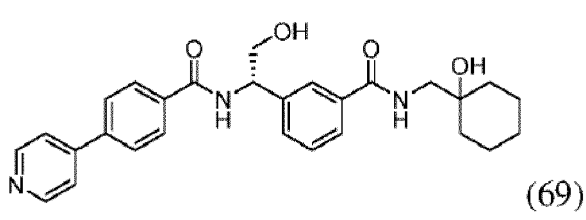 (69), 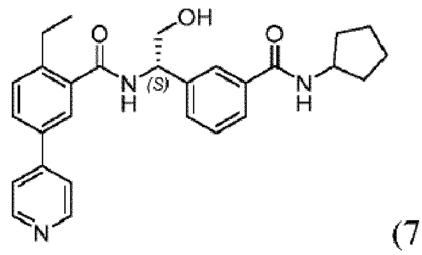 (70),

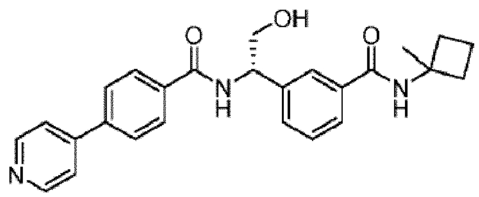 (71), 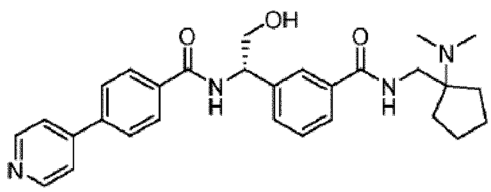 (72),

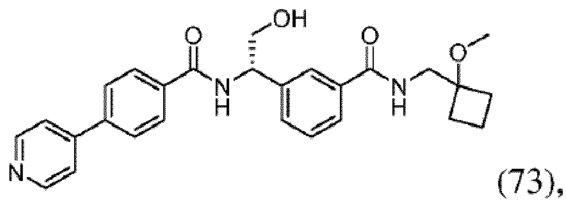 (73), 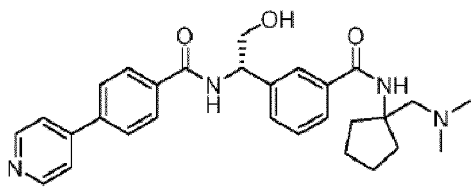 (74),

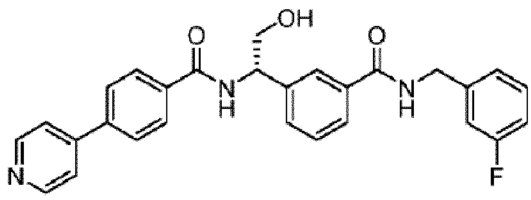 (75), 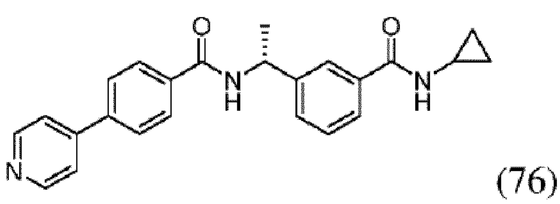 (76),

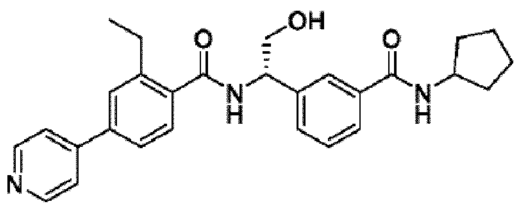 , 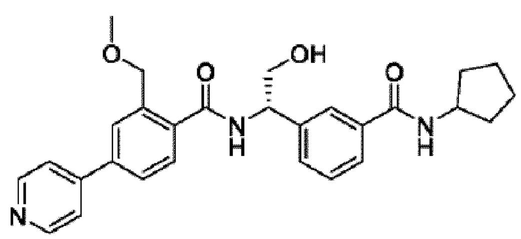 ,

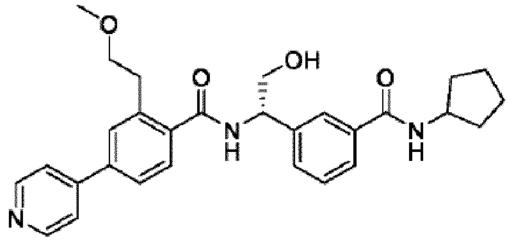 , 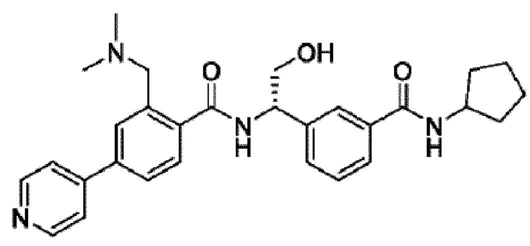 ,

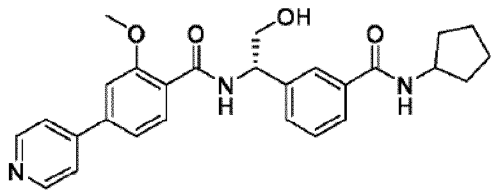,

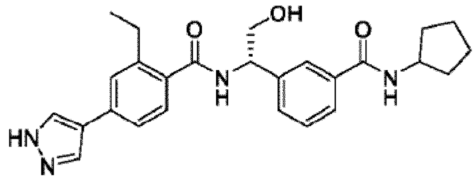,

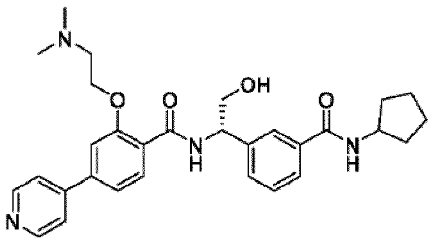,

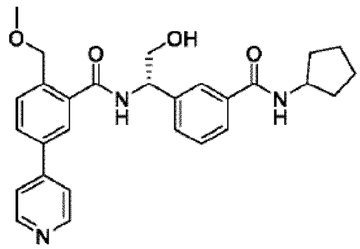,

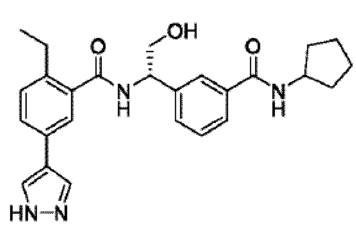,

und

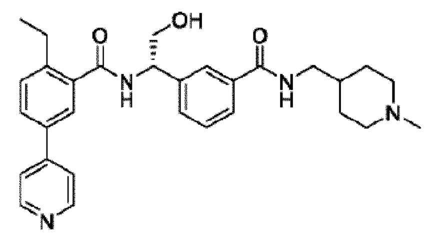.

**12.** Verbindung nach Anspruch 11, ausgewählt aus der Gruppe bestehend aus:

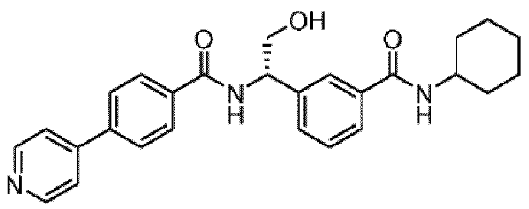

(26),

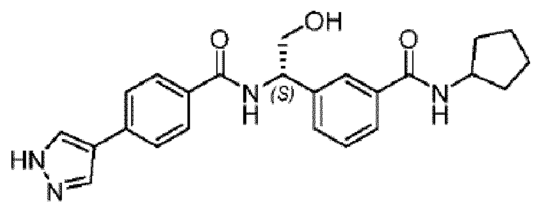

(46),

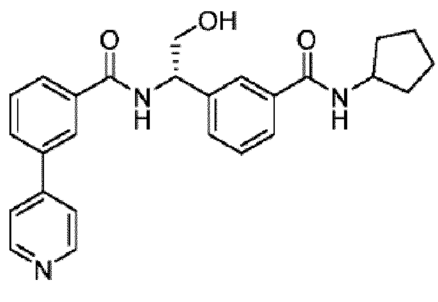

(63)und

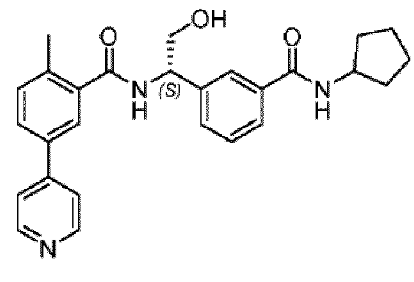

(64).

**13.** Verbindung nach Anspruch 11, wobei die Verbindung aus

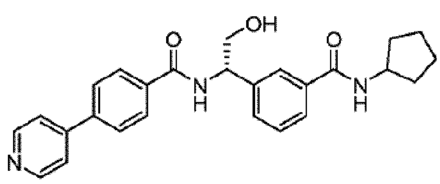

(10)

besteht.

**14.** Pharmazeutische Zusammensetzung, umfassend eine Verbindung nach einem der Ansprüche 1 bis 13 und einen pharmazeutisch verträglichen Träger.

**15.** Verbindung nach einem der Ansprüche 1 bis 13 oder pharmazeutische Zusammensetzung nach Anspruch 14 zur Verwendung bei der Vorbeugung oder Behandlung einer mit Rhoassoziierter Proteinkinase-Modulation assoziierten Erkrankung bei einem Subjekt, wobei

die Erkrankung ausgewählt ist aus der Gruppe bestehend aus: Krebs, einer neurodegenerativen Erkrankung, einer neurologischen Entwicklungsstörung, einer entzündlichen oder Autoimmunerkrankung, einer Infektion, einer Stoffwechselerkrankung, einer kardiovaskulären Erkrankung oder einer Dysfunktion der vaskulären glatten Muskulatur, einer hämatologischen Erkrankung, einer hämatologischen malignen neoplastischen Störung und einer Augenstörung; wobei
Krebs ausgewählt ist aus der Gruppe bestehend aus: kutanem T-Zell-Lymphom (CTCL), peripherem T-Zell-Lymphom (PTCL), angioimmunoblastischem T-Zell-Lymphom (AITL), multiplem Myelom, Leukämie, Lymphom, Lungenkrebs, Eierstockkrebs, Brustkrebs, Magenkrebs, Prostatakrebs, Bauchspeicheldrüsenkrebs, hepatozellulärem Krebs und Nierenkrebs;
die neurodegenerative Erkrankung ausgewählt ist aus der Gruppe bestehend aus: Morbus Alzheimer, Morbus Huntington, Morbus Parkinson, amyotropher Lateralsklerose und spinaler Muskelatrophie;
die neurologische Entwicklungsstörung das Rett-Syndrom oder Morbus Niemann-Pick Typ C ist;
die entzündliche oder Autoimmunerkrankung ausgewählt ist aus der Gruppe bestehend aus: Asthma, kardiovaskulärer Entzündung, Nierenentzündung, Arteriosklerose, rheumatoider Arthritis, Spondylitis-Arthritis, Psoriasis-Arthritis, Psoriasis, atopischer Dermatitis, Ekzem, multipler Sklerose, systemischem Lupus erythematodes, entzündlicher Darmerkrankungen, Morbus Crohn, Graft-versus-Host-Erkrankung, Transplantatabstoßung und fibrotischer Erkrankung;
die kardiovaskuläre Erkrankung oder Dysfunktion der vaskulären glatten Muskulatur ausgewählt ist aus der Gruppe bestehend aus: Bluthochdruck, Atherosklerose, Restenose, Herzhypertrophie, okulärer Hypertonie, cerebraler Ischämie, cerebralem Vasospasmus und erektiler Dysfunktion;
die hämatologische maligne neoplastische Störung durch deregulierte FLT3-Rezeptor-Tyrosinkinase-Aktivität gekennzeichnet ist und ausgewählt ist aus der Gruppe bestehend aus: Leukämie, Myelom, myeloproliferativer Erkrankung, myelodysplastischem Syndrom, Morbus Hodgkin, Myelom, akuter lymphatischer Leukämie (ALL), akuter myeloischer Leukämie (AML), akuter Promyelozytenleukämie (APL), chronischer lymphatischer Leukämie (CLL), chronischer myeloischer Leukämie (CML), chronischer neutrophiler Leukämie (CNL), akuter undifferenzierter Leukämie (AUL), anaplastischem großzelligem Lymphom (ALCL), prolymphozytärer Leukämie (PML), juveniler myelomonozytischer Leukämie (JMML), adulter T-Zell-ALL, AML mit Trilineage-Myelodysplasie (AMLITMDS), Mixed-Lineage-Leukämie (MLL), myelodysplastischen Syndromen (MDSs), myeloproliferativen Erkrankungen (MPD) und multiplem Myelom (MM); und
die Augenerkrankung ausgewählt ist aus der Gruppe bestehend aus: altersbedingter Makuladegeneration (AMD), choroidaler Neovaskularisation (CNV), diabetischem Makulaödem (DME), Irisneovaskularisation, Uveitis, neovaskulärem Glaukom und Frühgeborenenretinitis (ROP).

## Revendications

**1.** Composé de formule (I) :

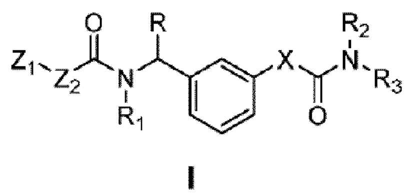

I

dans laquelle :

$Z_1$ représente un noyau pyridine ou pyrazole, ledit noyau pyridine ou pyrazole étant éventuellement substitué

par un groupe halogéno, -OH, -CN, -COOR', -OR', -SR', -OC(O)R', - NHR', -NR'R", -NHC(O)R', -NHC(O)NR'R", -C(O)NR'R", -NS(O)$_2$R', -S(O)$_2$NR'R", - S(O)$_2$R', guanidino, nitro, nitroso, C1-C6 alkyle, aryle, C3-C7 cycloalkyle, ou un noyau hétérocycle de 3 à 10 chaînons, ledit groupe C1-C6 alkyle, aryle, C3-C7 cycloalkyle, ou noyau hétérocycle de 3 à 10 chaînons étant non substitué ou substitué par un ou plusieurs des groupes suivants : un groupe halogéno, -OH, -CN, -COOR', -OR', -SR', -OC(O)R', -NHR', -NR'R", - NHC(O)R', -NHC(O)NR'R", -C(O)NR'R", -NS(O)$_2$R', -S(O)$_2$NR'R", -S(O)$_2$R', guanidino, nitro, nitroso, C1-C6 alkyle, aryle ou C3-C7 cycloalkyle ;

$Z_2$ représente un noyau phényle, naphtyle ou hétérocycle à chaînons en C5 à C10, ledit noyau phényle, naphtyle ou hétérocycle à chaînons en C5 à C10 étant éventuellement substitué par un groupe halogéno, -OH, -CN, -COOR', -OR', -SR', -OC(O)R', -NHR', -NR'R", - NHC(O)R', -NHC(O)NR'R", -C(O)NR'R", -NS(O)$_2$R', -S(O)$_2$NR'R", -S(O)$_2$R', guanidino, nitro, nitroso, C1-C6 alkyle, aryle, C3-C7 cycloalkyle ou un noyau hétérocycle de 3 à 10 chaînons, ledit groupe C1-C6 alkyle, aryle, C3-C7 cycloalkyle, ou noyau hétérocycle de 3 à 10 chaînons étant non substitué ou substitué par un ou plusieurs des groupes suivants : un groupe halogéno, -OH, - CN, -COOR', -OR', -SR', -OC(O)R', -NHR', -NR'R", -NHC(O)R', -NHC(O)NR'R", - C(O)NR'R", -NS(O)$_2$R', -S(O)$_2$NR'R", -S(O)$_2$R', guanidino, nitro, nitroso, C1-C6 alkyle, aryle ou C3-C7 cycloalkyle ;

R représente un groupe -C1-C6 alkyle, éventuellement substitué par un ou plusieurs groupes -OH ;

$R_1$ représente un atome H, un groupe C1-C6 alkyle ou C3-C7 cycloalkyle, ledit groupe C1-C6 alkyle ou C3-C7-cycloalkyle étant éventuellement substitué par un ou plusieurs des groupes suivants : un groupe halogéno, -OH, -CN, -COOR', -OR', -SR', -OC(O)R', -NHR', -NR'R", - NHC(O)R', -NHC(O)NR'R", -C(O)NR'R", -NS(O)$_2$R', - S(O)$_2$NR'R" ou -S(O)$_2$R' ;

X représente une liaison ou un groupe -O(C1-C6 alkyle) ; et

$R_2$ et $R_3$ représentent indépendamment un atome H, un groupe C1-C6 alkyle ou C3-C7 cycloalkyle, aryle, ou un noyau hétérocycle à chaînons en C5 à C10,

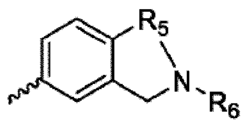
,
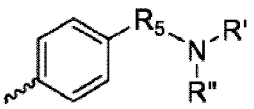
,
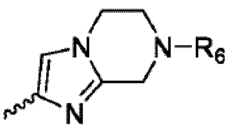
,

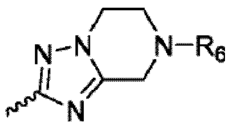
,

ou

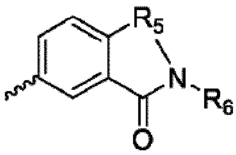
,

ledit groupe C1-C6 alkyle, C3-C7 cycloalkyle, aryle ou noyau hétérocycle à chaînons en C5 à C10 étant éventuellement substitué par un groupe halogéno, -OH, -CN, -COOR', -OR', -SR', -OC(O)R', -NHR', -NR'R", -CNR'R", -NHC(O)R', - NHC(O)NR'R", -C(O)NR'R", -NS(O)$_2$R', -S(O)$_2$NR'R", -S(O)$_2$R', guanidino, nitro, nitroso, C1-C6 alkyle, aryle, C3-C7 cycloalkyle or un noyau hétérocycle de 3 à 10 chaînons, ledit groupe C1-C6 alkyle, aryle, C3-C7 cycloalkyle, ou noyau hétérocycle de 3 à 10 chaînons étant non substitué ou substitué par un ou plusieurs des groupes suivants : un groupe halogéno, -OH, -CN, -COOR', -OR', -SR', -OC(O)R', -NHR', -NR'R", -NHC(O)R', -NHC(O)NR'R", -C(O)NR'R", -NS(O)$_2$R', -S(O)$_2$NR'R", -S(O)$_2$R', guanidino, nitro, nitroso, C1-C6 alkyle, aryle ou C3-C7 cycloalkyle ;

$R_5$ représentant un groupe C1-C6 alkyle, $-OCH_2CH_2-$, $-NR_6CH_2CH_2-$ ou - $NC(O)CH_2CH_2-$ ; et

$R_6$ représentant un atome H, un groupe C1-C6 alkyle ou C3-C7 cycloalkyle ; et

R' et R" représentant indépendamment un atome H ou un groupe C1-C6 alkyle ; et

R' et R" conjointement, éventuellement en se liant à un atome N ou O, pouvant former une structure cyclique de 4 à 8 chaînons.

**2.** Composé selon la revendication 1, R représentant un groupe $-CH_3$ ou $-CH_3OH$.

**3.** Composé selon la revendication 1, présentant une structure de formule (III) :

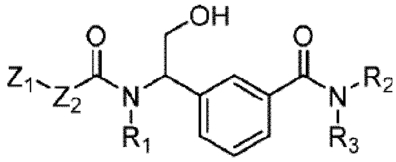

**III**

,

dans laquelle :

$Z_1$ représente un noyau pyridine ou pyrazole, ledit noyau pyridine ou pyrazole étant éventuellement substitué par un groupe halogéno, -OH, -CN, -COOR', -OR', -SR', -OC(O)R', - NHR', -NR'R", -NHC(O)R', -NHC(O)NR'R", -C(O)NR'R", $-NS(O)_2R'$, $-S(O)_2NR'R"$, - $S(O)_2R'$, guanidino, nitro, nitroso, C1-C6 alkyle, aryle, C3-C7 cycloalkyle, ou un noyau hétérocycle de 3 à 10 chaînons, ledit groupe C1-C6 alkyle, aryle, C3-C7 cycloalkyle, ou noyau hétérocycle de 3 à 10 chaînons étant non substitué ou substitué par un ou plusieurs des groupes suivants : un groupe halogéno, -OH, -CN, -COOR', -OR', -SR', -OC(O)R', -NHR', -NR'R", - NHC(O)R', -NHC(O)NR'R", -C(O)NR'R", $-NS(O)_2R'$, $-S(O)_2NR'R"$, $-S(O)_2R'$, guanidino, nitro, nitroso, C1-C6 alkyle, aryle ou C3-C7 cycloalkyle ;

$Z_2$ représente un noyau phényle, naphtyle ou hétérocycle à chaînons en C5 à C10, ledit noyau phényle, naphtyle ou hétérocycle à chaînons en C5 à C10 étant éventuellement substitué par un groupe halogéno, -OH, -CN, -COOR', -OR', -SR', -OC(O)R', -NHR', -NR'R", - NHC(O)R', -NHC(O)NR'R", -C(O)NR'R", $-NS(O)_2R'$, $-S(O)_2NR'R"$, $-S(O)_2R'$, guanidino, nitro, nitroso, C1-C6 alkyle, aryle, C3-C7 cycloalkyle ou un noyau hétérocycle de 3 à 10 chaînons, ledit groupe C1-C6 alkyle, aryle, C3-C7 cycloalkyle, ou noyau hétérocycle de 3 à 10 chaînons étant non substitué ou substitué par un ou plusieurs des groupes suivants : un groupe halogéno, -OH, - CN, -COOR', -OR', -SR', -OC(O)R', -NHR', -NR'R", -NHC(O)R', -NHC(O)NR'R", - C(O)NR'R", $-NS(O)_2R'$, $-S(O)_2NR'R"$, $-S(O)_2R'$, guanidino, nitro, nitroso, C1-C6 alkyle, aryle ou C3-C7 cycloalkyle ;

$R_1$ représente un atome H, un groupe C1-C6 alkyle ou C3-C7 cycloalkyle, ledit groupe C1-C6 alkyle ou C3-C7-cycloalkyle étant éventuellement substitué par un ou plusieurs des groupes suivants : un groupe halogéno, -OH, -CN, -COOR', -OR', -SR', -OC(O)R', -NHR', -NR'R", - NHC(O)R', -NHC(O)NR'R", -C(O)NR'R", $-NS(O)_2R'$, $-S(O)_2NR'R"$ ou $-S(O)_2R'$ ; et

$R_2$ et $R_3$ représentent indépendamment un atome H, un groupe C1-C6 alkyle, C3-C7 cycloalkyle, aryle, ou un noyau hétérocycle à chaînons en C5 à C10,

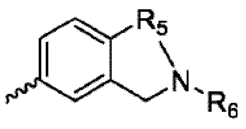 , 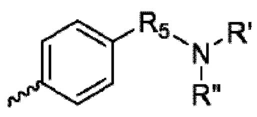 , 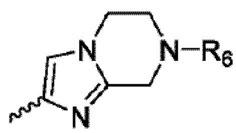 , 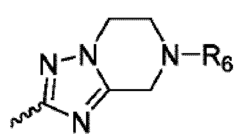 ,

ou

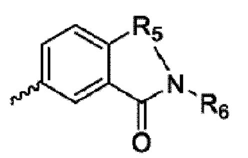

,

ledit groupe $C_1$-$C_6$ alkyle, $C_3$-$C_7$ cycloalkyle, aryle ou noyau hétérocycle à chaînons en C5 à C10 étant éventuellement substitué par un groupe halogéno, -OH, -CN, -COOR', -OR', -SR', -OC(O)R', -NHR', -NR'R", -CNR'R", -NHC(O)R', -NHC(O)NR'R", -C(O)NR'R", $-NS(O)_2R'$, $-S(O)_2NR'R"$, $-S(O)_2R'$, guanidino, nitro, nitroso, C1-C6 alkyle, aryle, C3-C7 cycloalkyle ou un noyau hétérocycle de 3 à 10 chaînons, ledit groupe C1-C6 alkyle, aryle, C3-C7 cycloalkyle, ou noyau hétérocycle de 3 à 10 chaînons étant non substitué ou substitué par un ou plusieurs

des groupes suivants : un groupe halogéno, -OH, -CN, -COOR', -OR', -SR', -OC(O)R', -NHR', -NR'R", -NHC(O)R', -NHC(O)NR'R", -C(O)NR'R", -NS(O)$_2$R', -S(O)$_2$NR'R", - S(O)$_2$R', guanidino, nitro, nitroso, C1-C6 alkyle, aryle ou C3-C7 cycloalkyle ;

R$_5$ représentant un groupe C1-C6 alkyle, -OCH$_2$CH$_2$-, -NR$_6$CH$_2$CH$_2$- ou - NC(O)CH$_2$CH$_2$- ; et R$_6$ représentant un atome H, un groupe C1-C6 alkyle ou C3-C7 cycloalkyle ; et

R' et R" représentant indépendamment un atome H ou un groupe C1-C6 alkyle ; ou R' et R" conjointement, éventuellement en se liant à un atome N ou O, pouvant former une structure cyclique de 4 à 8 chaînons.

**4.** Composé selon la revendication 3, présentant une structure de formule (IV) :

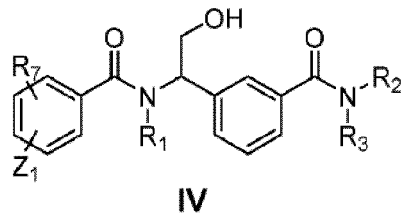

IV

dans laquelle :

Z$_1$ représente un noyau pyridine ou pyrazole, ledit noyau pyridine ou pyrazole étant éventuellement substitué par un groupe halogéno, -OH, -CN, -COOR', -OR', -SR', -OC(O)R', - NHR', -NR'R", -NHC(O)R', -NHC(O)NR'R", -C(O)NR'R", -NS(O)$_2$R', -S(O)$_2$NR'R", - S(O)$_2$R', guanidino, nitro, nitroso, C1-C6 alkyle, aryle, C3-C7 cycloalkyle, ou un noyau hétérocycle de 3 à 10 chaînons, ledit groupe C1-C6 alkyle, aryle, C3-C7 cycloalkyle, ou noyau hétérocycle de 3 à 10 chaînons étant non substitué ou substitué par un ou plusieurs des groupes suivants : un groupe halogéno, -OH, -CN, -COOR', -OR', -SR', -OC(O)R', -NHR', -NR'R", - NHC(O)R', -NHC(O)NR'R", -C(O)NR'R", -NS(O)$_2$R', -S(O)$_2$NR'R", -S(O)$_2$R', guanidino, nitro, nitroso, C1-C6 alkyle, aryle ou $C_3$-$C_7$ cycloalkyle ;

R$_1$ représente un atome H, un groupe C1-C6 alkyle ou C3-C7 cycloalkyle, ledit groupe C1-C6 alkyle ou C3-C7-cycloalkyle étant éventuellement substitué par un ou plusieurs des groupes suivants : un groupe halogéno, -OH, -CN, -COOR', -OR', -SR', -OC(O)R', -NHR', -NR'R", - NHC(O)R', -NHC(O)NR'R", -C(O)NR'R", -NS(O)$_2$R', -S(O)$_2$NR'R" ou -S(O)$_2$R' ; et

R$_2$ et R$_3$ représentent indépendamment un atome H, un groupe C1-C6 alkyle, C3-C7 cycloalkyle, aryle, ou un noyau hétérocycle à chaînons en C5à C10,

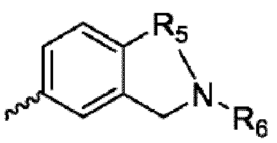,
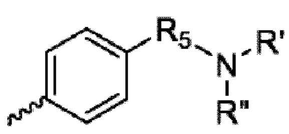,
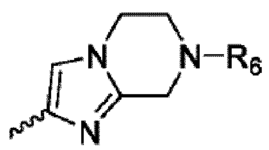,
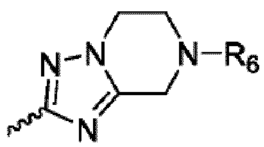,

ou

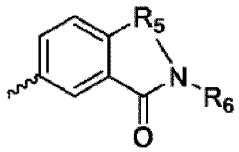,

ledit groupe $C_1$-$C_6$ alkyle, $C_3$-$C_7$ cycloalkyle, aryle ou noyau hétérocycle à chaînons en C5 à C10 étant éventuellement substitué par un groupe halogéno, -OH, -CN, - COOR', -OR', -SR', -OC(O)R', -NHR', -NR'R", -CNR'R", -NHC(O)R', -NHC(O)NR'R", - C(O)NR'R", -NS(O)$_2$R', -S(O)$_2$NR'R", -S(O)$_2$R', guanidino, nitro, nitroso, C1-C6 alkyle, aryle, C3-C7 cycloalkyle or un noyau hétérocycle de 3 à 10 chaînons, ledit groupe C1-C6 alkyle, aryle, C3-C7 cycloalkyle, ou noyau hétérocycle de 3 à 10 chaînons étant non substitué ou substitué par un ou plusieurs des groupes suivants : un groupe halogéno, -OH, -CN, -COOR', -OR', -SR', - OC(O)R', -NHR', -NR'R", -NHC(O)R', -NHC(O)NR'R", -C(O)NR'R", -NS(O)$_2$R', - S(O)$_2$NR'R", -S(O)$_2$R', guanidino, nitro, nitroso, C1-C6

alkyle, aryle ou C3-C7 cycloalkyle ;
$R_5$ représentant un groupe C1-C6 alkyle, $-OCH_2CH_2-$, $-NR_6CH_2CH_2-$ ou - $NC(O)CH_2CH_2-$ ; et $R_6$ représentant un atome H, un groupe C1-C6 alkyle ou C3-C7 cycloalkyle ;
R' et R" représentant indépendamment un atome H ou un groupe C1-C6 alkyle ; ou R' et R" conjointement, éventuellement en se liant à un atome N ou O, pouvant former une structure cyclique de 4 à 8 chaînons ; et
$R_7$ représentant un atome H, un groupe halogéno, -OH, -CN, -COOR', -OR', -SR', - OC(O)R', -NHR', -NR'R", -NHC(O)R', -NHC(O)NR'R", -C(O)NR'R", $-NS(O)_2R'$, - $S(O)_2NR'R"$, $-S(O)_2R'$, C1-C6 alkyle, C3-C7 cycloalkyle, aryle, ou un noyau hétérocycle à chaînons en C5 à C10, ledit groupe C1-C6 alkyle, C3-C7 cycloalkyle, aryle, ou noyau hétérocycle à chaînons en C5-C10 étant éventuellement substitué par un groupe halogéno, -OH, -CN, -COOR', -OR', -SR', -OC(O)R', -NHR', -NR'R", -NHC(O)R', -NHC(O)NR'R", -C(O)NR'R", $-NS(O)_2R'$, $-S(O)_2NR'R"$, $-S(O)_2R'$, guanidino, nitro, nitroso, C1-C6 alkyle, aryle, C3-C7 cycloalkyle ou un noyau hétérocycle de 3 à 10 chaînons.

**5.** Composé selon l'une quelconque des revendications 1 à 4, $R_1$ représentant un atome H.

**6.** Composé selon l'une quelconque des revendications 1 à 5, $R_2$ représentant un atome H.

**7.** Composé selon l'une quelconque des revendications 1 à 6, R représentant un groupe méthyle avec une configuration R ou hydroxyméthyle avec une configuration S.

**8.** Composé selon l'une quelconque des revendications 1 à 7, $R_3$ représentant un atome H, un groupe C3-C7 cycloalkyle ou C3-C7 cycloalkylméthyle, et ledit groupe C3-C7 cycloalkyle ou C3-C7 cycloalkylméthyle étant éventuellement substitué par un groupe halogéno, -OH, -CN, -COOR', -OR', -SR', -OC(O)R', -NHR', -NR'R", -CNR'R", -NHC(O)R', -NHC(O)NR'R", -C(O)NR'R", $-NS(O)_2R'$, $-S(O)_2NR'R"$, $-S(O)_2R'$, guanidino, nitro, nitroso, C1-C6 alkyle, aryle, C3-C7 cycloalkyle ou un noyau hétérocycle de 3 à 10 chaînons.

**9.** Composé selon l'une quelconque des revendications 1 à 7, $R_3$ représentant un atome H, un groupe cyclohexyle, cyclopentyle, cyclobutyle, cyclopropyle, cyclohexylméthyle, cyclopentylméthyle, cyclobutylméthyle, cyclopropylméthyle, phényle ou benzyle, ledit groupe cyclohexyle, cyclopentyle, cyclobutyle, cyclopropyle, cyclohexylméthyle, cyclopentylméthyle, cyclobutylméthyle, cyclopropylméthyle, phényle ou benzyle étant éventuellement substitué par un groupe halogéno, -OH, -CN, -COOR', -OR', -SR', -OC(O)R', -NHR', -NR'R", -CNR'R", - NHC(O)R', -NHC(O)NR'R", -C(O)NR'R", $-NS(O)_2R'$, $-S(O)_2NR'R"$, $-S(O)_2R'$, guanidino, nitro, nitroso, C1-C6 alkyle, aryle, C3-C7 cycloalkyle ou un noyau hétérocycle 3 à 10 chaînons.

**10.** Composé selon l'une quelconque des revendications 1, 2 et 5 à 9, $Z_2$ représentant un noyau pyridine ou pyrazole.

**11.** Composé selon la revendication 1, choisi dans le groupe constitué par :

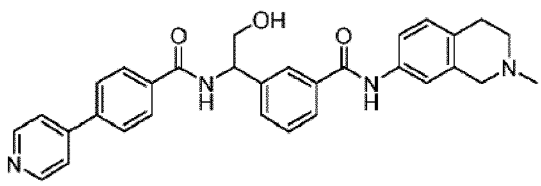 (2), 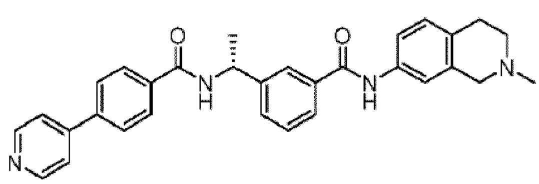 (6),

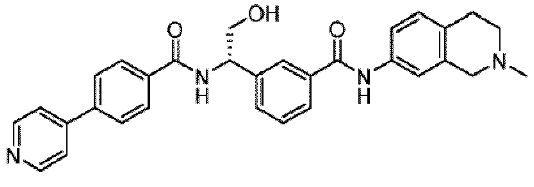 (7), 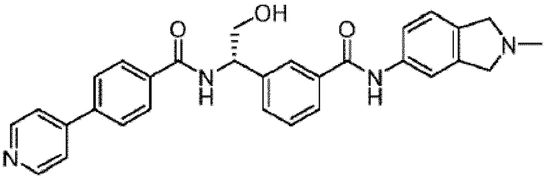 (8),

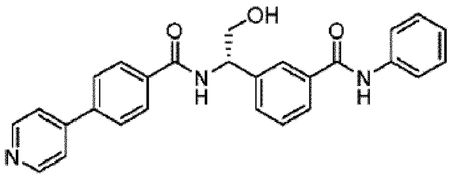 (9), 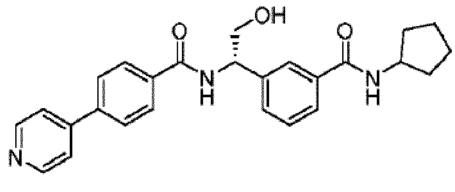 (10),

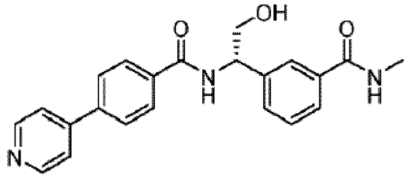 (11), 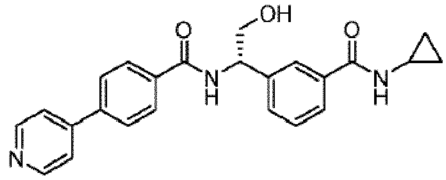 (12),

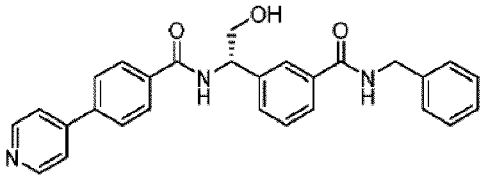 (13), 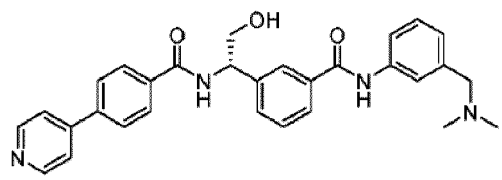 (14),

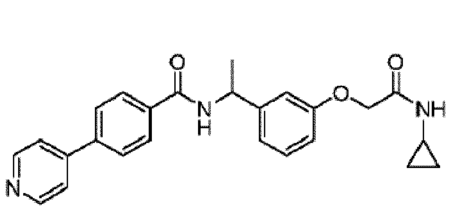 (15), 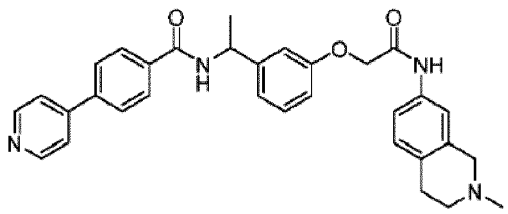 (16),

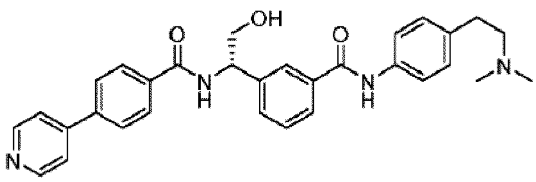 (20), 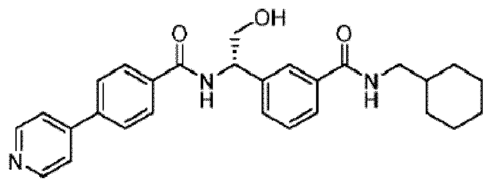 (21),

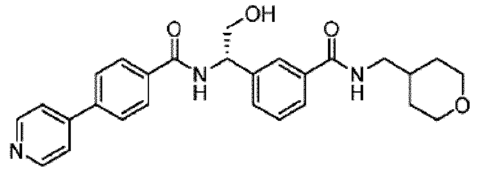 (22), 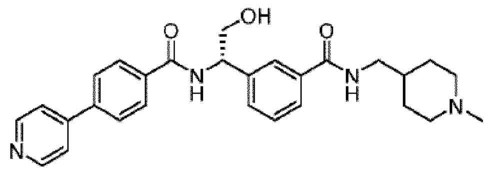 (23),

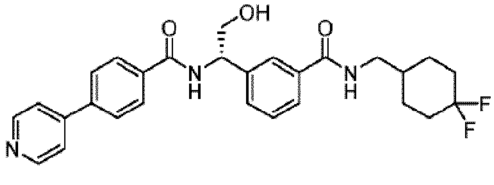 (24), 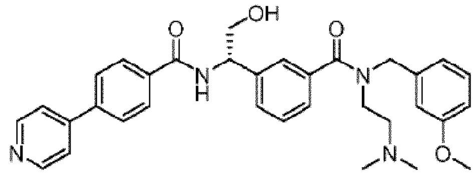 (25),

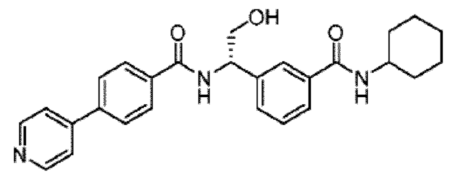 (26), 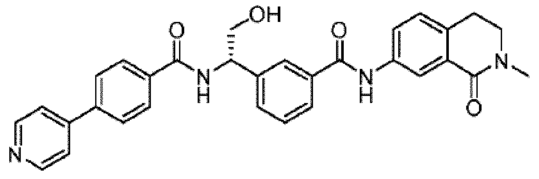 (27),

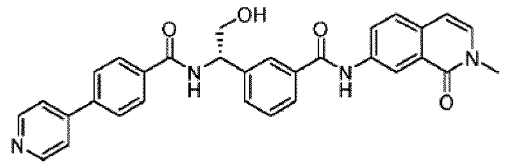 (28), 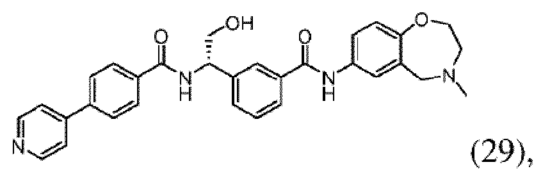 (29),

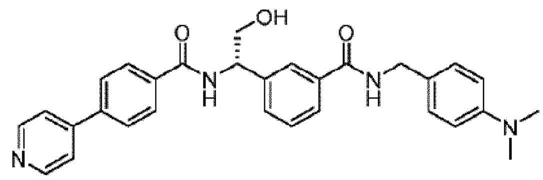 (30), 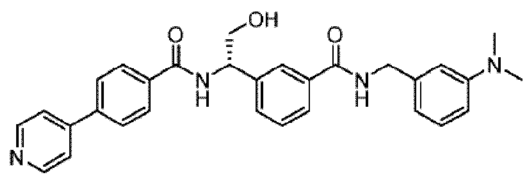 (31),

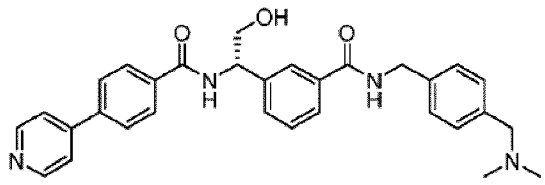 (32), 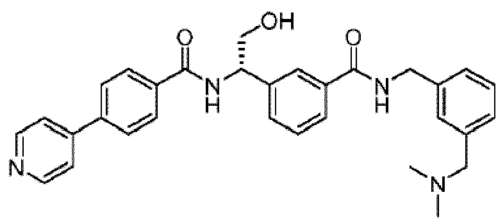 (33),

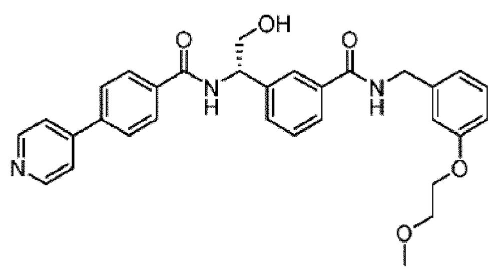 (34), 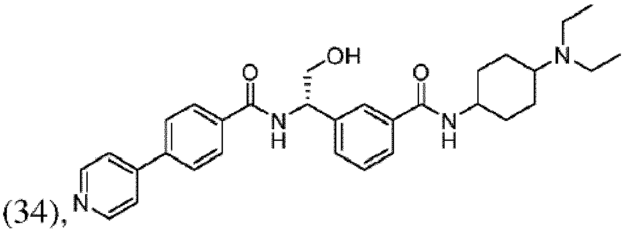 (35),

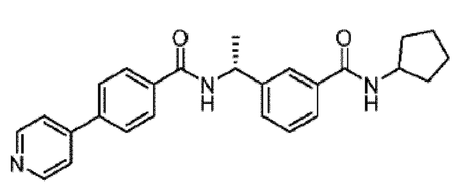 (36), 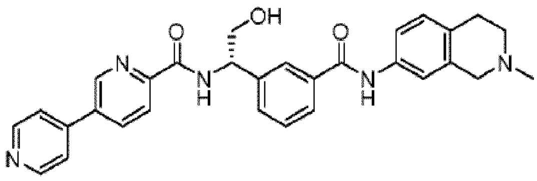 (37),

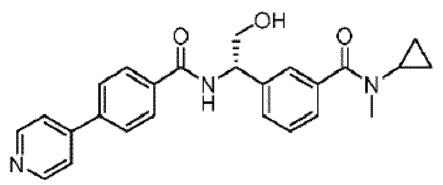 (38), 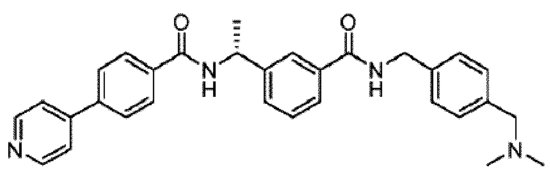 (39),

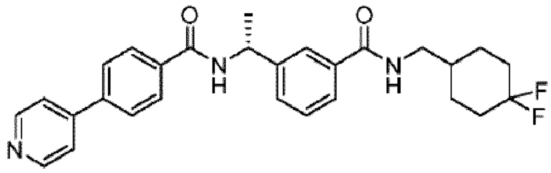 (40), 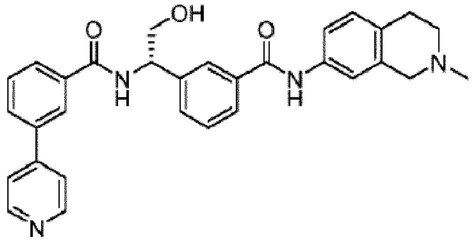 (41),

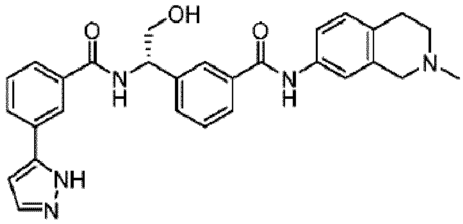 (42), 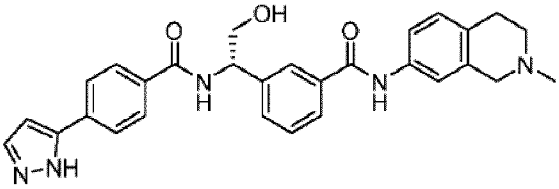 (43),

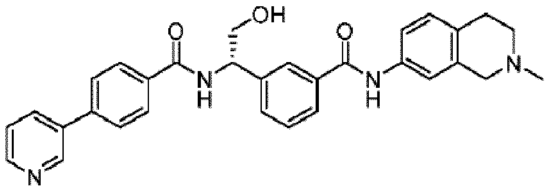 (44), 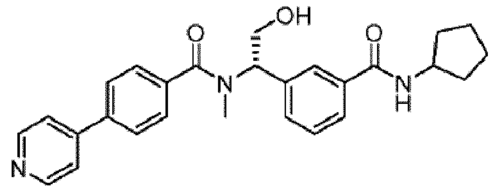 (45),

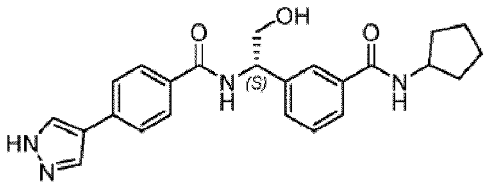 (46), 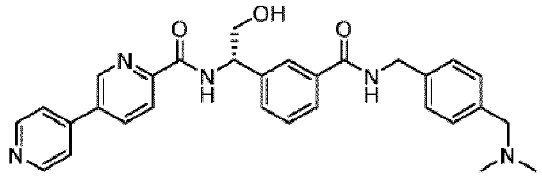 (47),

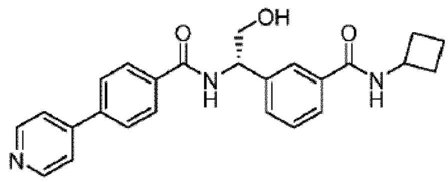 (48), 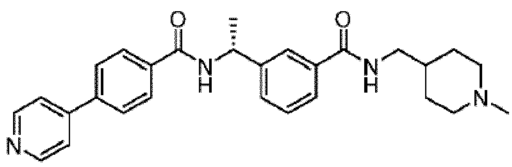 (49),

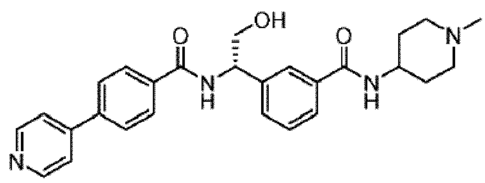 (50), 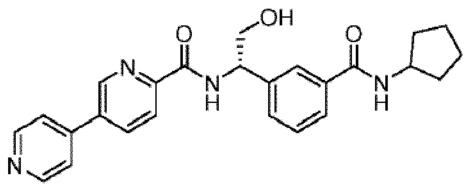 (51),

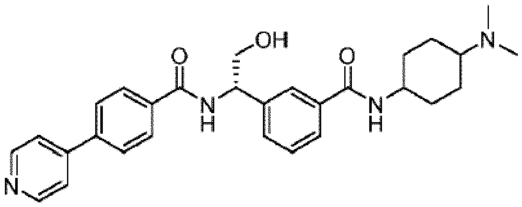 (52), 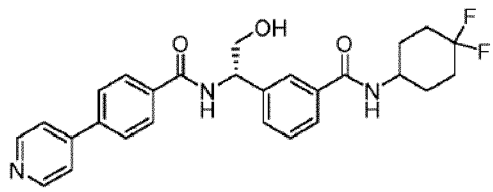 (53),

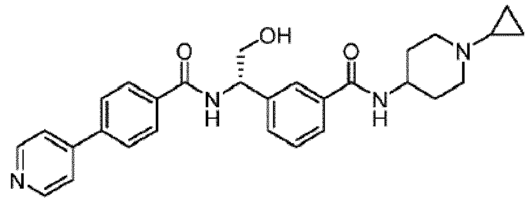 (54), 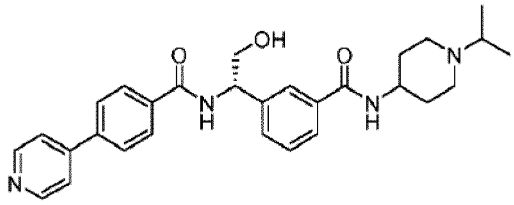 (55),

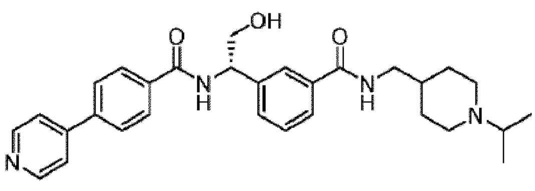 (56), 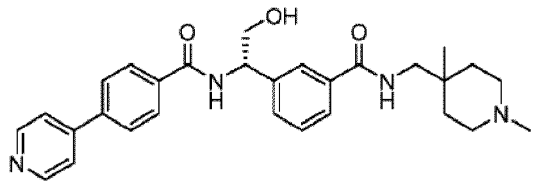 (57),

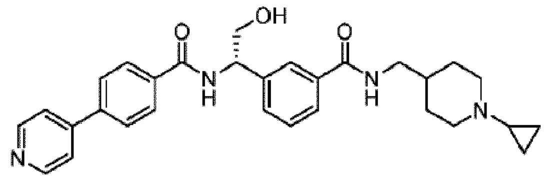 (58), 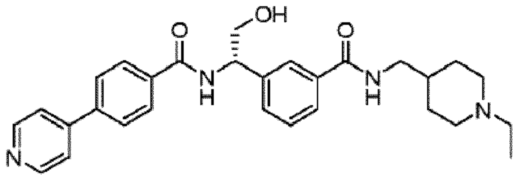 (59),

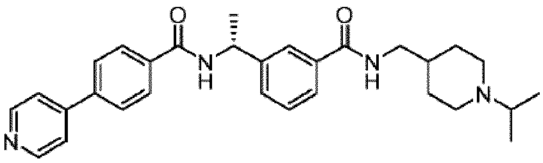 (60), 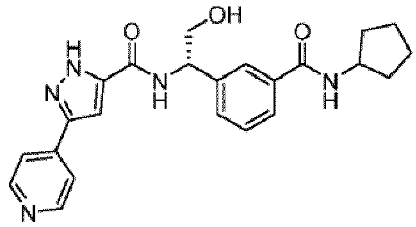 (61),

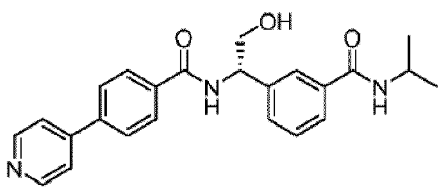 (62), 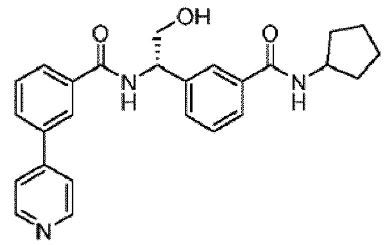 (63),

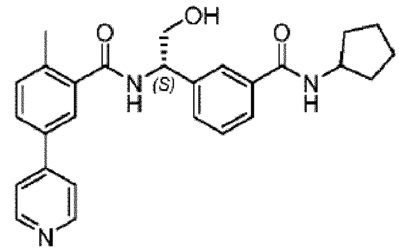 (64), 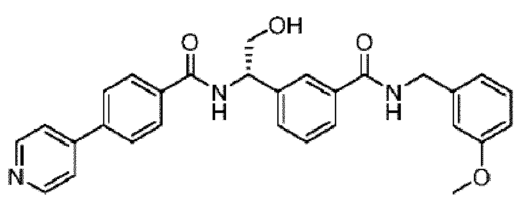 (65),

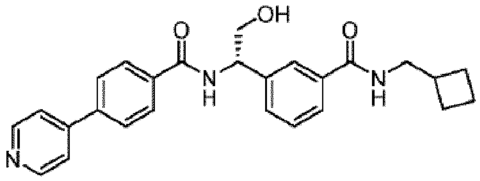 (66), 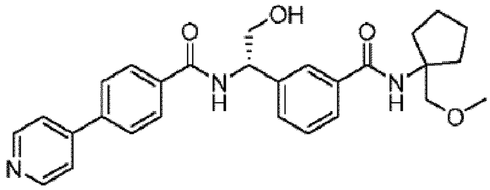 (67),

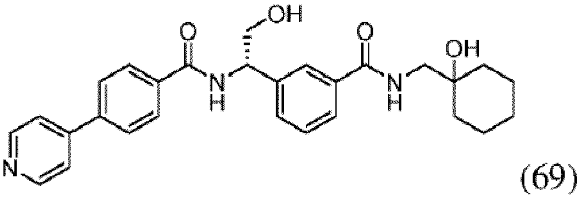 (69), 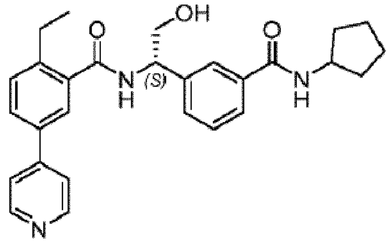 (70),

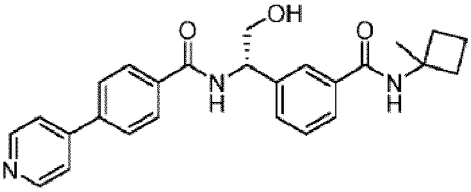 (71), 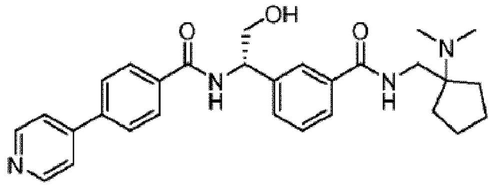 (72),

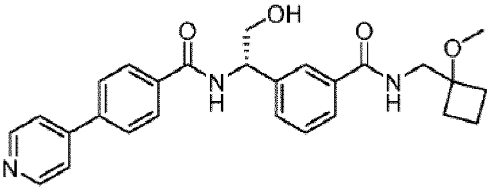 (73), 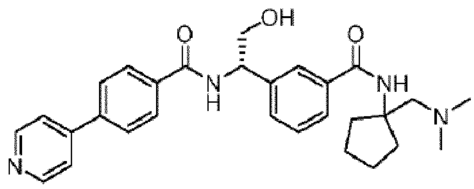 (74),

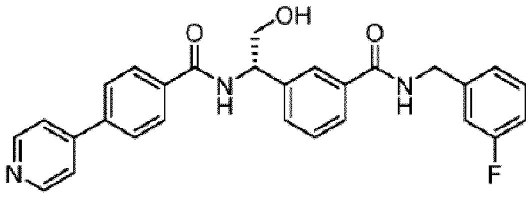 (75), 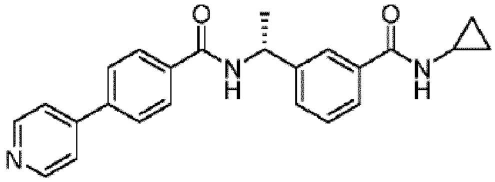 (76),

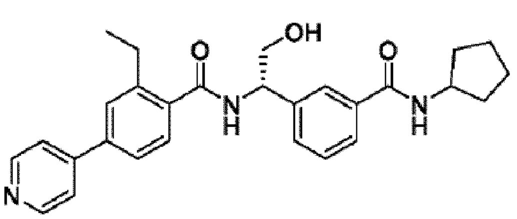 , 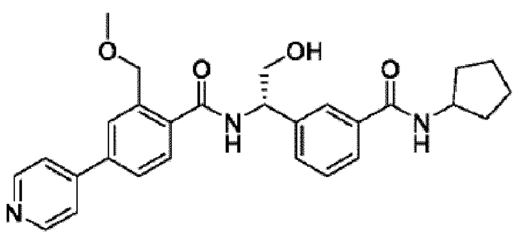 ,

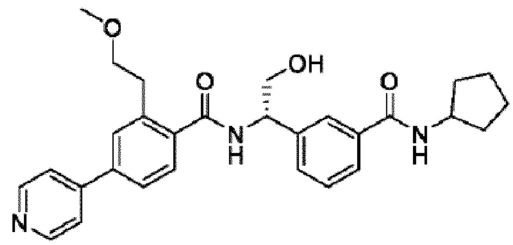

,

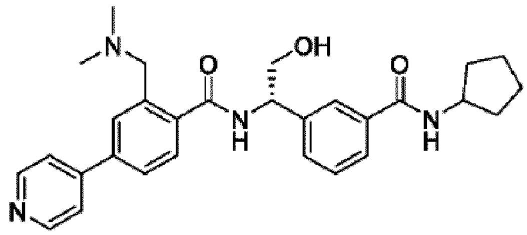

,

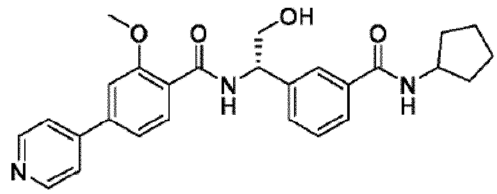

,

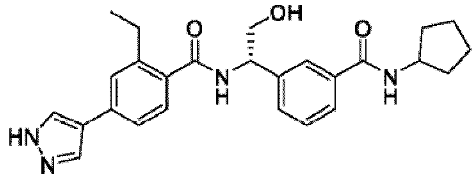

,

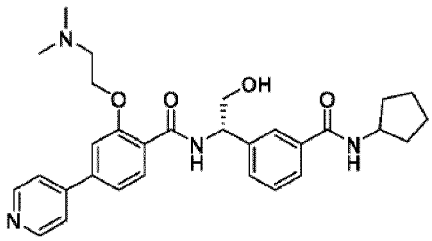

,

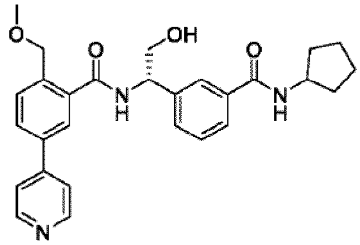

,

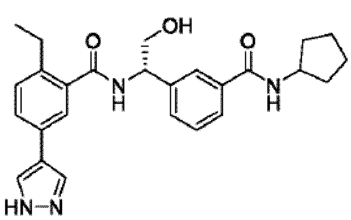

,

et

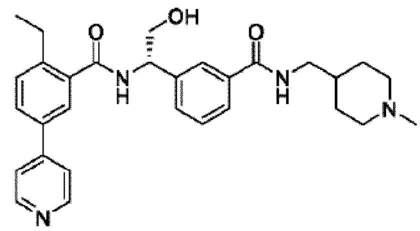

.

**12.** Composé selon la revendication 11, choisi dans le groupe constitué par :

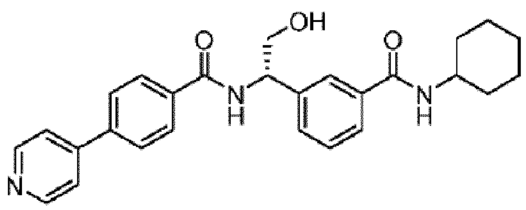

(26),

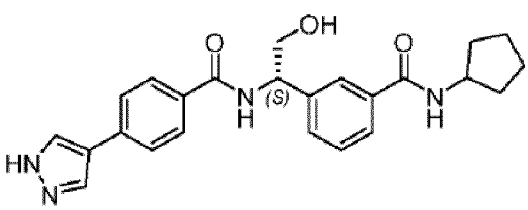

(46),

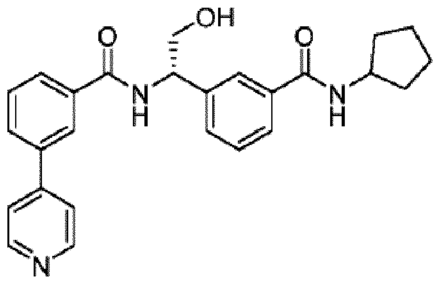 (63), et 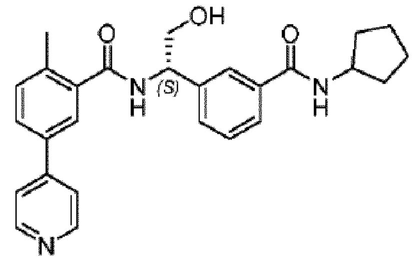 (64).

13. Composé selon la revendication 11, ledit composé consistant en

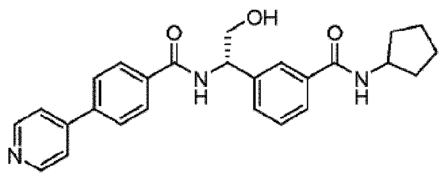 (10).

14. Composition pharmaceutique comprenant un composé selon l'une quelconque des revendications 1 à 13 et un vecteur pharmaceutiquement acceptable.

15. Composé selon l'une quelconque des revendications 1 à 13, ou composition pharmaceutique selon la revendication 14, destiné à être utilisé dans la prévention ou le traitement d'une maladie associée à la modulation des protéines kinase associées à rho chez un sujet,

ladite maladie étant choisie dans le groupe constitué par : un cancer, une maladie neurodégénérative, un trouble du développement neurologique, une maladie inflammatoire ou auto-immune, une infection, une maladie métabolique, une maladie cardiovasculaire ou un dysfonctionnement vasculaire des muscles lisses, une maladie hématologique, un trouble hématologique néoplasique malin et un trouble oculaire ;
ledit cancer étant choisi dans le groupe constitué par : un lymphome cutané à lymphocytes T (CTCL), un lymphome à lymphocytes T périphérique (PTCL), un lymphome à lymphocytes T angio-immunoblastique (AITL), un myélome multiple, une leucémie, un lymphome, le cancer du poumon, le cancer de l'ovaire, le cancer du sein, le cancer de l'estomac, le cancer de la prostate, le cancer du pancréas, le cancer hépatocellulaire et le cancer du rein ;
ladite maladie neurodégénérative étant choisie dans le groupe constitué par : la maladie d'Alzheimer, la maladie de Huntington, la maladie de Parkinson, la sclérose latérale amyotrophique et une atrophie musculaire spinale ;
ledit trouble du développement neurologique étant le syndrome de Rett ou la maladie de Niemann-Pick de type C ;
ladite maladie inflammatoire ou auto-immune étant choisie dans le groupe constitué par : l'asthme, une inflammation cardiovasculaire, une inflammation rénale, l'artériosclérose, la polyarthrite rhumatoïde, la spondylarthrite, la polyarthrite psoriasique, le psoriasis, une dermatite atopique, l'eczéma, la sclérose en plaques, le lupus érythémateux disséminé, les maladies inflammatoires de l'intestin, la maladie de Crohn, la maladie du greffon contre l'hôte, le rejet de greffe et une maladie fibrotique ;
ladite maladie cardiovasculaire ou ledit dysfonctionnement vasculaire des muscles lisses étant choisi dans le groupe comprenant : l'hypertension, l'athérosclérose, une resténose, une hypertrophie cardiaque, l'hypertension oculaire, une ischémie cérébrale, un vasospasme cérébral et un dysfonctionnement érectile ;
ledit trouble hématologique néoplasique malin étant **caractérisé par** une activité tyrosine kinase dérégulée du récepteur FLT3 et étant choisi dans le groupe constitué par : une leucémie, un myélome, une maladie myéloproliférative, le syndrome myélodysplasique, la maladie de Hodgkin, un myélome, la leucémie lymphocytaire aiguë (LLA), la leucémie myéloïde aiguë (LMA), la leucémie promyélocytaire aiguë (LPA), la leucémie lymphocytaire chronique (LLC), la leucémie myéloïde chronique (LMC), la leucémie neutrophile chronique (LNC), la leucémie aiguë indifférenciée (LAI), un lymphome anaplasique à grandes cellules (ALCL), la leucémie prolymphocytaire (PML), la leucémie myélomonocytaire juvénile (JMML), la LLA à lymphocytes T de l'adulte, la LMA, avec myélodysplasie trilignée (AMLITMDS), la leucémie à lignée mixte (MLL), les syndromes myélodysplasiques (MDS), les troubles myéloprolifératifs (MPD) et un myélome multiple (MM) ; et
ledit trouble oculaire étant choisi dans le groupe constitué par : la dégénérescence maculaire liée à l'âge (DMLA),

la néovascularisation choroïdienne (CNV), l'oedème maculaire diabétique (OMD), la néovascularisation de l'iris, une uvéite, un glaucome néo-vasculaire et la rétinopathie du prématuré (RDP).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62411908
- WO 2012006202 A1
- WO 2012006203 A1
- US 5262564 A
- US 8697911 B, Cook


### Non-patent literature cited in the description


- **T. W. GREENE ; P. G. M. WUTS.** Protective Groups in Organic Synthesis. Wiley, 1999
- **E. L. ELIEL ; S. H. WILEN ; L. N. MANDER.** Stereochemistry of Organic Compounds. Wiley -Interscience, 1994
- **QUENTMEIER et al.** FLT3 Mutations in Acute Myeloid Leukemia Cell Lines. *Leukemia,* 2003, vol. 17 (1), 120-124
- **GRAFONE et al.** Monitoring of FLT3 Phosphorylation Status and Its Response to Drugs By Flow Cytometry in AML Blast Cells. *Hematol Oncol.,* 2008, vol. 26 (3), 159-166
- **ONISH et al.** Internal Tandem Duplication Mutations in FLT3 Gene Augment Chemotaxis to Cxcl12 Protein by Blocking the Down-regulation of the Rho-associated Kinase via the Cxcl12/Cxcr4 Signaling Axis. *J. Biol. Chem.,* 2014, vol. 289 (45), 31053-31065
- **SCHIROK et al.** Design and Synthesis of Potent and Selective Azaindole-Based Rho Kinase (ROCK) Inhibitors. *ChemMedChem,* 2008, vol. 3, 1893-1904